# EUROPEAN PATENT SPECIFICATION

(11) **EP 0 642 518 B1**
(45) Date of publication and mention of the grant of the patent: **05.08.1998**
(21) Application number: 93914150.3
(22) Date of filing: 26.05.1993
(51) Int. Cl.: C07F 9/38, A61K 31/66, C07F 9/553, C07F 9/6506, C07F 9/6512

(54) **PHOSPHONOSULFONATE COMPOUNDS FOR TREATING ABNORMAL CALCIUM AND PHOSPHATE METABOLISM**
PHOSPHONO SUPHONATE VERBINDUNGEN ZUR BEHANDLUNG ABNORMALEN KALZIUM- UND PHOSPAT- METABOLISMUSES
COMPOSES DE PHOSPHONOSULFONATE DESTINES AU TRAITEMENT D'UN METABOLISME ANORMAL DE CALCIUM ET PHOSPHATE

(30) Priority: 29.05.1992 US 890885; 29.05.1992 US 890886; 29.05.1992 US 891309; 29.05.1992 US 891355; 29.05.1992 US 891487; 29.05.1992 US 891490; 07.05.1993 US 55809
(43) Date of publication of application: 15.03.1995
(73) Proprietor: PROCTER & GAMBLE PHARMACEUTICALS, Norwich, NY 13815 (US)
(72) Inventor: DANSEREAU, Susan, Mary, Sherburne, NY 13460 (US); EBETINO, Frank, Hallock, Cincinnati, OH 45249 (US); BAYLESS, Allan, Vincent, Cincinnati, OH 45241 (US)
(74) Representative: Brooks, Maxim Courtney
(86) International application number: US9304976
(87) International publication number: WO9324495

(56) References cited:
- EP-A- 0 175 315
- EP-A- 0 310 214
- FR-A- 2 385 728

## Description

### BACKGROUND OF THE INVENTION

This invention relates to novel phosphonosulfonate compounds. This invention further relates to pharmaceutical compositions containing these novel compounds, as well as to methods for treating or preventing metabolic bone disorders characterized by abnormal calcium and phosphate metabolism by utilizing a compound or pharmaceutical composition of the present invention. Specifically, this invention relates to methods for treating or preventing osteoporosis, or arthritis, especially rheumatoid arthritis and osteoarthritis, by utilizing a compound or pharmaceutical composition of the present invention.

A number of pathological conditions which can afflict humans and warm blooded animals involve abnormal calcium and phosphate metabolism. Such conditions may be divided into two broad categories:
(1) Conditions which are characterized by anomalous mobilization of calcium and phosphate leading to general or specific bone loss, such as osteoporosis and Paget's disease, or excessively high calcium and phosphate levels in the fluids of the body, such as hypercalcemia of tumor origin. Such conditions are sometimes referred to herein as pathological hard tissue demineralizations.
(2) Conditions which cause or result from deposition of calcium and phosphate anomalously in the body, such as arthritis, including rheumatoid arthritis and osteoarthritis. These conditions are sometimes referred to herein as pathological calcifications.

The first category includes the most common metabolic bone disorder, osteoporosis; osteoporosis is a condition in which bone hard tissue is lost disproportionately to the development of new hard tissue. Osteoporosis can be generally defined as the reduction in the quantity of bone, or the atrophy of skeletal tissue. Marrow and bone spaces become larger, fibrous binding decreases, and compact bone becomes fragile. Osteoporosis can be subclassified as menopausal, senile, drug-induced (e.g. adrenocorticoid, as can occur in steroid therapy); disease-induced (arthritic and tumor), etc.; however, the manifestations are essentially the same.

In general, there are two types of osteoporosis: primary and secondary. "Secondary osteoporosis" is the result of a separate disease process or agent. However, approximately 90% of all osteoporosis cases are "primary osteoporosis". Such primary osteoporosis includes postmenopausal osteoporosis, disuse osteoporosis, age-associated osteoporosis (affecting a majority of individuals over the age of 70 to 80), and idiopathic osteoporosis affecting middle-aged and younger men and women.

For some osteoporotic individuals, the loss of bone tissue is sufficiently great so as to cause mechanical failure of the bone structure. Bone fractures often occur, for example, in the hip and spine of women suffering from postmenopausal osteoporosis. Kyphosis (abnormally increased curvature of the thoracic spine) may also result.

The mechanism of bone loss in osteoporotics is believed to involve an imbalance in the process of "bone remodeling". Bone remodeling occurs throughout life, renewing the skeleton and maintaining the strength of bone. This remodeling involves the erosion and filling of discrete sites on the surface of bones, by an organized group of cells called "basic multicellular units" or "BMUs". BMUs primarily consist of "osteoclasts", "osteoblasts", and their cellular precursors. In the remodeling cycle, bone is resorbed at the site of an "activated" BMU by an osteoclast, forming a resorption cavity. This cavity is then filled with bone by an osteoblast.

Normally, in adults, the remodeling cycle results in a small deficit in bone, due to incomplete filling of the resorption cavity. Thus, even in healthy adults, age-related bone loss occurs. However, in osteoporotics, there may be an increase in the number of BMUs that are activated. This increased activation accelerates bone remodeling, resulting in abnormally high bone loss.

Although its etiology is not fully understood, there are many risk factors thought to be associated with osteoporosis. These include low body weight, low calcium intake, physical inactivity, and estrogen deficiency.

Current osteoporosis treatment consists primarily of calcium and estrogen administration.

In addition to osteoporosis, bone loss can result from arthritis, including rheumatoid arthritis and osteoarthritis. Rheumatoid arthritis is a chronic, systemic and articular inflammatory disorder characterized by weakening of the joint capsules and ligaments, followed by destruction of cartilage, ligaments, tendon and bone, and a decrease in viscosity and other alterations in the synovial fluid. Rheumatoid arthritis symptoms include systemic weakness, fatigue, localized pain, stiffness and weakness and swelling and deformation of the joints of the body. Rheumatoid arthritis is most common in women in the fourth to sixth decade of life.

Osteoarthritis is an inherently non-inflammatory disorder of the movable joints characterized by deterioration and abrasion of articular cartilage, as well as by formation of new bone at the joint surface. As osteoarthritis progresses, the surface of the articular cartilage is disrupted and wear particles gain access to the synovial fluid which in turn stimulates phagocytosis by macrophage cells. Thus, an inflammatory response is eventually induced in osteoarthritis. Common clinical symptoms of osteoarthritis include cartilaginous and bony enlargements of the finger joints and stiffness on awakening and painful movement.

A variety of polyphosphonic acid derivatives have been proposed for use in the treatment and prophylaxis of diseases involving abnormal calcium and phosphate metabolism. For example, numerous references all incorporated by reference herein, disclose compositions containing polyphosphonates, in particular bisphosphonates, such as ethane-1-hydroxy-1,1diphosphonic acid ("EHDP"), and their use in inhibiting anomalous deposition and mobilization of calcium and phosphate in animal tissue: U. S. Patents, 3,683,080, issued August 8, 1972 and U.S. 4,230,700, issued October 28, 1980, both to Francis, and U. S. Patent 4,868,164 to Ebetino, issued September 19, 1989. Numerous other references describe substituted phosphonic acids useful for the treatment of osteoporosis and/or arthritis, and are hereby incorporated by reference herein: U. S. Patents 5,071,840 to Ebetino, et al, issued December 10, 1991, U.S. Patent 4,868,164, to Ebetino, et al., issued September 19, 1989; U.S. Patent 5,104,863, to Benedict, et al., issued April 14, 1992; U.S. Patent 4,267,108, to Blum et al., issued May 12, 1981; U.S. Patent to Breliere, et al., issued May 24, 1988; U.S. Patent 4,876,247 to Barbier, et al., issued October 24, 1989; European Patent Application Publication No. 100,718, of Breliere S.A., published February 15, 1984; European Patent Application Publication No. 170,228, of Boehringer Mannheim GmbH, published February 5, 1986; European Patent Application Publication No. 186,405, of Benedict and Perkins, published July 2, 1986; European Patent Application Publication No. 298,553, of Ebetino, published January 11, 1989; U.S. 4,754,993, to Bosies, et al., issued November 15, 1988; U.S. 4,939,130 to Jaeggi, et al., issued July 3, 1990; U.S. 4,971,958 to Bosies, et al., issued November 20, 1990; WO 90/12017 to Dunn, et al., published October 18, 1990; WO 91/10646 to Youssefyeh, R., et al., published July 25, 1991; AU-A-26738/88 to Jaeggi, K. A., publication date June 15, 1989; AU-A-45467/89 of Ciba-Geigy, publication date May 31, 1990.

A limited number of phosphonosulfonate-containing compounds are disclosed in the literature. See for example, U.S. Patent No. 4,032,521, to Christiansen et al., issued June 28, 1977; PTC Patent Publication 90/07480, by King et al., published July 12, 1990; Great Britain Patent Publication No. 2,248,831, by Doyle et al., published April 22, 1992, and PCT Patent Publication 91/12822, by Erfinder, published September 5, 1991 intermediates. None of these references suggest the utility of phosphonosulfonate compounds, useful in preventing and treating bone metabolism disorders.

It has been surprisingly discovered that the compounds of the present invention, having a phosphonosulfonate moiety, may have potent bone antiresorptive activity and therapeutic utility in treating osteoporosis and/or arthritis. Moreover, these compounds have reduced bone affinity compared with bisphosphonates. This reduced bone affinity may decrease side effects generally associated with the high bone affinity bisphosphonates. Such side effects include inhibition of bone formation and inhibition of bone remodeling activation frequency.

Certain compounds of the present invention contain a quaternary nitrogen moiety. These compounds exhibit unusual solubility properties. Thus, the quaternary nitrogen-containing phosphonosulfonate compounds of the present invention may be more readily orally absorbed. Increased oral absorption allows for improved therapeutic effect at lower doses. Lower doses are generally preferable because undesirable side effects are decreased.

It is therefore an object of the present invention to provide a new potent class of compounds which are potent bone resorption inhibiting agents useful in osteoporosis therapy and anti-arthritic agents useful in the treatment of arthritis, especially osteoarthritis and rheumatoid arthritis. It is a further object of the present invention to provide pharmaceutical compositions useful for the treatment and prophylaxis of abnormal calcium and phosphate metabolism. In addition, it is an object of the present invention to provide methods for treating or preventing diseases characterized by abnormal calcium and phosphate metabolism in humans or other mammals.

These and other objects of the present invention will become apparent from the detailed disclosure of the present invention provided hereinafter.

### SUMMARY OF THE INVENTION

The present invention relates to phosphonosulfonates and the pharmaceutically-acceptable salts and esters thereof, having a structure according to formula (I): 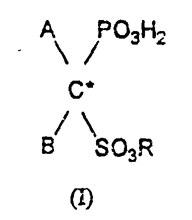 wherein
(A)
   (1) A is selected from the group consisting of hydrogen; halogen; SR¹; R²SR¹; amino; hydroxy; and substituted or unsubstituted C₁-C₈ alkyl;
   (2) B is
      (a) -NH₂;
      (b) a saturated or unsaturated C₁-C₁₅ alkyl chain substituted with one or more substituents selected from the group consisting of -R³N(R⁴)₂; -R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
      (c) a substituted or unsubstituted, saturated or unsaturated heteroalkyl chain having from 2 to 15 chain atoms, where one or more of said chain atoms is nitrogen;
      (d) a saturated or unsaturated heteroalkyl chain having from 2 to 15 chain atoms, where one or more of said chain atoms is selected from S and O; and where said heteroalkyl chain is substituted with one or more substituents selected from the group consisting of -R³N(R⁴)2; -R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂; or
      (e) R⁶-L- where
         (i) L is selected from the group consisting of nil; S; O; a substituted or unsubstituted, saturated or unsaturated C₁-C₁₅ alkylene chain; and a substituted or unsubstituted, saturated or unsaturated heteroalkylene chain having from 2 to 15 chain atoms, where one or more of said chain atoms is N, S, or O; and
         (ii) R⁶ is selected from the group consisting of saturated monocyclic or polycyclic carbocyclic rings; unsaturated monocyclic or polycyclic carbocyclic rings; saturated monocyclic or polycyclic heterocyclic rings; and unsaturated monocyclic or polycyclic heterocyclic rings; wherein R⁶ may be substituted with one or more substituents independently selected from the group consisting of -R³SR¹; substituted or unsubstituted C₁-C₈ alkyl; -R³OR⁴; -R³CO₂R⁴; -R³O₂CR⁴; -R³N(R⁴)₂; R³[-N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; arylalkyl; nitro; substituted or unsubstituted aryl; and hydroxy; and
   (3)
      (a) R¹ is independently selected from the group consisting of hydrogen; -C(O)R⁷; -C(S)R⁷; -C(O)N(R⁷)₂; -C(O)OR⁷; -C(S)N(R⁷)₂; and -C(S)OR⁷; where R⁷ is hydrogen or substituted or unsubstituted C₁-C₈ alkyl;
      (b) R² is substituted or unsubstituted C₁-C₈ alkylene;
      (c) R³ is selected from the group consisting of nil and substituted or unsubstituted C₁-C₈ alkylene;
      (d) R⁴ is independently selected from the group consisting of hydrogen; substituted or unsubstituted C₁-C₈ alkyl; and -R²SR¹; and
      (e) R⁵ is independently selected from the group consisting of substituted or unsubstituted C₁-C₁₅ alkyl; substituted or unsubstituted phenyl; benzyl; and -R²SR¹ ;
   or
(B) A and B are covalently linked together with C* to form a monocyclic or bicyclic ring having the following structure: 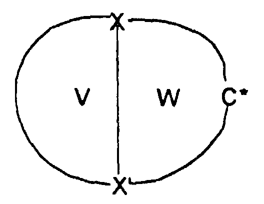 where
   (1) W is a substituted or unsubstituted, saturated or unsaturated carbocyclic ring comprising C*, X, and X', said carbocyclic ring having a total of from 3 to 6 ring carbon atoms; or a substituted or unsubstituted, saturated or unsaturated heterocyclic ring comprising C*, X, and X', said heterocyclic ring having a total of from 4 to 6 ring atoms, where one or more of said ring atoms is N, O, or S;
   (2) V is nil; a substituted or unsubstituted, saturated or unsaturated carbocyclic ring comprising X and X', said carbocyclic ring having a total of from 3 to 8 ring carbon atoms; or a substituted or unsubstituted, saturated or unsaturated heterocyclic ring comprising X and X', said heterocyclic ring having a total of from 3 to 8 ring atoms, where one or more of said ring atoms is N, O, or S; and
   (3) X and X' are independently N or C; except that if neither V nor W is a nitrogen containing heterocycle, then at least one of V or W is substituted with one or more substituents selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
and wherein R is selected from the group consisting of hydrogen, lower alkyl, lower acyloxyalkyl, aminocarbonyloxyalkyl, pivaloyloxymethyl, lower alkoxyacyloxyalkyl, alkoxyalkyl, choline and acylamino alkyl.

The present invention further relates to pharmaceutical compositions comprising a safe and effective amount of a phosphonosulfonate, or a pharmaceutically-acceptable salt or ester thereof. Finally, the present invention relates to uses of the compounds herein for the manufacture of a medicament for treating or preventing pathological conditions characterized by abnormal calcium and phosphate metabolism in humans or other mammals. These uses comprise administering to a human or other mammal in need of such treatment a safe and effective amount of a compound or a composition of the present invention.

### DETAILED DESCRIPTION OF THE INVENTION

### Phosphonosulfonate Compounds

The novel compounds of the present invention are phosphono-sulfonates and the pharmaceutically-acceptable salts and esters thereof, having a structure according to formula (I): 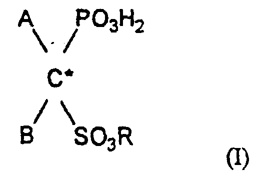 wherein
(A)
   (1) A is selected from the group consisting of hydrogen; halogen; SR¹; R²SR¹; amino; hydroxy; and substituted or unsubstituted C₁-C₈ alkyl;
   (2) B is
      (a) -NH₂;
      (b) a saturated or unsaturated C₁-C₁₅ alkyl chain substituted with one or more substituents selected from the group consisting of -R³N(R⁴)₂; -R³(-N(R⁵)₃]+; -R³N(R⁴)C(O)R⁴; -R³N(R⁴)C(S)R⁴; -R³N(R⁴)C(N)R⁴; and -R³C(O)N(R⁴)₂;
      (c) a substituted or unsubstituted, saturated or unsaturated heteroalkyl chain having from 2 to 15 chain atoms, where one or more of said chain atoms is nitrogen;
      (d) a saturated or unsaturated heteroalkyl chain having from 2 to 15 chain atoms, where one or more of said chain atoms is selected from S and O; and where said heteroalkyl chain is substituted with one or more substituents selected from the group consisting of -R³N(R⁴)2; -R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂; or
      (e) R⁶-L- where
         (i) L is selected from the group consisting of nil; S; O; a substituted or unsubstituted, saturated or unsaturated C₁-C₁₅ alkylene chain; and a substituted or unsubstituted, saturated or unsaturated heteroalkylene chain having from 2 to 15 chain atoms, where one or more of said chain atoms is N, S, or O; and
         (ii) R⁶ is selected from the group consisting of saturated monocyclic or polycyclic carbocyclic rings; unsaturated monocyclic or polycyclic carbocyclic rings; saturated monocyclic or polycyclic heterocyclic rings; and unsaturated monocyclic or polycyclic heterocyclic rings; wherein R⁶ may be substituted with one or more substituents independently selected from the group consisting of -R³SR¹; substituted or unsubstituted C₁-C₈ alkyl; -R³OR⁴; -R³CO₂R⁴; -R³O₂CR⁴; -R³N(R⁴)₂; R³[-N(R⁵)₃)+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; arylalkyl; nitro; substituted or unsubstituted aryl; and hydroxy; and
   (3)
      (a) R¹ is independently selected from the group consisting of hydrogen; -C(O)R⁷; -C(S)R⁷; -C(O)N(R⁷)₂; -C(O)OR⁷; -C(S)N(R⁷)₂; and -C(S)OR⁷; where R⁷ is hydrogen or substituted or unsubstituted C₁-C₈ alkyl;
      (b) R² is substituted or unsubstituted C₁-C₈ alkylene;
      (c) R³ is selected from the group consisting of nil and substituted or unsubstituted C₁-C₈ alkylene;
      (d) R⁴ is independently selected from the group consisting of hydrogen; substituted or unsubstituted C₁-C₈ alkyl; and -R²SR¹; and
      (e) R⁵ is independently selected from the group consisting of substituted or unsubstituted C₁-C₁₅ alkyl; substituted or unsubstituted phenyl; benzyl; and -R²SR¹;
   or
(B) A and B are covalently linked together with C* to form a monocyclic or bicyclic ring having the following structure: 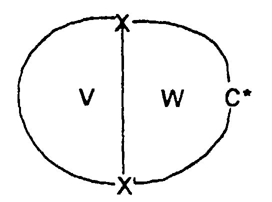 where
   (1) W is a substituted or unsubstituted, saturated or unsaturated carbocyclic ring comprising C*, X, and X', said carbocyclic ring having a total of from 3 to 6 ring carbon atoms; or a substituted or unsubstituted, saturated or unsaturated heterocyclic ring comprising C*, X, and X', said heterocyclic ring having a total of from 4 to 6 ring atoms, where one or more of said ring atoms is N, O, or S;
   (2) V is nil; a substituted or unsubstituted, saturated or unsaturated carbocyclic ring comprising X and X', said carbocyclic ring having a total of from 3 to 8 ring carbon atoms; or a substituted or unsubstituted, saturated or unsaturated heterocyclic ring comprising X and X', said heterocyclic ring having a total of from 3 to 8 ring atoms, where one or more of said ring atoms is N, O, or S; and
   (3) X and X' are independently N or C;
   except that if neither V nor W is a nitrogen containing heterocycle, then at least one of V or W is substituted with one or more substituents selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
and wherein R is selected from the group consisting of hydrogen, lower alkyl, lower acyloxyalkyl, aminocarbonyloxyalkyl, pivaloyloxymethyl, lower alkoxyacyloxyalkyl, alkoxyalkyl, choline and acylamino alkyl.

### Definitions and Usage of Terms

The following is a list of definitions for terms used herein.

"Heteroatom" is a nitrogen, sulfur, or oxygen atom. Groups containing one or more heteroatoms may contain different heteroatoms.

"Alkyl" is an unsubstituted or substituted, straight-chain or branched, saturated or unsaturated hydrocarbon chain, said hydrocarbon chain may be saturated, having 1 to 15 carbon atoms, and preferably, unless otherwise stated, from 1 to 4 carbon atoms; said hydrocarbon chain may be unsaturated, having 2 to 8 carbon atoms, and preferably, unless otherwise stated, 2 to 4 carbon atoms. Accordingly, the term "alkyl", as used herein, encompasses alkenyl hydrocarbon unsaturated chains having at least one olefinic double bond and alkynyl hydrocarbon unsaturated chains having at least one triple bond. Preferred alkyl groups include, but are not limited to, methyl, ethyl, propyl, isopropyl, and butyl.

"Heteroalkyl" is an unsubstituted or substituted, saturated or unsaturated, straight-chain or branched heteroalkyl chain, said chain having from 2 to 15, preferably 2 to 8 members, and comprising at least one carbon atom and at least one heteroatom. The term "heteroalkyl", as used herein, encompasses alkenyl heteroalkyl unsaturated chains having at least one olefinic double bond and alkynyl heteroalkyl unsaturated chains having at least one triple bond.

"Carbocyclic ring" or "Carbocycle" as used herein is an unsubstituted or substituted, saturated, unsaturated or aromatic, hydrocarbon ring. Carbocycles may be monocyclic or polycyclic: Monocyclic rings generally contain from 3 to 8 atoms, preferably 5 to 8 atoms. Polycyclic rings containing two rings contain 6-16, preferably 10 to 12, atoms and those with three rings generally contain 13 to 17, preferably 14 to 15, atoms.

"Heterocyclic ring" or "Heterocycle" as used herein is an unsubstituted or substituted, saturated, unsaturated or aromatic ring comprised of carbon atoms and one or more heteroatoms in the ring. Heterocyclic rings may be monocyclic or polycyclic. Monocyclic rings generally contain from 3 to 8 atoms, preferably 5 to 7 atoms. Polycyclic ring systems consisting of two rings generally contain 6 to 16, preferably from 10 to 12 atoms. Polycyclic ring systems consisting of three rings generally contain 13 to 17 atoms, preferably 14 to 15 atoms. Unless otherwise stated the heteroatoms may be independently chosen from nitrogen, sulfur, and oxygen. Unsaturated, non-aromatic heterocycles include, but are not limited to, substituted or unsubstituted thiophene, substituted or unsubstituted oxathiazole, substituted or unsubstituted pyrans, and substituted or unsubstituted furans.

"Aryl" is an aromatic carbocyclic ring. Preferred aryl groups include, but are not limited to, phenyl, tolyl, xylyl, cumenyl, and naphthyl.

"Heteroaryl" is an aromatic heterocyclic ring. Preferred heteroaryl groups include, but are not limited to, thienyl, furyl, pyrrolyl, pyridinyl, pyrazinyl, oxazolyl, thiazolyl, quinolinyl, pyrimidinyl, and tetrazolyl.

"Alkoxy" is an oxygen atom having a hydrocarbon chain substituent, where the hydrocarbon chain is an alkyl or alkenyl (e.g., -O-alkyl or -O-alkenyl). Preferred alkoxy groups include, but are not limited to, methoxy, ethoxy, propoxy, and alkyloxy.

"Hydroxyalkyl" is a substituted hydrocarbon chain which has a hydroxy substituent (e.g., -OH), and may have other substituents. Preferred hydroxyalkyl groups include, but are not limited to, hydroxyethyl and hydroxypropyl.

"Carboxyalkyl" is a substituted hydrocarbon chain which has a carboxy substituent (e.g. -COOH) and may have other substituents. Preferred carboxyalkyl groups include carboxymethyl, carboxyethyl, and their acids and esters.

"Aminoalkyl" is a hydrocarbon chain (e.g. alkyl) substituted with an amine moiety (e.g., NH-alkyl-), such as aminomethyl alkyl.

"Alkylamino" is an amino moiety having one or two alkyl substituents (e.g., -N-alkyl), such as dimethylamino.

"Alkenylamino" is an amino moiety having one or two alkenyl substituents (e.g., -N-alkenyl).

"Alkynylamino" is an amino moiety having one or two alkynyl substituents (e.g., -N-alkynyl).

"Alkylimino" is an imino moiety having one or two alkyl substituents (e.g., -N-alkyl-).

"Arylalkyl" is an alkyl moiety substituted with an aryl group. Preferred arylalkyl groups include benzyl and phenylethyl.

"Arylamino" is an amine moiety substituted with an aryl group (e.g.,-NH-aryl).

"Aryloxy" is an oxygen atom having an aryl substituent (e.g., -O-aryl).

"Acyl" or "carbonyl" is a carbon to oxygen double bond, e.g. R-C(=O). Preferred acyl groups include, but are not limited to, acetyl, propionyl, butanoyl and benzoyl.

"Acyloxy" is an oxygen atom having an acyl substituent (e.g., -O-acyl); for example, -O-C(=O)-alkyl.

"Acylamino" is an amino moiety having an acyl substituent (e.g., -N-acyl); for example, -NH-(C=O)-alkyl.

"Halo", "halogen", or "halide" is a chloro, bromo, fluoro, or iodo atom radical. Chloro, bromo, and fluoro are preferred halides.

Also, as referred to herein, a "lower" hydrocarbon moiety (e.g., "lower" alkyl) is a hydrocarbon chain comprised of from, unless otherwise stated, 1 to 6, preferably from 1 to 4, carbon atoms.

As used herein the term "thio-substituent" (SR¹ or R²SR¹) includes thiols [-SH] where R¹=H; thioesters [-SC(O)R⁷] where R¹=C(O)R⁷; dithioesters [-SC(S)R⁷] where R¹=C(S)R⁷; thiocarbamates [-SC(O)N(R⁷)₂] where R¹=C(O)N(R⁷)₂; dithiocarbamates [-SC(S)N(R⁷)₂] where R¹=C(S)N(R⁷)₂; thiocarbonates [-SC(O)OR⁷] where R¹=C(O)OR⁷; and dithiocarbonates [-SC(S)OR⁷] where R¹=C(S)OR⁷. R⁷ is a hydrogen or C₁-C₈ alkyl. Any of the SR¹ substituents may themselves be substituted with an R² moiety, where R² is a substituted or unsubstituted C₁-C₈ alkyl. Accordingly, additional thio-substituents denoted by R²SR¹ are alkylthiols, alkylthioesters, alkyldithioesters, alkylthiocarbamates, alkyldithiocarbamates, alkylthiocarbonates and alkyl dithiocarbonates.

The term "phosphonosulfonate", as used herein, relates to compounds that have a phosphonate group (PO₃H₂) and a sulfonate group (SO₃H) attached to the same carbon atom.

A "pharmaceutically-acceptable" salt is a cationic salt formed at any acidic (e.g., carboxyl) group, or an anionic salt formed at any basic (e.g., amino) group. Many such salts are known in the art, as described in World Patent Publication 87/05297, Johnston et al., published September 11, 1987, hereby incorporated by reference herein. Preferred cationic salts include the alkali-metal salts (such as sodium and potassium), and alkaline earth metal salts (such as magnesium and calcium). Preferred anionic salts include the halides (such as chloride), acetate and phosphate salts.

A "biohydrolyzable ester" is an ester of the phosphonosulfonate compounds that does not interfere with the therapeutic activity of the compounds, or that is readily metabolized by a human or other mammal. Many such esters are known in the art, as described in World Patent Publication 87/05297, Johnston et al., published September 11, 1987, and hereby incorporated by reference herein. Such esters include lower alkyl esters, lower acyloxyalkyl esters (such as acetoxylmethyl, acetoxyethyl, aminocarbonyloxymethyl, pivaloyloxymethyl, and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters, and acylamino alkyl esters (such as acetamidomethyl esters). While the preceding esters are prefered, any pharmaceutically-acceptable ester is deemed to be within the scope of the present invention.

As defined above and as used herein, substituent groups may themselves be substituted. Such substitution may be with one or more substituents. Such substituents include, but are not limited to, those listed in C. Hansch and A. Leo, Substituent Constants for Correlation Analysis in Chemistry and Biology (1979), hereby incorporated by reference herein. Preferred substituents include, but are not limited to, alkyl, alkenyl, alkoxy, hydroxy, oxo, thioxo (-C(=S)-); amino, aminoalkyl (e.g. aminomethyl, etc.), cyano, quaternary amino, quaternary aminoalkyl, amidino, amidinoalkyl, halo, carboxy, alkoxyacetyl (e.g. carboethoxy, etc.), thio, thiol, aryl, cycloalkyl, heteroaryl, heterocycloalkyl (e.g., piperidinyl, morpholinyl, piperazinyl, pyrrolidinyl, etc.), imino, hydroxyalkyl, aryloxy, arylalkyl, alkynyl and combinations thereof. Particularly preferred substituents include, but are not limited to, amino, aminoalkyl, quaternary amino, amidino, quaternary aminoalkyl and amidinoalkyl.

Also, as used in defining the structure of the compounds of this invention, a particular radical may be defined for use as a substituent, in multiple locations. As used herein, such a radical is independently selected each time it is used.

In formula (I), when A is halogen, it is preferably chlorine or bromine. When A is a sulfur containing moiety, the preferred moiety is SR¹, where R¹ is preferably hydrogen or acyl. Particularly preferred is where R¹ is hydrogen. Preferred A moieties are amino and hydroxy. Particularly preferred is where A is hydroxy. When B is saturated or unsaturated C₁-C₁₅ alkyl, the alkyl chain must be substituted with one or more substituents selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂. Preferably the required substituent is selected from -R³N(R⁴)₂; R³[-N(R⁵)₃]+;
-R³N(R⁴)C(O)R⁴; -R³N(R⁴)C(S)R⁴; and -R³N(R⁴)C(N)R⁴. Most preferred is where the required substituent is selected from -R³N(R⁴)₂; R³[-N(R⁵)₃]+; and -R³N(R⁴)C(O)R⁴. The alkyl chain may also be substituted with one or more substituents selected from the group consisting of nil; -R³SR¹; hydrogen; substituted or unsubstituted C₁-C₈ alkyl; -R³OR⁴; -R³CO₂R⁴; -R³O₂CR⁴; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings. Preferred are C₁-C₈ alkyl chains.

When B is saturated or unsaturated heteroalkyl having from 2 to 15 atoms, where one of said atoms is a nitrogen, the heteroalkyl chain may be substituted with one or more substituents selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; -R³[N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings. Preferred nitrogen-containing heteroalkyl chains have from 2 to 8 chain atoms.

When B is saturated or unsaturated heteroalkyl having from 2 to 15 atoms, where one of said atoms is a sulfur or oxygen, and where a no nitrogen atom is in the heteroalkyl chain, then the heteroalkyl chain must be substituted with one or more substituents selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂. The required substituent is preferably one of -R³N(R⁴)₂; R³[-N(R⁵)₃]+; -R³N(R⁴)C(O)R⁴; -R³N(R⁴)C(S)R⁴; or -R³N(R⁴)C(N)R⁴. Most preferred is where the required substituent is -R³N(R⁴)₂; R³[-N(R⁵)₃]+; or -R³N(R⁴)C(O)R⁴. The heteroalkyl chain may also be substituted with one or more substituents selected from the group consisting of nil; -R³SR¹; hydrogen; substituted or unsubstituted C₁-C₈ alkyl; -R³OR⁴; -R³CO₂R⁴; -R³O₂CR⁴; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings. Preferred non-nitrogen containing heteroalkyl chains have from 2 to 8 chain atoms.

When B is R⁶-L-, the L moiety may be substituted with one or more substituents selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; -R³[N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings. The L moiety is preferably a nitrogen containing heteroalkyl, or alkyl. Where L is a heteroalkyl chain or an alkyl chain, the chain preferably has 1 to 8 chain atoms.

The R⁶ moiety may be a saturated or unsaturated, monocyclic or polycyclic carbocycle or heterocycle. Where R⁶ is a monocyclic carbocycle, it is preferably cycloheptyl or cyclohexyl. When R⁶ is a monocyclic heterocycle, preferred are six-membered nitrogen containings rings including pyridine, pyrimidine, piperidine. Also preferred are those six-membered heterocycles having a quaternary nitrogen ring atom, including pyridinium, pyrimidinium, piperidinium, and pyrazolium. Preferred monocyclic heterocycles also include five-membered nitrogen containing heterocycles, including imidazol, pyrrole, and pyrrolidine. Also preferred are five-membered heterocycles having a quaternary nitrogen ring atom, including imidazolium, pyrrolium, and pyrrolidinium. Where R⁶ is a polycycle, preffered are polycyclic heterocycles having a six-membered ring fused to another six-membered ring and those having a six-membered ring fused to a five-membered ring. Preferred polycyclic heterocycles include those having a quaternary ring nitrogen atom. Particularly preferred R⁶ moieties include pyridyl and cycloheptyl.

The R³ moiety is preferably nil.

The R⁴ moiety is preferably hydrogen.

The R⁵ moiety comprises a nitrogen atom bound to three carbon-containing moieties. The R⁵ moiety is substituted on a carbon atom of another moiety, thus providing a quaternary amine group. As indicated in the general structure, the quaternary amine moiety may be a substituent on any of the chain or cylcic moieties described above.

B is preferably a heteroalkyl chain having at least one nitrogen chain atom, or R⁶-L-. Particularly preferred B moieties are R⁶-L-.

According to formula (I), A and B may, together with C*, X and X', form a cyclic structure. Preferred cyclic structures are those where V is a heterocyle having at least one ring nitrogen atom. This ring nitrogen atom may be a secondary, tertiary or quaternary amine. Where neither V nor W is a heterocycle having at least one ring nitrogen atom, then at least one of V or W must be substituted with one or more moieties selected from the group consisting of -R³N(R⁴)₂; R³[-N(R⁵)₃]+; -R³-N(R⁴)C(O)R⁴;
-R³N(R⁴)C(S)R⁴; -R³N(R⁴)C(N)R⁴; and -R³C(O)N(R⁴)₂. In addition to the above requirement, each of V and W may be substituted with one or more substituents selected from the group consisting of -R³SR¹; hydrogen; substituted or unsubstituted C₁-C₈ alkyl; -R³OR⁴; -R³CO₂R⁴; -R³O₂CR⁴; halogen; -R³C(O)R⁴; hydroxy; substituted or unsubstituted arylalkyl; nitro; and unsubstituted or substituted aryl.

Preferred compounds of the present invention are phosphono-sulfonates and the pharmaceutically-acceptable salts and esters thereof, having a general structure according to formula (I): 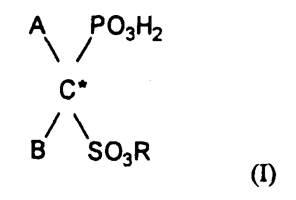 wherein
(A)
   (1) A is selected from the group consisting of amino and hydroxy; and
   (2) B is 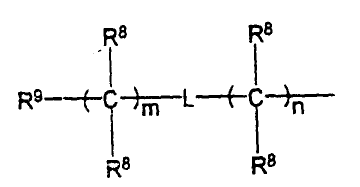 wherein
      (a) m is an integer from 0 to 10; n is an integer from 0 to 10; and m+n is an integer from 0 to 10;
      (b) R⁸ is independently selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; -R³[N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings;
      (c) R¹ is independently selected from the group consisting of hydrogen; -C(O)R⁷; -C(S)R⁷; -C(O)N(R⁷)₂; -C(O)OR⁷; -C(S)N(R⁷)₂; and -C(S)OR⁷; where R⁷ is hydrogen or substituted or unsubstituted C₁-C₈ alkyl;
      (d) R³ is selected from the group consisting of nil and substituted or unsubstituted C₁-C₈ alkylene;
      (e) R⁴ is independently selected from the group consisting of hydrogen; substituted or unsubstituted C₁-C₈ alkyl; and -R²SR¹;
      (f) R⁵ is independently selected from the group consisting of substituted or unsubstituted C₁-C₁₅ alkyl; substituted or unsubstituted phenyl; benzyl; and -R²SR¹ ;
      (g) L is selected from the group consisting of nil; -N(R⁸)-; [-N(R⁵)₂-)+; -S-; -O-; and -D-C(=E)-S-, where D is selected from the group consisting of covalent bond, O, or S, and E is O or S; and wherein
         (i) when L is -N(R⁸)-, or when L is [-N(R⁵)₂-]+ and m is an integer from 1 to 10, R⁹ is independently selected from the group consisting of hydrogen; substituted or unsubstituted C₁-C₁₅ alkyl; R²SR¹; and R¹⁰;
         (ii) when L is [-N(R⁵)₂-]+ and m = 0, R⁹ is selected from the group consisting of substituted or unsubstituted C₁-C₁₅ alkyl; R²SR¹; and R¹⁰; or
         (iii) when L is nil, -S-, -O-, or -D-C(=E)-S, R⁹ is R¹⁰;
      (h) R¹⁰ is a saturated, unsaturated, or aromatic monocyclic or polycylic carbocycle or a saturated, unsaturated, or aromatic monocyclic or polycylic heterocycle containing one or more heteroatoms; where said carbocycle or heterocycle is substituted with one or more R¹¹ substituents; and
      (i) each R¹¹ is independently selected from the group consisting of -R³SR¹; substituted or unsubstituted C₁-C₈ alkyl; -R³OR⁴; -R³CO₂R⁴; -R³O₂CR⁴; -R³N(R⁴)₂; R³[-N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; hydroxy; substituted or unsubstituted arylalkyl; nitro; and unsubstituted or substituted aryl;
      (j) R² is substituted or unsubstituted C₁-C₈ alkylene;
or (B) A and B are covalently linked together with C* to form a monocyclic or bicyclic ring having the following structure: 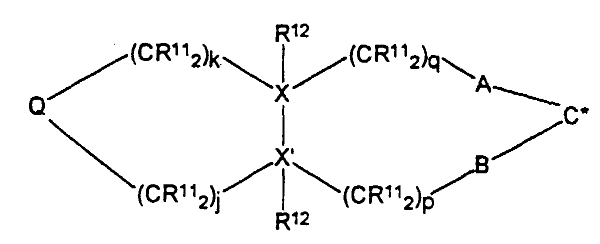 wherein
   (a) A and B are independently selected from the group consisting of nil, -O-, -S-, and -NR¹²-;
   (b) Q is selected from the group consisting of nil; -NR¹²-; and [-N(R¹³)₂-]+;
   (c) X and X' are independently selected from C or N;
   (d) each R¹² is independently selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; R³[-N(R⁵)₃]+; -R³N(R⁴)C(O)R⁴; -R³C(O)N(R⁴)₂; halogen; -R³C(O)R⁴; hydroxy; substituted or unsubstituted arylalkyl; nitro; and unsubstituted or substituted aryl; and
   (e) each R¹³ is selected from the group consisting of substituted or unsubstituted C₁-C₁₅ alkyl; substituted or unsubstituted phenyl; benzyl; and -R²SR¹;
   (f) When Q is other than nil, k and j and k+j are integers from 0 to 5; when Q is nil, k and j and k+j are integers from 0 to 6; and
   (g) p and q and p+q are independently integers from 0 to 3; except that if Q is nil, then at least one of R¹¹ or R¹² is selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
and wherein R is selected from the group consisting of hydrogen, lower alkyl, lower acyloxyalkyl, aminocarbonyloxyalkyl, pivaloyloxymethyl, lower alkoxyacyloxyalkyl, alkoxyalkyl, choline and acylamino alkyl.

Preferred phosphonosulfonate compounds of formula (I) have a nitrogen containing heterocycle linked to the phosphonosulfonate geminal carbon via a linking chain. Included are phosphonosulfonate compounds having the following general structures: 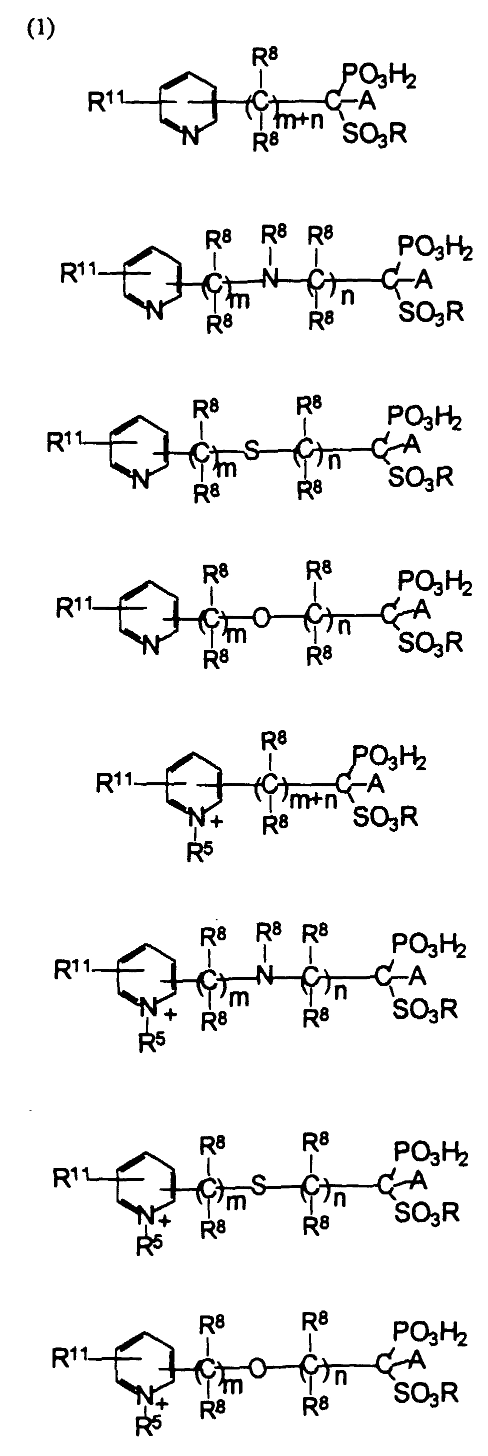 where the nitrogen containing heterocycle is a pyridyl or pyridinium; 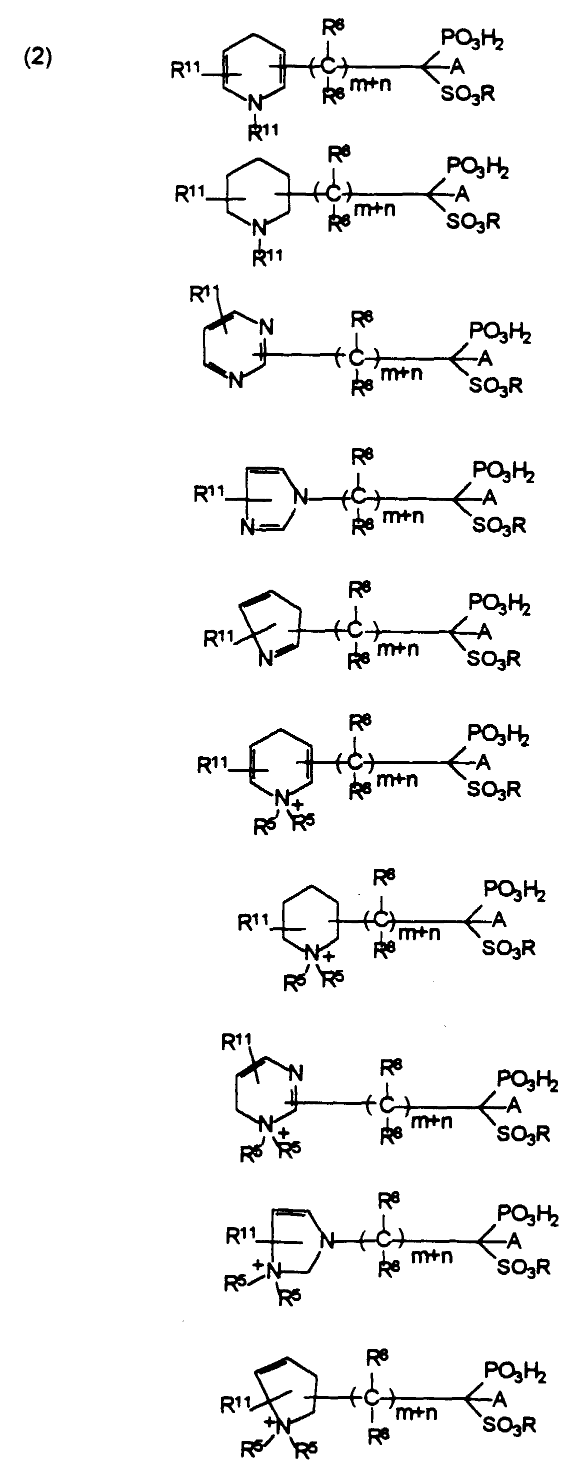 where the nitrogen containing heterocycle is a monocycle other than pyridyl or pyridinium; 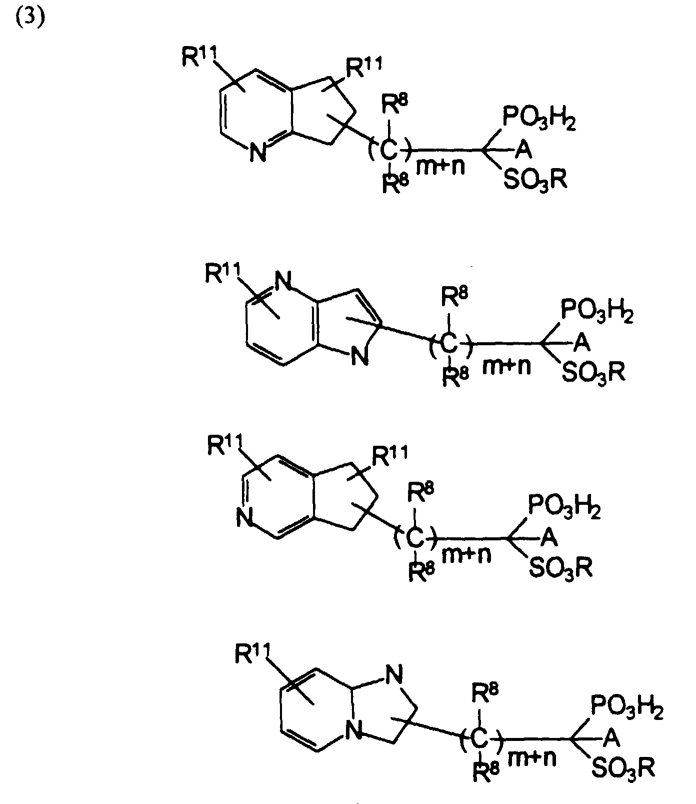 where the nitrogen containing heterocycle is a polycycle.

Also preferred are those phosphonosulfonate compounds having a nitrogen containing heteroalkyl moiety linked to the phosphonosulfonate containing geminal carbon. Such compounds include those having the following structure, where R⁸ and R⁹ are non-cyclic substituents:

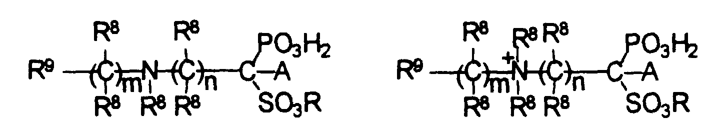

Also preferred are those compounds having the following structure, where R⁹ is a cycloheptyl ring:

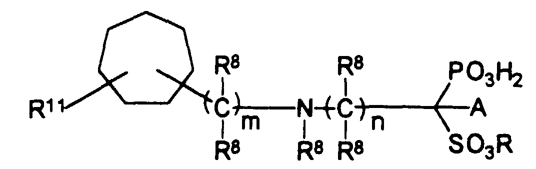

Also preferred are substituted or unsubstituted octahydro phosphonosulfono pyrindines having the general structures: 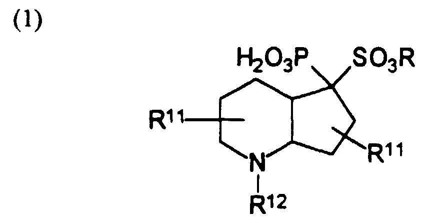 referred to herein as unsubstituted or substituted octahydro-5-phosphono-5-sulfono-1-pyrindines; 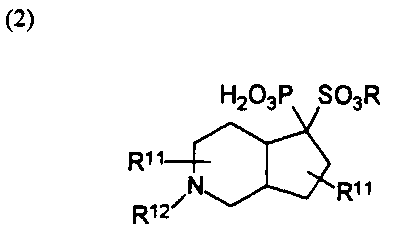 referred to herein as unsubstituted or substituted octahydro-5-phosphono-5-sulfono-2-pyrindines; 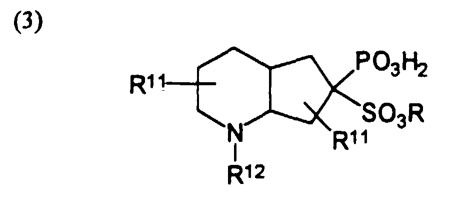 referred to herein as unsubstituted or substituted octahydro-6-phosphono-6-sulfono-1-pyrindines; 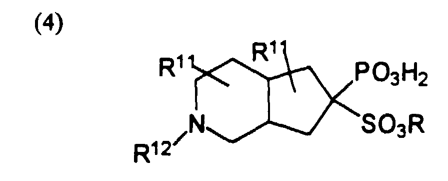 referred to herein as unsubstituted or substituted octahydro-6-phosphono-6-sulfono-2-pyrindines; 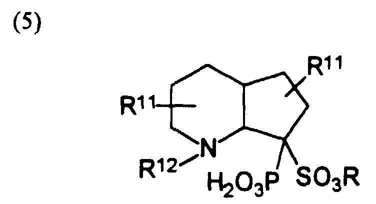 referred to herein as octahydro-7-phosphono-7-sulfono-1-pyrindines; 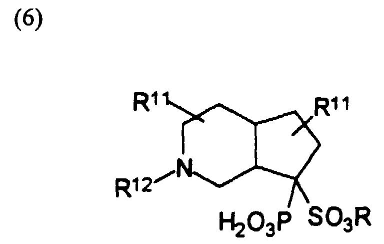 referred to herein as octahydro-7-phosphono-7-sulfono-2-pyrindines;

Also preferred are substituted or unsubstituted octahydro phosphonosulfono pyrindiniums having the general structures: 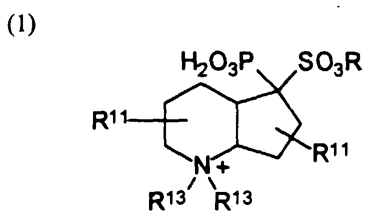 referred to herein as octahydro-5-phosphono-5-sulfono-1-pyrindinium; 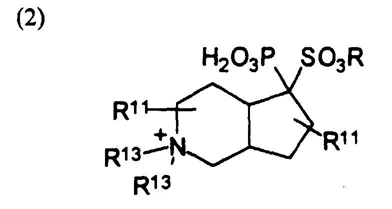 referred to herein as octahydro-5-phosphono-5-sulfono-2-pyrindinium; 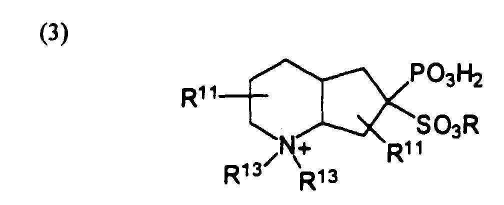 referred to herein as octahydro-6-phosphono-6-sulfono-1-pyrindinium; 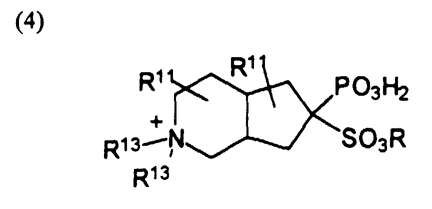 referred to herein as octahydro-6-phosphono-6-sulfono-2-pyrindinium; 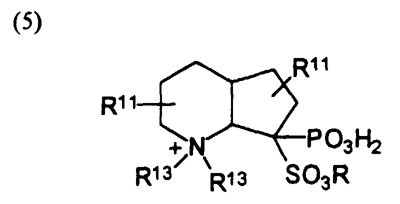 referred to herein as octahydro-7-phosphono-7-sulfono-1-pyrindinium; and 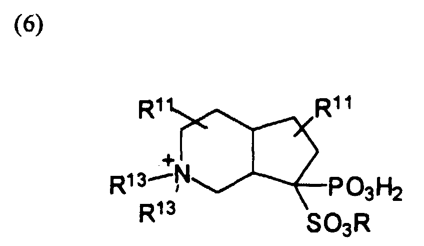 referred to herein as octahydro-7-phosphono-7-sulfono-2-pyrindinium.

Specific examples of compounds of the present invention are: 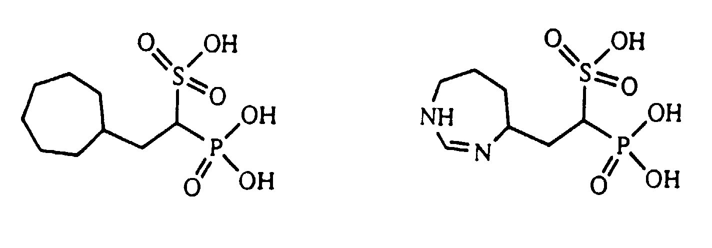 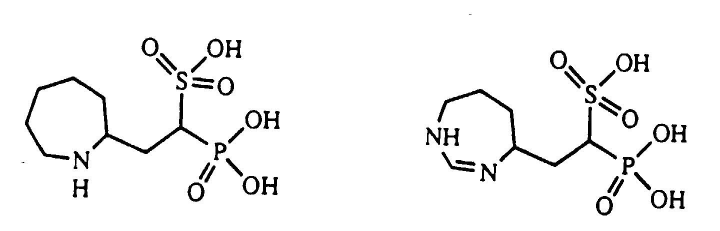 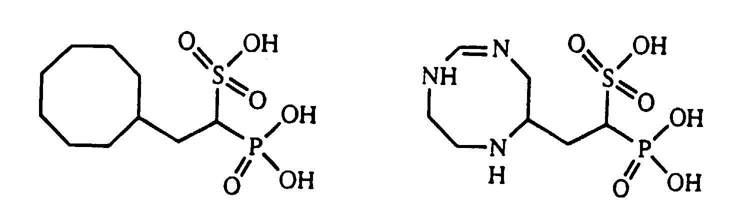 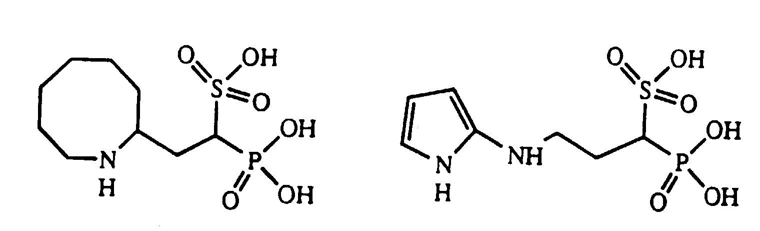 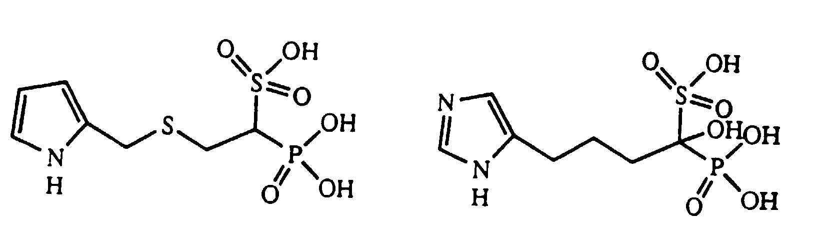 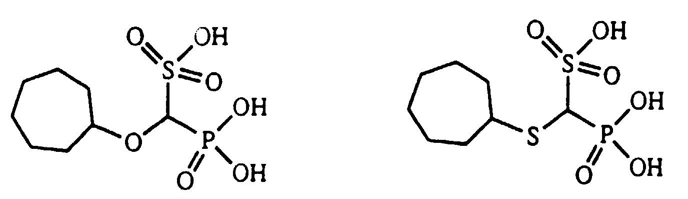 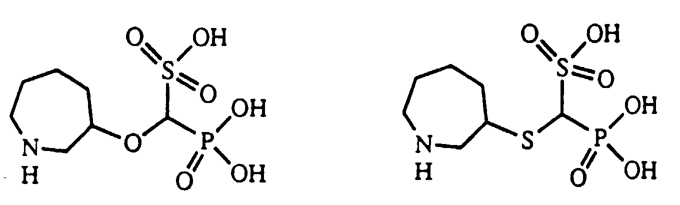 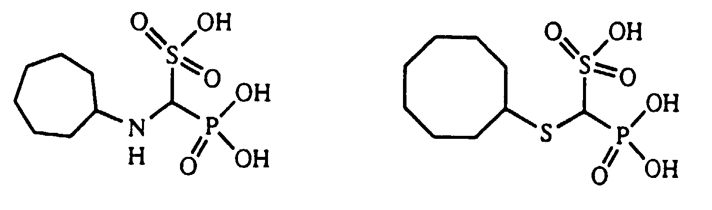 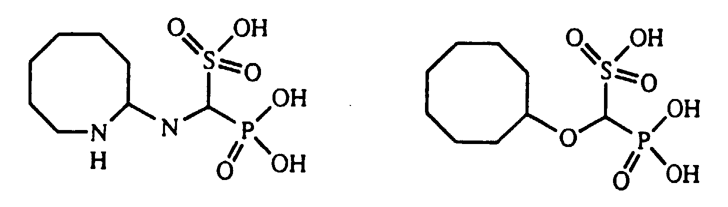 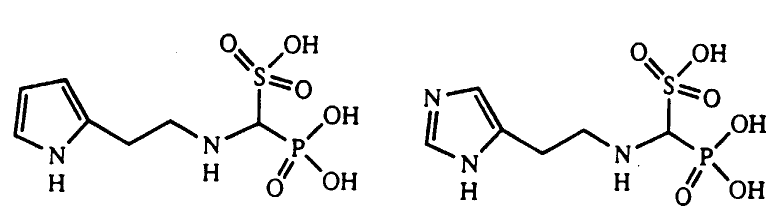 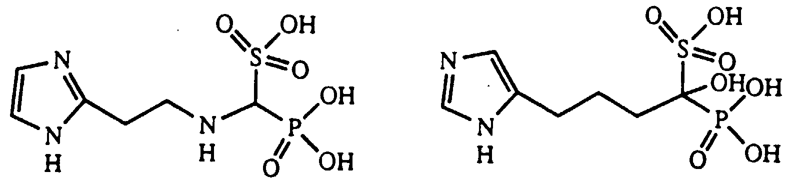 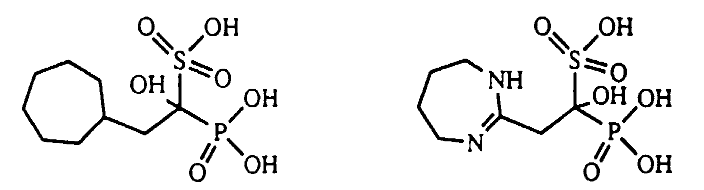 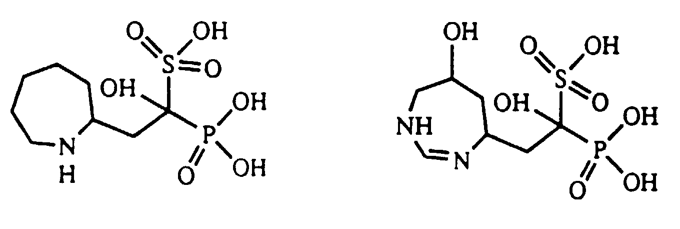 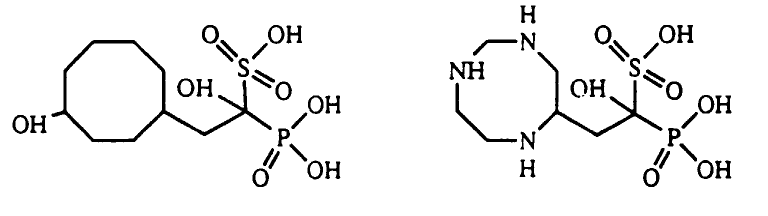 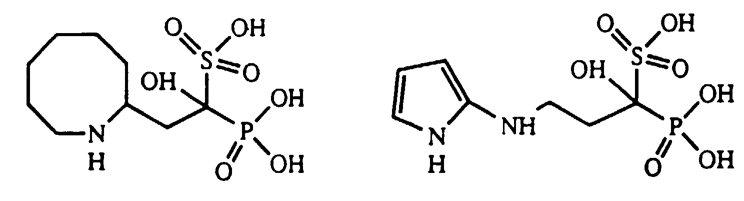 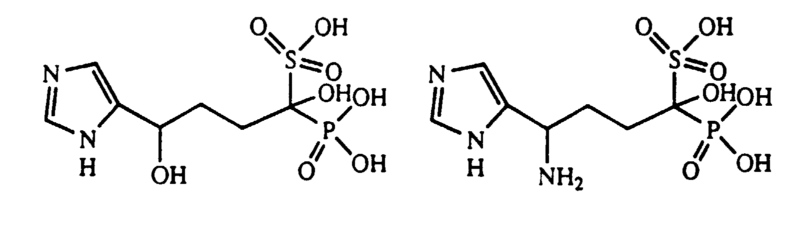 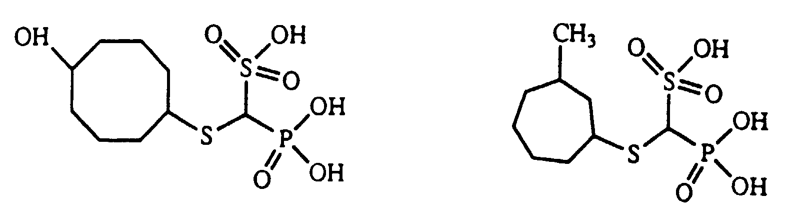 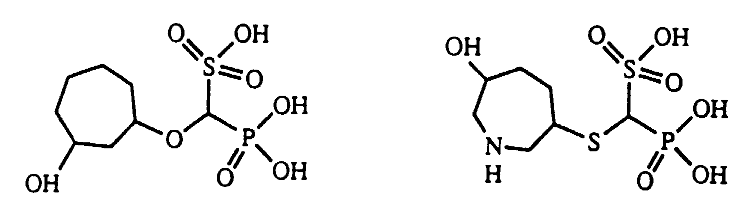 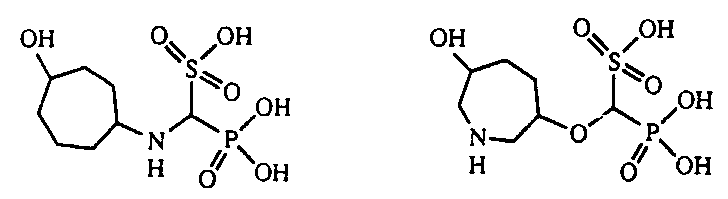 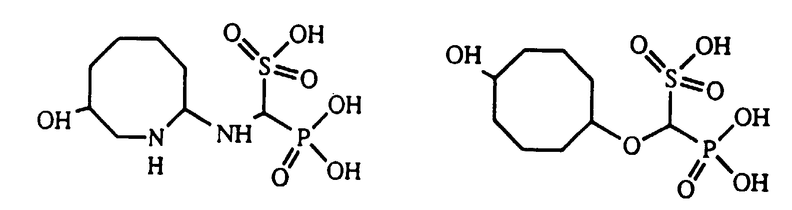 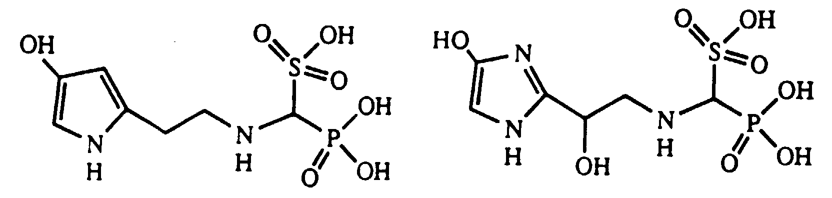 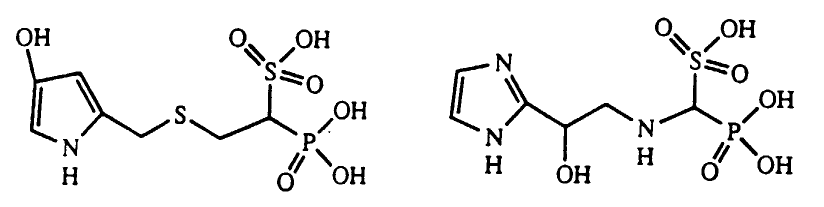 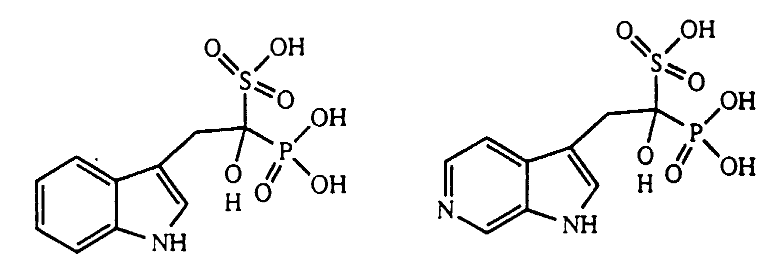 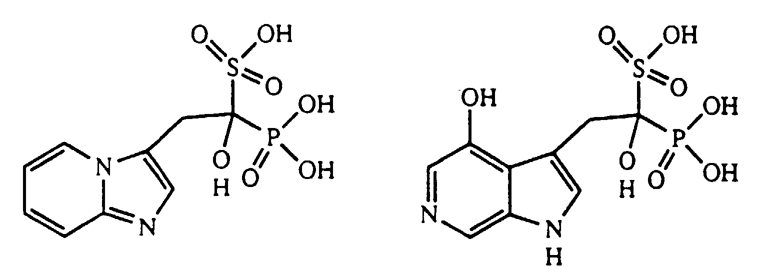 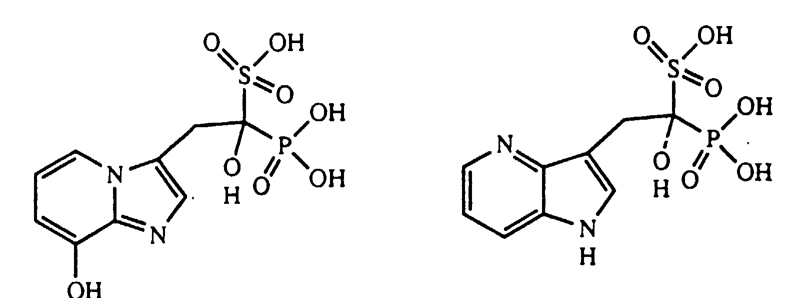 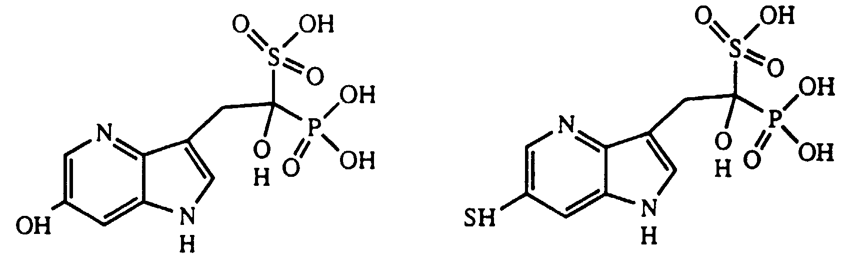 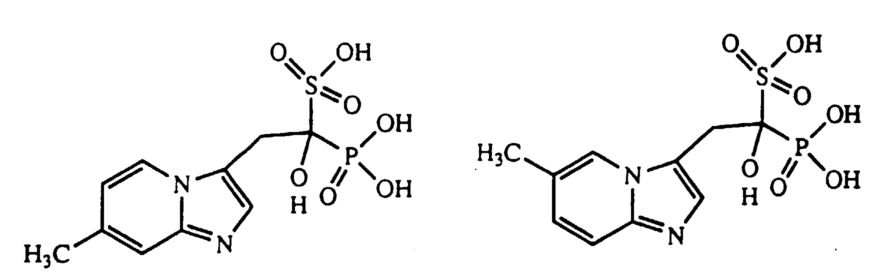 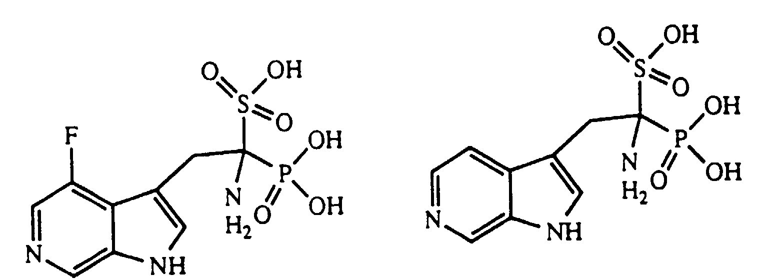 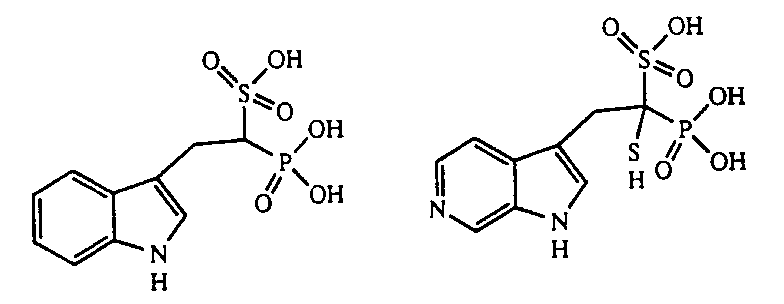 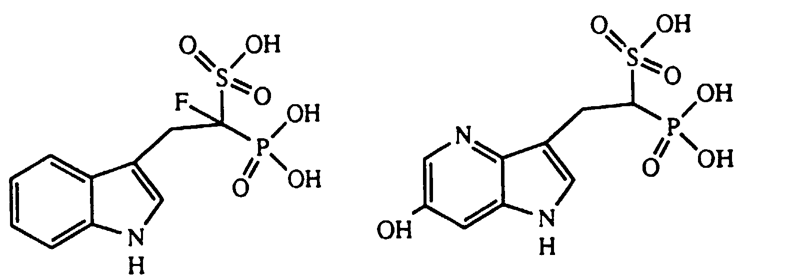 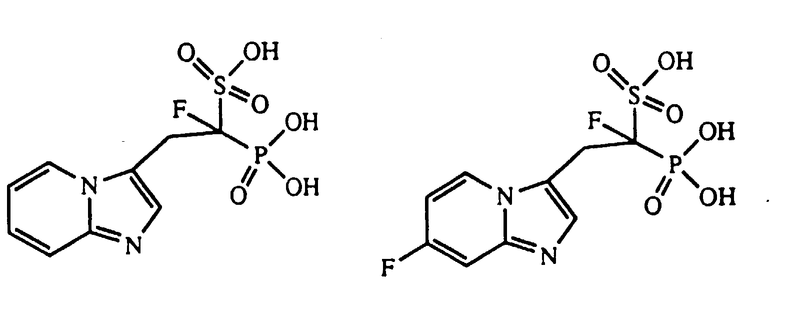 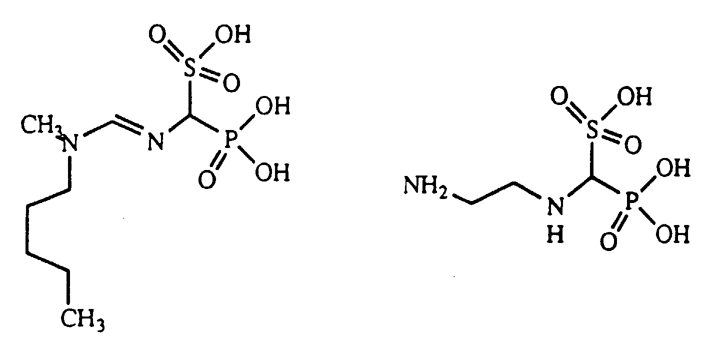 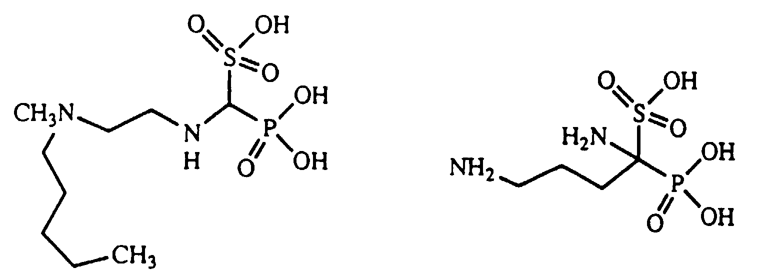 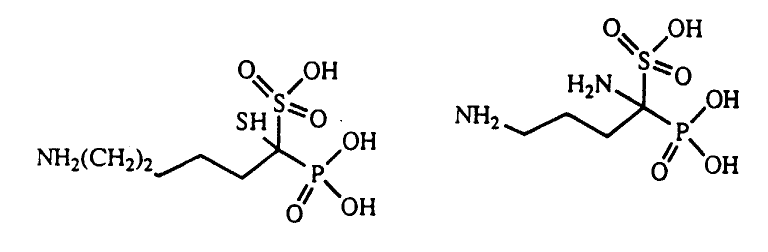 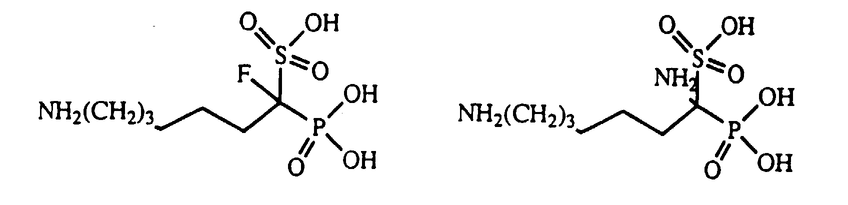 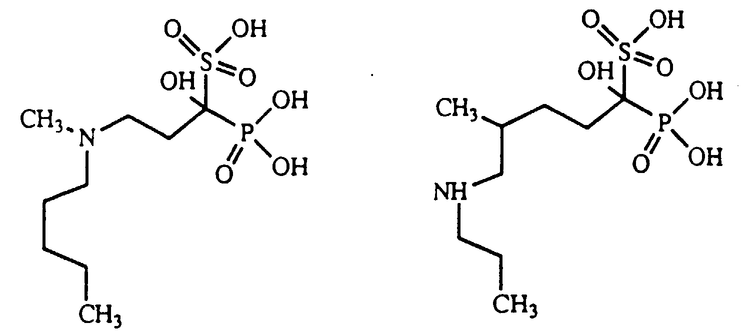 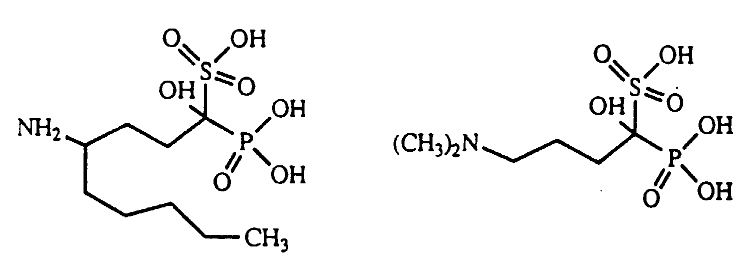 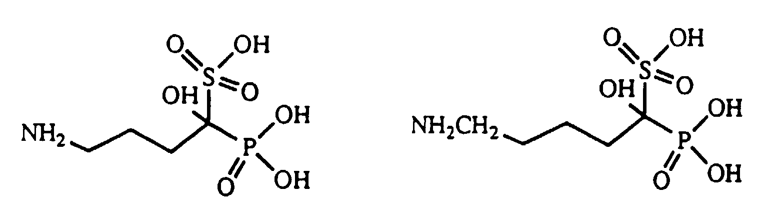 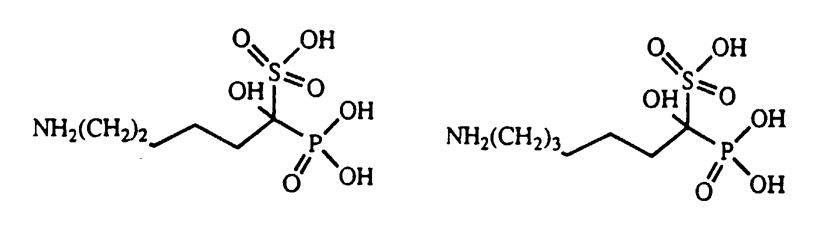 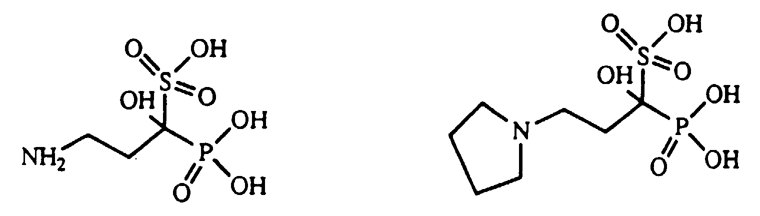 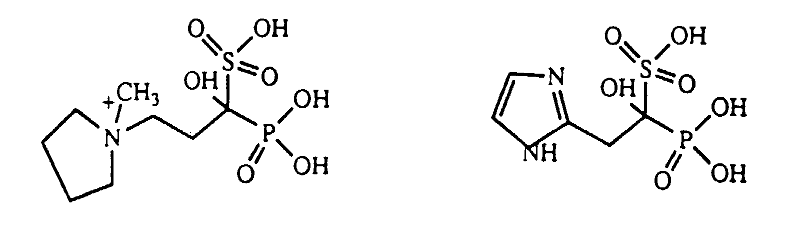 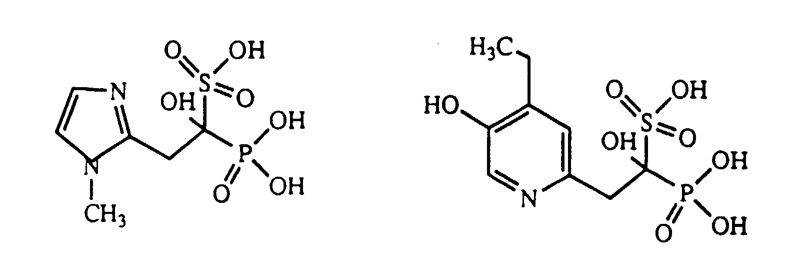 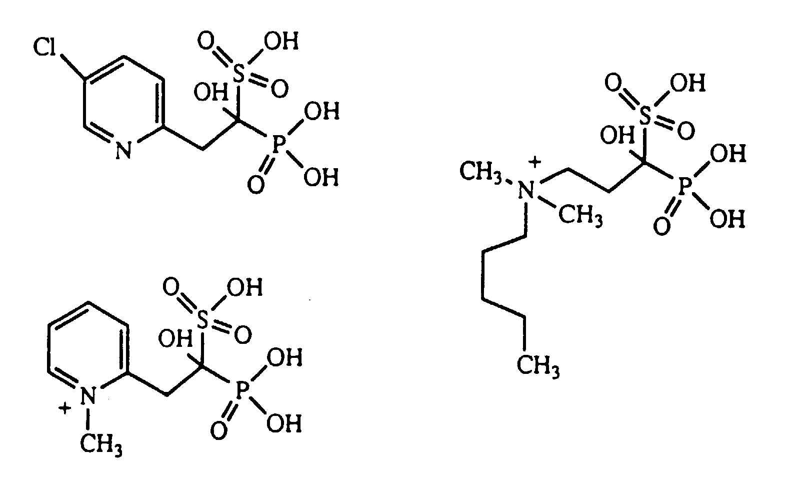 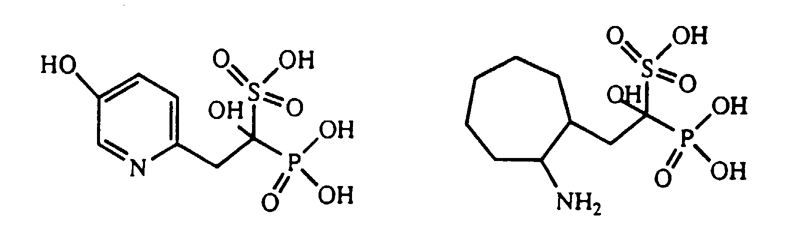 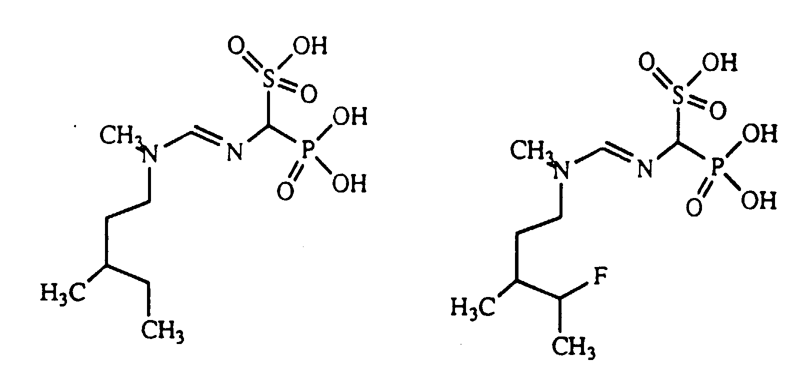 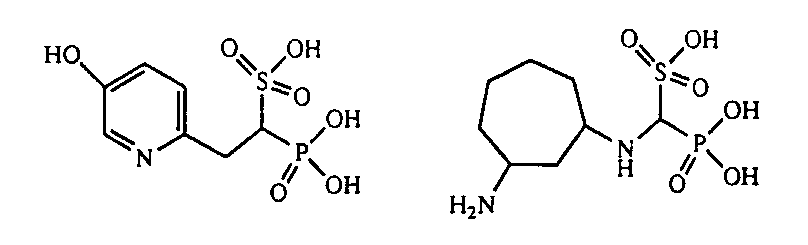 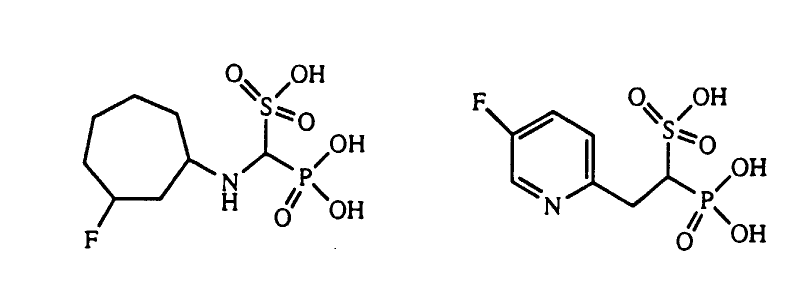 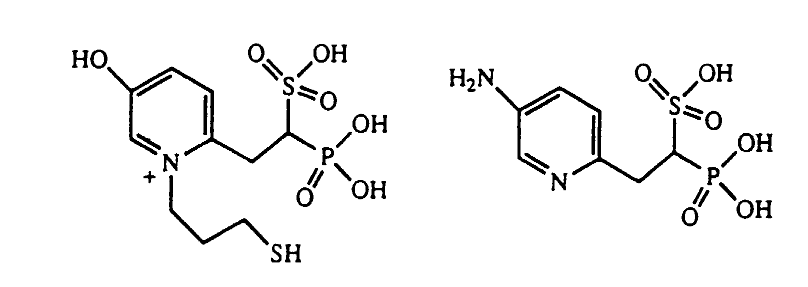 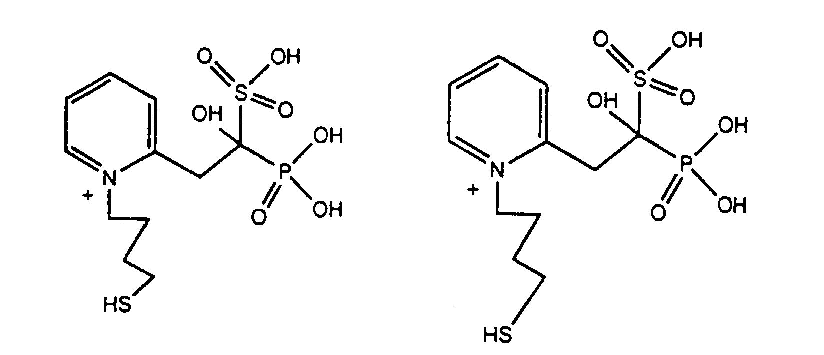 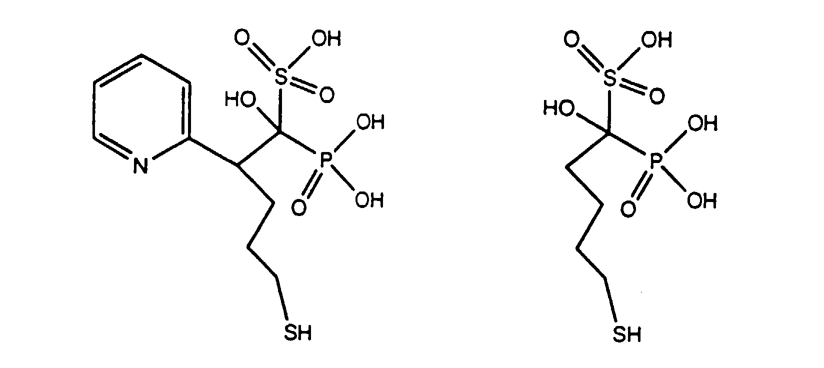 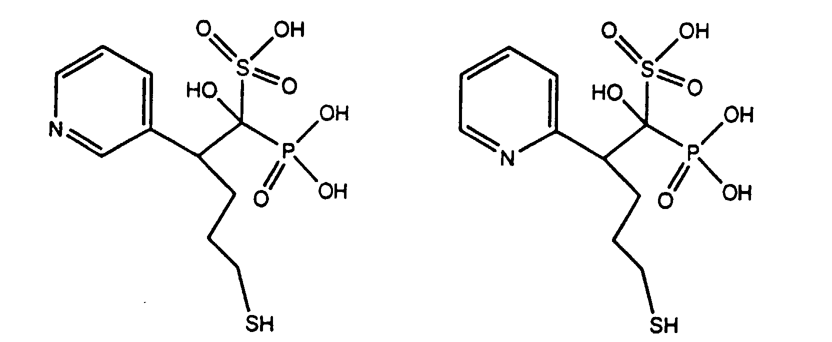 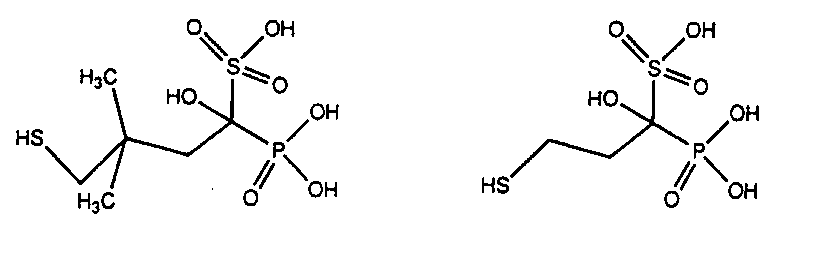 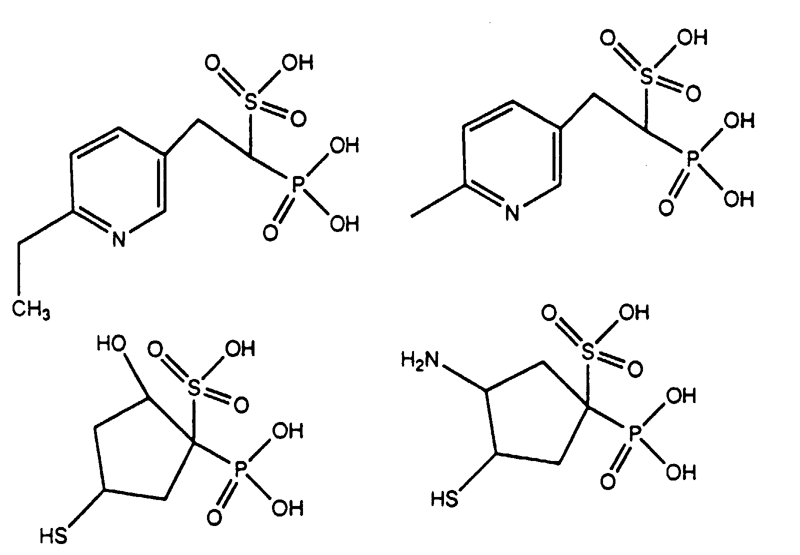 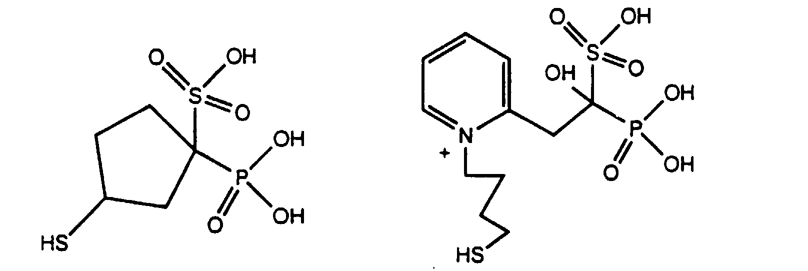 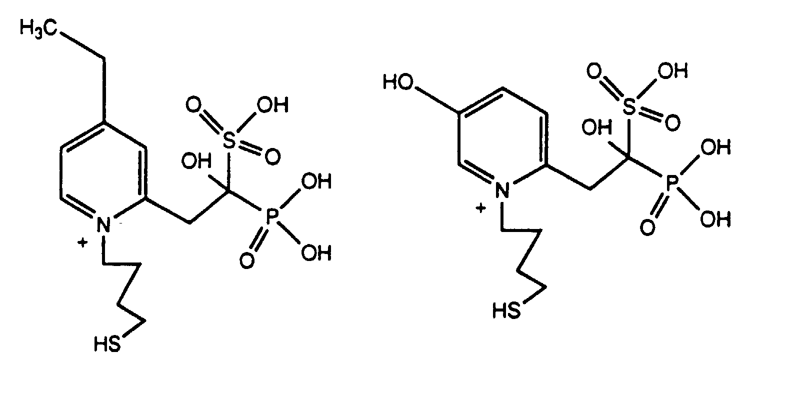 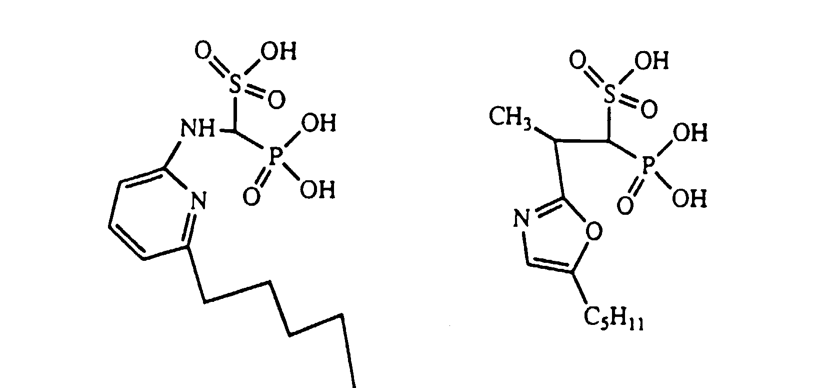 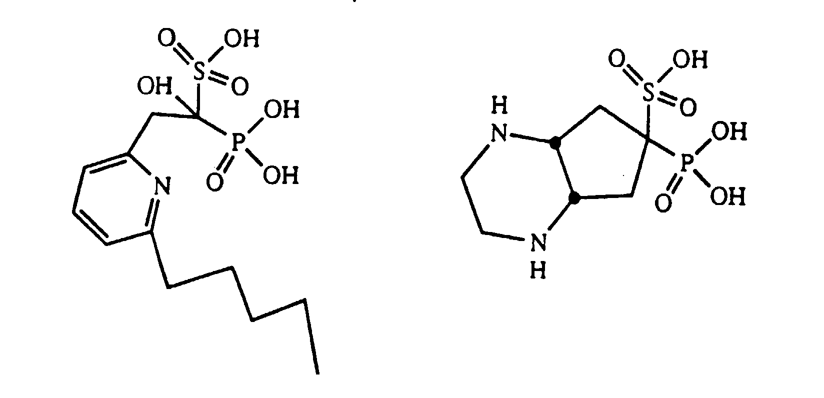 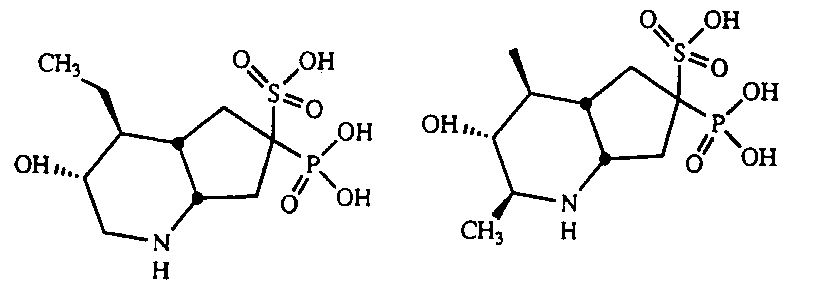 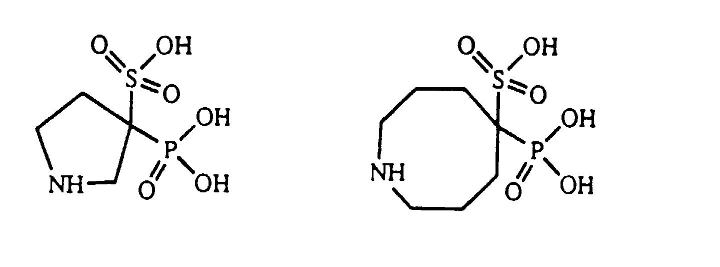 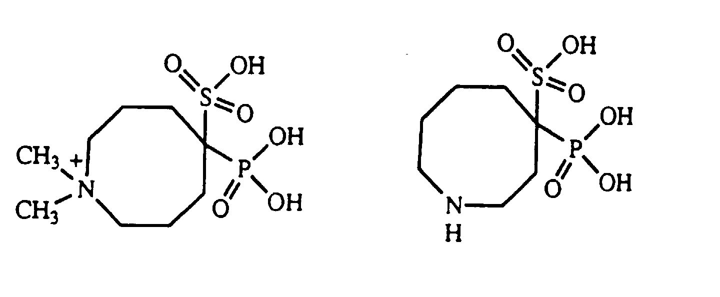 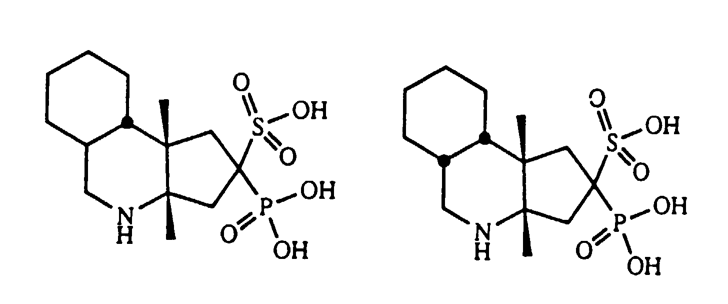 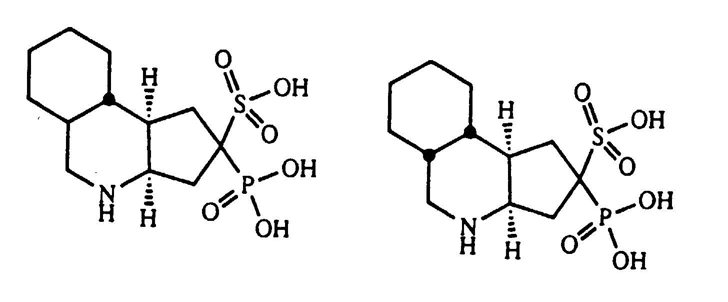 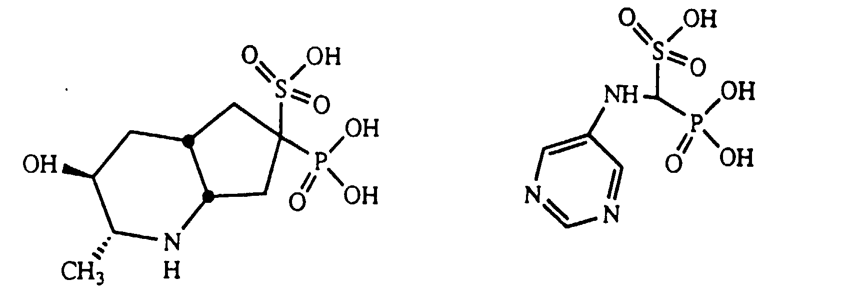 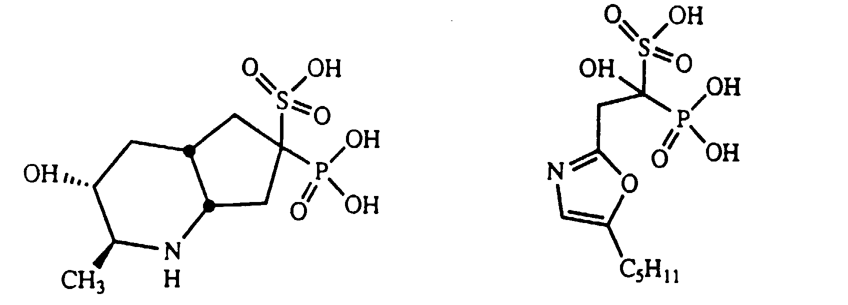 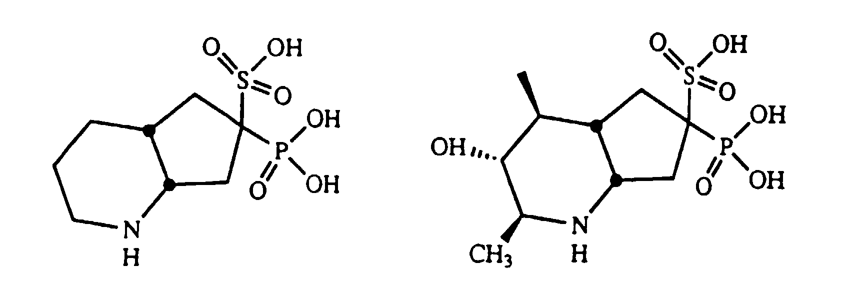 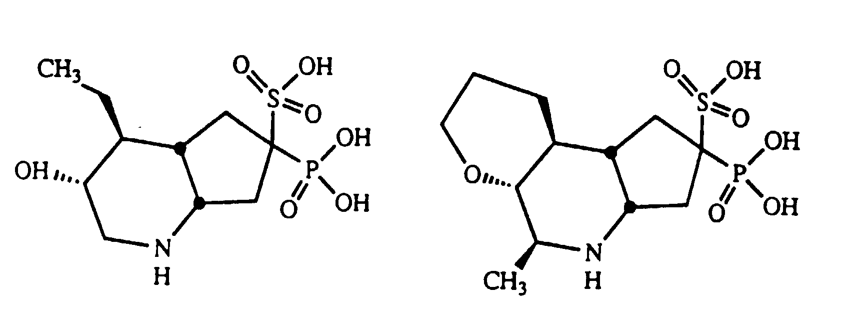 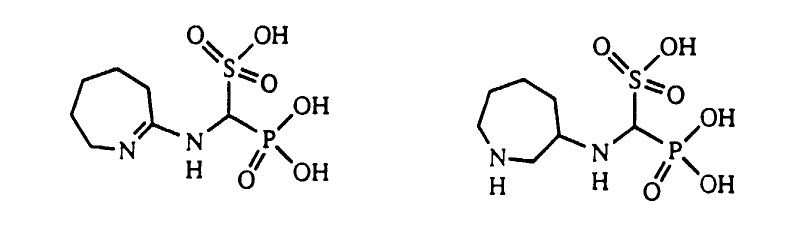 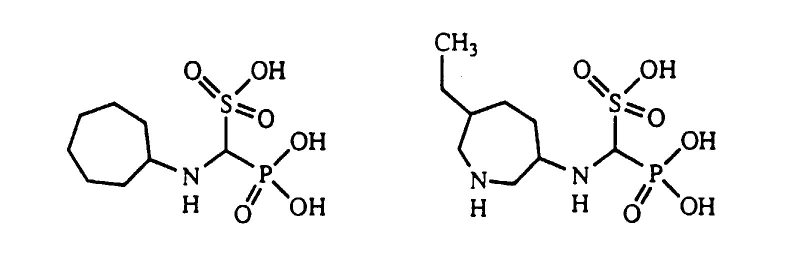 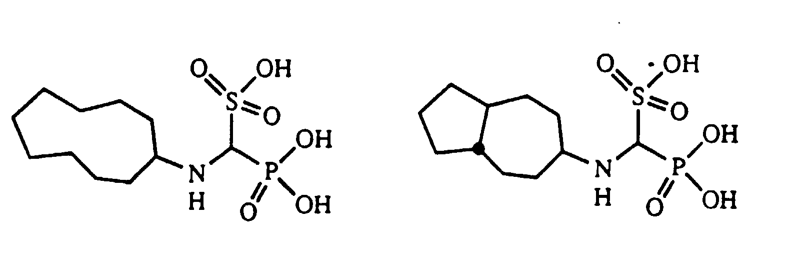 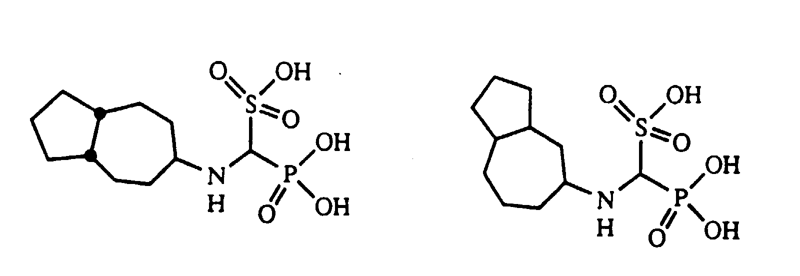 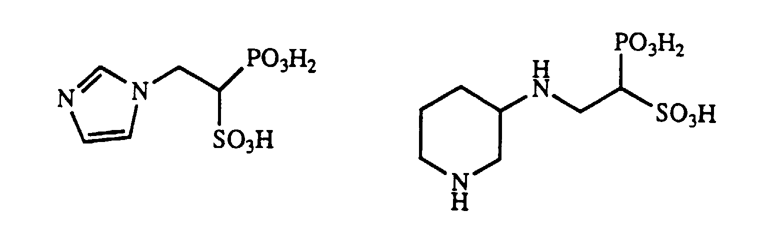 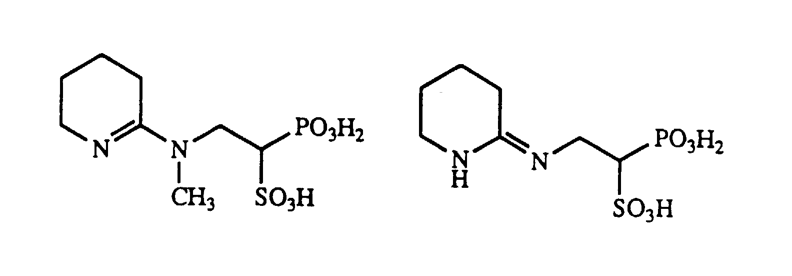 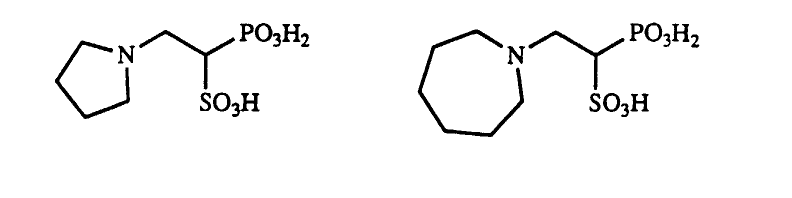 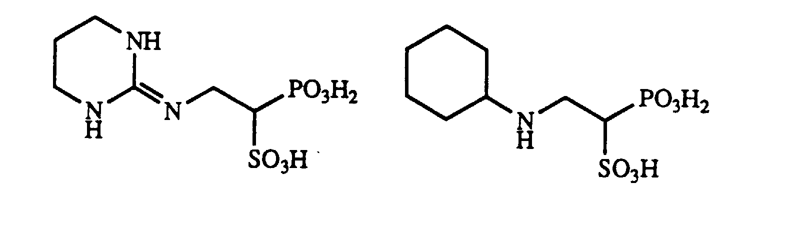 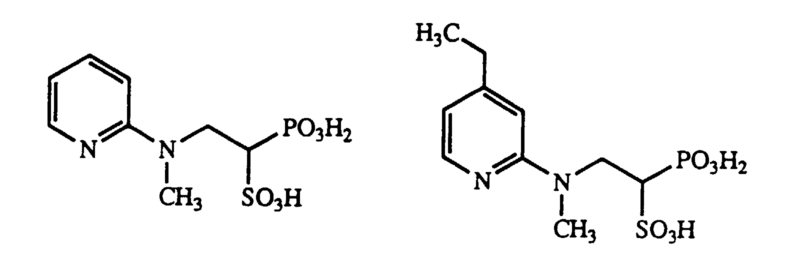 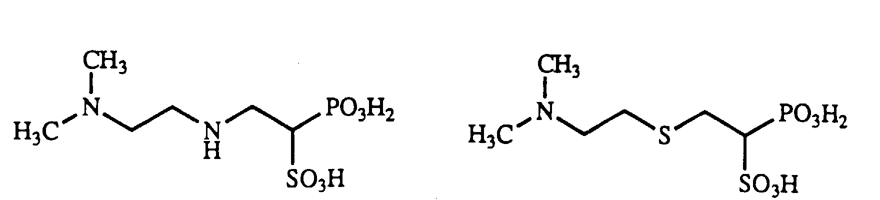 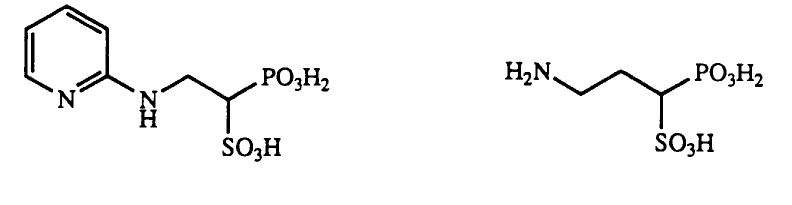 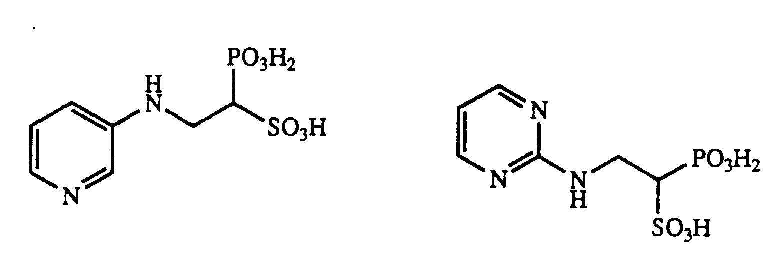 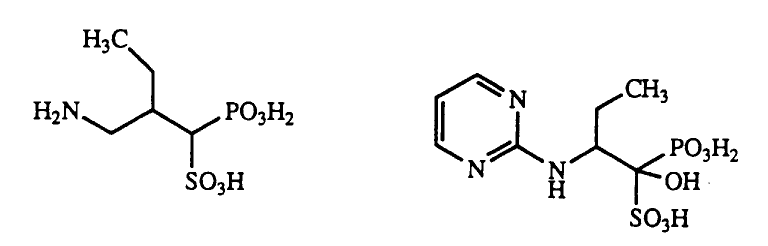 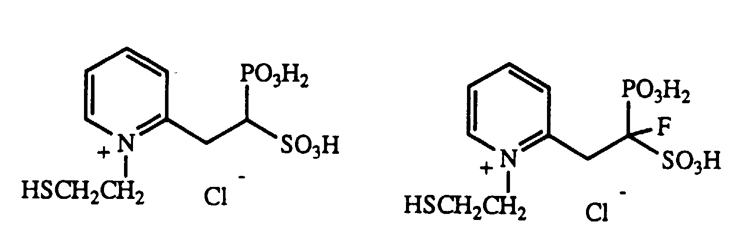 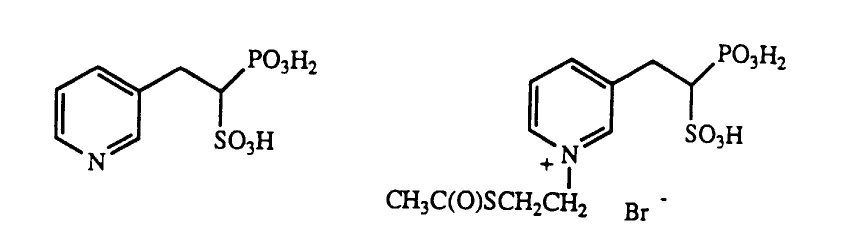 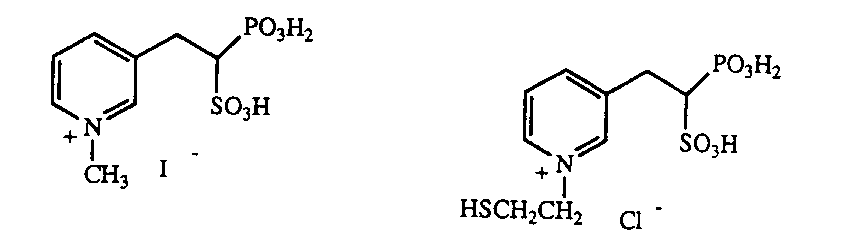 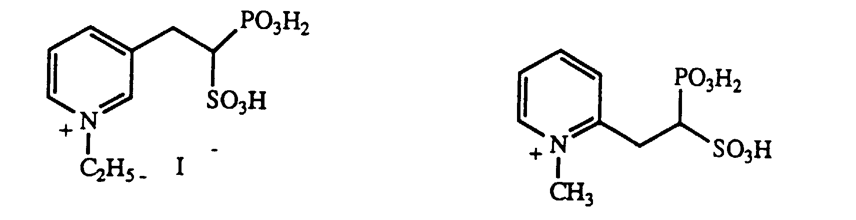 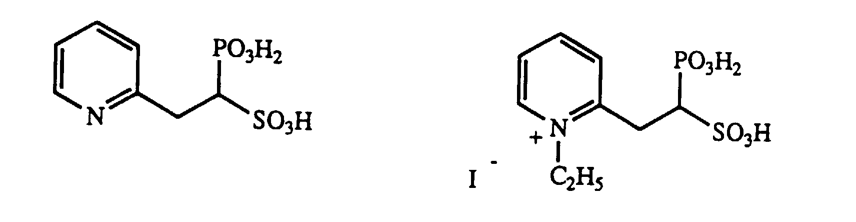 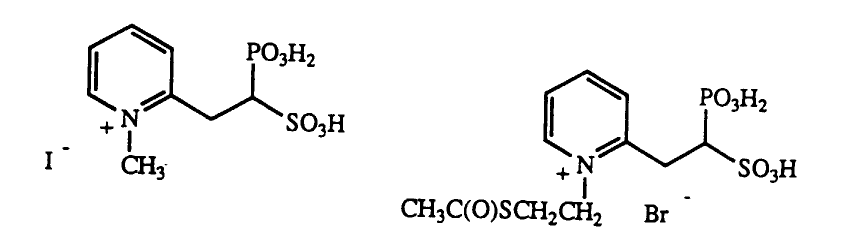 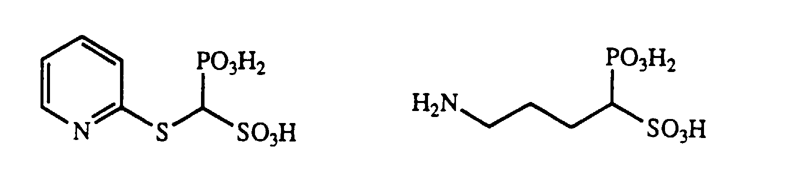 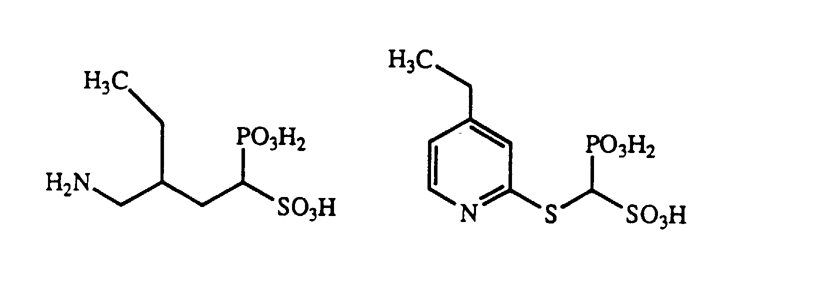 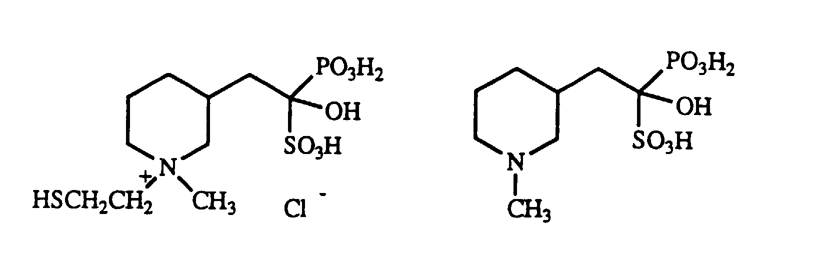 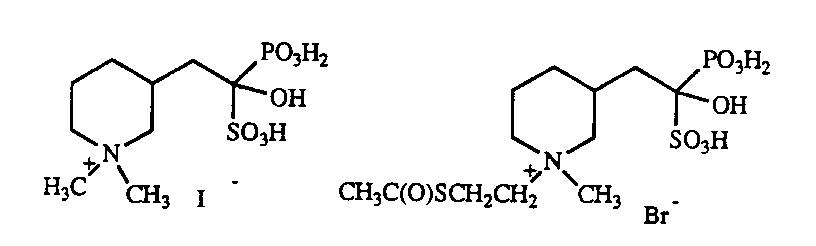 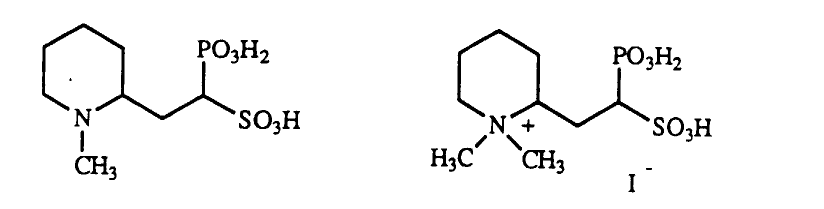 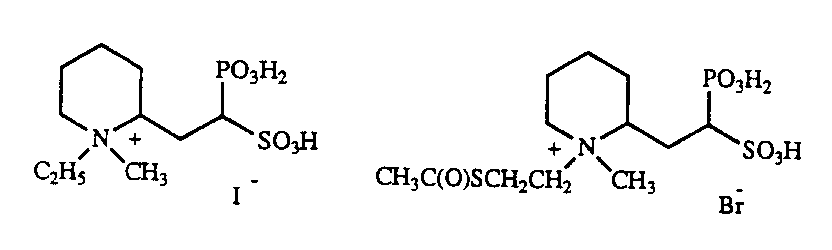 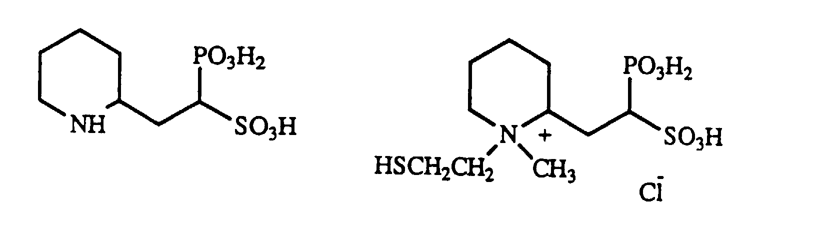 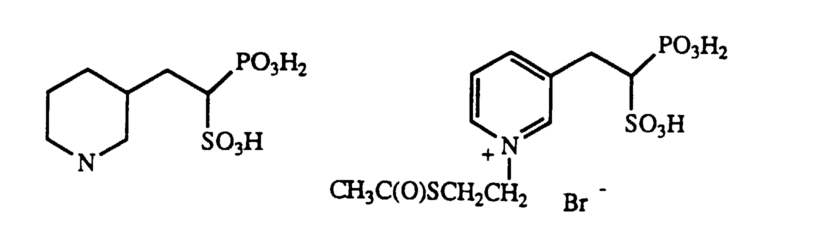 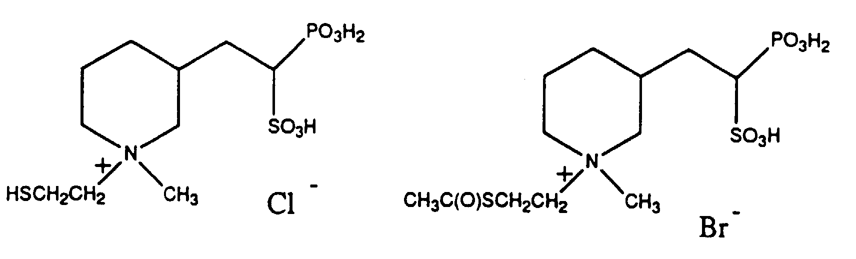 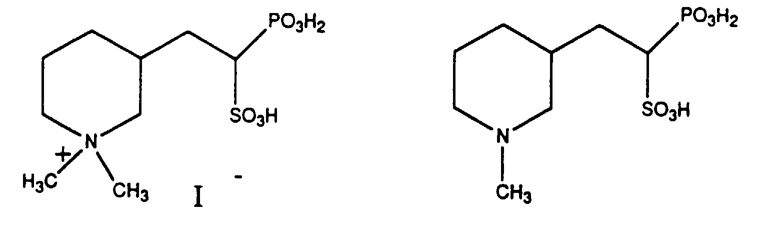 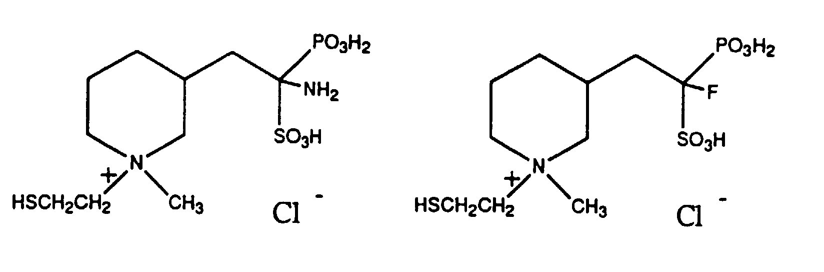 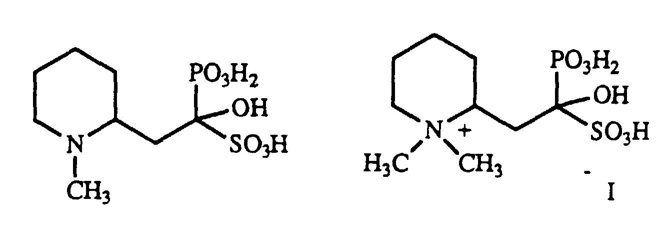 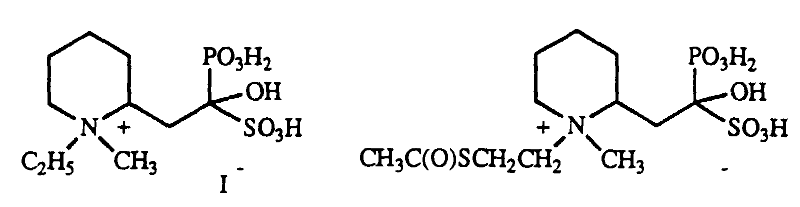 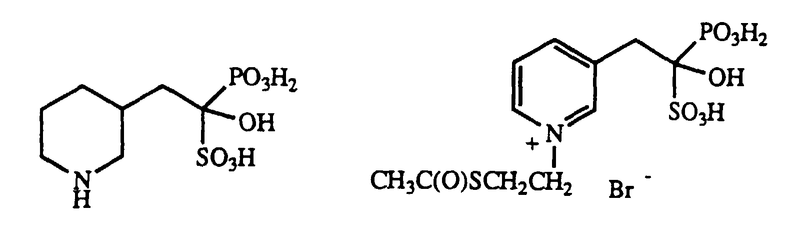 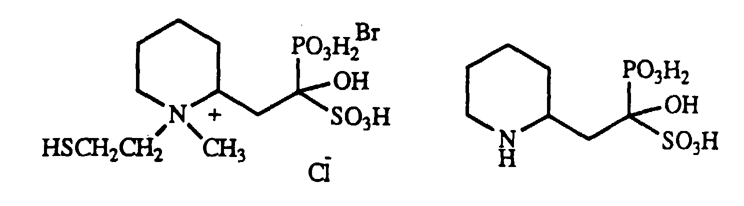 and the pharmaceutically-acceptable salts and esters thereof.

The term "pharmaceutically-acceptable salts and esters", as used herein, means hydrolyzable esters and salts of the phosphonosulfonate compounds which have the same general pharmacological properties as the acid form from which they are derived, and which are acceptable from a toxicity viewpoint. Pharmaceutically acceptable salts include alkali metals (e.g., sodium and potassium), alkaline earth metals (e.g. , calcium and magnesium), non-toxic heavy metals (e.g. , stanous and indium), and ammonium and low molecular weight substituted ammonium (e.g., mono-, di- and triethanolamine) salts. Preferred compounds are the sodium, potassium, and ammonium salts.

In order to determine and assess pharmacological activity, testing of the phosphonosulfonate compounds in animals is carried out using various assays known to those skilled in the art. Thus, the in vivo bone antiresorptive activity may be conveniently demonstrated using an assay designed to test the ability of these compounds to inhibit the resorption of bone, which bone resorption is characteristic of abnormal calcium and phosphate metabolism. One such test known to those skilled in the art is the Schenk model. Another useful art-known test is the adjuvant arthritis test. Also useful is the in vitro hydroxyapatite crystal growth inhibition test. These and other appropriate tests for pharmacological activity are disclosed and/or referred to in Shinoda et al., Calcified Tissue International, 35, pp 87-99 (1983); Schenk et al., Calcified Tissue Research, 11, pp 196-214 (1973); Russell et al., Calcified Tissue Research, 6, pp 183-196 (1970); Muhlbauer and Fleisch, Mineral Electrolyte Metab., 5, pp 296-303 (1981); Nancollas et al., Oral Biol., 15, 731 (1970); U.S. Patent 3,683,080, to Francis, issued August 8, 1972;- U. S. Patent 4,134,969, to Schmidt-Dunker, issued January 16, 1979; and EPO Patent Application Publication No. 189,662, published August 6, 1986; the disclosures of all these articles and patent specifications being incorporated herein by reference in their entirety. Certain of these tests for pharmacological activity are also described in more detail in the Examples provided hereinafter.

In addition to being useful for treating or preventing pathological conditions characterized by abnormal calcium or phosphate metabolism, the compounds of the present invention may have other uses. For example, the compounds of the present invention are believed to be useful as bone scanning agents after labeling with 99m-technetium. In addition, the compounds of the present invention are useful as sequestering agents for polyvalent metal ions, particularly di-(e.g. calcium and magnesium) and trivalent (e.g. indium) metal ions. Thus, the compounds of the present invention are useful as builders in detergents and cleansers, or for treating water. They are also useful as stabilizers for compounds. In addition, they may be useful in preventing the formation of tartar (i.e., calculus) and/or plaque on teeth. Finally, the compounds of the present invention may be useful as herbicides which are non-toxic to animals.

The phosphonosulfonate compounds of the present invention are prepared from commercially-available materials according to non-limiting Examples 1 to 61. Generally, the synthesis reaction may be carried out in the following way: In a first step, a compound containing a carbon geminally substituted with a phosphono group and a sulfono group (for example methyl 1-dimethoxyphosphinylethenesulfonate or diethoxyphosphinylmethanesulfonate lithium salt) is coupled with a second compound to produce a product with a new group off of the geminally substituted carbon. If so desired, the geminally substituted carbon can be hydroxylated in a second step. In a third step, any phosphonic and sulfonic esters are removed. In a fourth step, if saturated heterocyclic rings are desired, hydrogenation is carried out. If quaternary amines are desired, these may be produced in a fifth step by reaction with alkylating reagents. Finally, if different salts are desired these may be prepared, for example, by conversion with ion exchange resins in the proper salt form.

### Compositions Containing Novel Phosphonosulfonate Compounds

The phosphonosulfonate compounds of the present invention may be administered to humans or other mammals by a variety of routes, including, but not limited to, oral dosage forms and injections (intravenous, intramuscular, intraperitoneal and subcutaneous). Numerous other dosage forms containing the novel phosphonosulfonate compounds of the present invention can be readily formulated by one skilled in the art, utilizing the suitable pharmaceutical excipients as defined below. For considerations of patient compliance, oral dosage forms are generally most preferred.

The term "pharmaceutical composition" as used herein means a combination comprised of a safe and effective amount of the phosphonosulfonate compound active ingredient, or mixtures thereof, and pharmaceutically-acceptable excipients.

The phrase "safe and effective amount", as used herein, means an amount of a compound or composition great enough to significantly positively modify the symptoms and/or condition to be treated, but small enough to avoid serious side effects (at a reasonable benefit/risk ratio), within the scope of sound medical judgment. The safe and effective amount of active ingredient for use in the pharmaceutical compositions to be used in the method of the invention herein will vary with the particular condition being treated, the age and physical condition of the patient being treated, the severity of the condition, the duration of the treatment, the nature of concurrent therapy, the particular active ingredient being employed, the particular pharmaceutically-acceptable excipients utilized, and like factors within the knowledge and expertise of the attending physician.

The term "pharmaceutically-acceptable excipients" as used herein includes any physiologically inert, pharmacologically inactive material known to one skilled in the art, which is compatible with the physical and chemical characteristics of the particular phosphonosulfonate compound active ingredient selected for use. Pharmaceutically-acceptable excipients include, but are not limited to, polymers, resins, plasticizers, fillers, binders, lubricants, glidants, disintegrants, solvents, co-solvents, buffer systems, surfactants, preservatives, sweetening agents, flavoring agents, pharmaceutical grade dyes or pigments, and viscosity agents.

The term "oral dosage form" as used herein means any pharmaceutical composition intended to be systemically administered to an individual by delivering said composition to the gastrointestinal tract of an individual, via the mouth of said individual. For purposes of the present invention, the delivered form can be in the form of a tablet, coated or non-coated; solution; suspension; or a capsule, coated or non-coated.

The term "injection" as used herein means any pharmaceutical composition intended to be systemically administered to a human or other mammal, via delivery of a solution or emulsion containing the active ingredient, by puncturing the skin of said individual, in order to deliver said solution or emulsion to the circulatory system of the individual either by intravenous, intramuscular, intraperitoneal or subcutaneous injection.

Compounds of the present invention may comprise from about 0.1% to about 99.9% by weight of the pharmaceutical compositions of the present invention. Preferably the compounds of the present invention comprise from about 20% to about 80% by weight of the pharmaceutical compositions of the present invention.

Accordingly, the pharmaceutical compositions of the present invention include from 15-95% of a phosphonosulfonate compound active ingredient, or mixture, thereof; 0-2% flavoring agents; 0-50% co-solvents; 0-5% buffer system; 0-2% surfactants; 0-2% preservatives; 0-5% sweeteners; 0-5% viscosity agents; 0-75% fillers; 0.5-2% lubricants; 1-5% glidants; 4-15% disintegrants; and 1-10% binders.

Suitable pharmaceutical compositions are described herein in Examples 62-64. It is well within the capabilities of one skilled in the art to vary the non-limiting examples described herein to achieve a broad range of pharmaceutical compositions.

The choice of a pharmaceutically-acceptable excipient to be used in conjunction with the phosphonate compounds of the present invention is basically determined by the way the phosphonate compound is to be administered. If the compound is to be injected, the preferred pharmaceutical carrier is sterile physiological saline, the pH of which has been adjusted to about 7.4. Suitable pharmaceutically-acceptable carriers for topical application include those suited for use in creams, gels, tapes and the like.

The pharmaceutically-acceptable carrier employed in conjunction with the phosphonosulfonate compounds of the present invention is used at a concentration sufficient to provide a practical size to dosage relationship. The pharmaceutically-acceptable carriers, in total, may comprise from 0.1% to 99.9% by weight of the pharmaceutical compositions of the present invention, and preferably from 20% to 80%.

The preferred mode of administering the phosphonosulfonate compound of the present invention is orally. The preferred unit dosage form is therefore tablets, capsules and the like, comprising a safe and effective amount of the phosphonate compound of the present invention. Preferably, the compositions comprise from about 1 mg P to about 600 mg P of a phosphonosulfonate compound of the present invention. Pharmaceutically-acceptable carriers suitable for the preparation of unit dosage forms for oral administration are well known in the art. Their selection will depend on secondary considerations like taste, cost, and shelf stability, which are not critical for the purposes of the present invention, and can be made without difficulty by a person skilled in the art.

The term "mg P", as used herein, means the weight of the phosphorus atom present in an amount of a phosphonosulfonate compound of the present invention. This unit is used to standardize the amount of the phosphonosulfonate compounds of the present invention to be used in the pharmaceutical compositions and methods of the present inventions. For example, 1-hydroxy-1-phosphono-2-(3-pyridinyl)ethanesulfonic acid has a molecular weight of 283 g/mole, of which 11% (31 g/mole) is due to the phosphorus atom present in this molecule. One milligram of this compound is therefore calculated to have 0.11 mg P. Thus, to prepare a pharmaceutical composition containing 0.11 mgP of this compound, the composition should contain 1 mg of the compound; and to dose 0.11 mg P/kg of this compound to a 50 kg patient, the patient would be dosed with 50 mg of this compound.

The rate of systemic delivery can be satisfactorily controlled by one skilled in the art, by manipulating any one or more of the following:
(a) the active ingredient;
(b) the pharmaceutically-acceptable excipients; so long as the variants do not interfere in the activity of the particular active ingredient selected;
(c) the type of the excipient, and the concomitant desirable thickness and permeability (swelling properties) of said excipients;
(d) the time-dependent conditions of the excipient itself and/or within the excipients;
(e) the particle size of the granulated active ingredient; and
(f) the pH-dependent conditions of the excipients.

In particular, the solubility, acidity, and susceptibility to hydrolysis of the different phosphonosulfonate active ingredients, such as acid addition salts, salts formed with the carboxylic group, e.g., alkali metal salts, alkaline earth metal salts, etc., and esters, e.g., alkyl, alkenyl, aryl, aralkyl, may be used as guidelines for the proper choice. In addition, suitable pH-conditions might be established within the oral dosage forms by adding a suitable buffer to the active ingredient in accordance with the desired release pattern.

As stated hereinabove, pharmceutically-acceptable excipients include, but are not limited to, resins, fillers, binders, lubricants, solvents, glidants, disintegrants co-solvents, surfactants, preservatives, sweetener agents, flavoring agents, buffer systems, pharmaceutical-grade dyes or pigments, and viscosity agents.

The preferred solvent is water.

Flavoring agents among those useful herein include those described in Remington's Pharmaceutical Sciences, 18th Edition, Mack Publishing Company, 1990, pp. 1288-1300, incorporated by reference herein. The pharmaceutical compositions suitable for use herein generally contain from 0-2% flavoring agents.

Dyes or pigments among those useful herein include those described in Handbook of Pharmaceutical Excipients, pp. 81-90, 1986 by the American Pharmaceutical Association & the Pharmaceutical Society of Great Britain, incorporated by reference herein. The pharmaceutical compositions herein generally contain from 0-2% dyes or pigments.

Preferred co-solvents include, but are not limited to, ethanol, glycerin, propylene glycol, polyethylene glycols. The pharmaceutical compositions of the present invention include from 0-50% co-solvents.

Preferred buffer systems include, but are not limited to, acetic, boric, carbonic, phosphoric, succinic, malaic, tartaric, citric, acetic, benzoic, lactic, glyceric, gluconic, glutaric and glutamic acids and their sodium, potassium and ammonium salts. Particularly preferred are phosphoric, tartaric, citric, and acetic acids and salts. The pharmaceutical composition of the present invention generally contain from 0-5% buffer systems.

Preferred surfactants include, but are not limited to, polyoxyethylene sorbitan fatty acid esters, polyoxyethylene monoalkyl ethers, sucrose monoesters and lanolin esters and ethers, alkyl sulfate salts, sodium, potassium, and ammonium salts of fatty acids. The pharmaceutical compositions of the present invention include 0-2% surfactants.

Preferred preservatives include, but are not limited to, phenol, alkyl esters of parahydroxybenzoic acid, o-phenylphenol benzoic acid and the salts thereof, boric acid and the salts thereof, sorbic acid and the salts thereof, chlorobutanol, benzyl alcohol, thimerosal, phenylmercuric acetate and nitrate, nitromersol, benzalkonium chloride, cetylpyridinium chloride, methyl paraben, and propyl paraben. Particularly preferred are the salts of benzoic acid, cetylpyridinium chloride, methyl paraben and propyl paraben. The compositions of the present invention generally include from 0-2% preservatives.

Preferred sweeteners include, but are not limited to, sucrose, glucose, saccharin, sorbitol, mannitol, and aspartame. Particularly preferred are sucrose and saccharin. Pharmaceutical compositions of the present invention include 0-5% sweeteners.

Preferred viscosity agents include, but are not limited to, methylcellulose, sodium carboxymethylcellulose, hydroxypropyl methylcellulose, hydroxypropylcellulose, sodium alginate, carbomer, povidone, acacia, guar gum, xanthan gum and tragacanth. Particularly preferred are methylcellulose, carbomer, xanthan gum, guar gum, povidone, sodium carboxymethylcellulose, and magnesium aluminum silicate. Compositions of the present invention include 0-5% viscosity agents.

Preferred fillers include, but are not limited to, lactose, mannitol, sorbitol, tribasic calcium phosphate, dibasic calcium phosphate, compressible sugar, starch, calcium sulfate, dextro and microcrystalline cellulose. The compositions of the present invention contain from 0-75% fillers.

Preferred lubricants include, but are not limited to, magnesium stearate, stearic acid, and talc. The pharmaceutical compositions of the present invention include 0.5-2% lubricants.

Preferred glidants include, but are not limited to, talc and colloidal silicon dioxide. The compositions of the present invention include from 1-5% glidants.

Preferred disintegrants include, but are not limited to, starch, sodium starch glycolate, crospovidone; croscarmelose sodium, and microcrystalline cellulose. The pharmaceutical compositions of the present invention include from 4-15% disintegrants.

Preferred binders include, but are not limited to, acacia, tragacanth, hydroxypropylcellulose, pregelatinized starch, gelatin, povidone, hydroxypropylcellulose, hydroxypropylmethylcellulose, methylcellulose, sugar solutions, such as sucrose and sorbitol, and ethylcellulose. The compositions of the present invention include 1-10% binders.

### Method for Treating or Preventing Diseases Characterized by Abnormal Calcium and Phosphate Metabolism

The compounds of the present invention are used in methods for treating or preventing diseases characterized by abnormal calcium and phosphate metabolism. Such methods comprise administering to a human or lower animal in need of such treatment a safe and effective amount of a phosphonosulfonate compound of the present invention.

The preferred mode of administration is oral, but other known methods of administration are contemplated as well, e.g., dermato mucosally (for example, dermally, rectally and the like) and parenterally (for example, by subcutaneous injection, intramuscular injection, intra-articular injection, intravenous injection and the like). Inhalation is also included. Thus, specific modes of administration include, without limitation, oral, transdermal, mucosal, sublingual, intramuscular, intravenous, intraperitoneal, and sub cutaneous administration, as well as topical application.

The term "abnormal calcium and phosphate metabolism", as used herein, means (1) conditions which are characterized by anomalous mobilization of calcium and phosphate leading to general or specific bone loss, or excessively high calcium and phosphate levels in the fluids of the body; and (2) conditions which cause or result from deposition of calcium and phosphate anomalously in the body. The first category includes, but is not limited to, osteoporosis, Paget's disease, hyperparathyroidism, hypercalcemia of malignancy, heterotopic ossification, and osteolytic bone metastases. The second category includes, but is not limited to, myositis ossificans progressiva, calcinosis universalis, and such afflictions as arthritis, osteoarthritis, neuritis, bursitis, tendonitis and other inflammatory conditions which predispose involved tissue to deposition of calcium phosphates.

The term "rheumatoid arthritis" as used herein, means a chronic systemic and articular inflammatory disorder of unknown etiology. It is characterized by destruction of articular cartilage, ligaments, tendons, and bone.

The term "osteoarthritis" as used herein, means a non-inflammatory disorder of the movable joints. It is characterized by deterioration and abrasion of the articular cartilage; and new bone formation at the joint surface.

The terms "person at risk" and "person in need of such treatment", as used herein, mean any human or lower animal which suffers a significant risk of abnormal calcium and phosphate metabolism if left untreated, and any human or lower animal diagnosed as being afflicted with abnormal calcium and phosphate metabolism. For example, postmenopausal women; persons undergoing certain steroid therapy; persons on certain anticonvulsant drugs; persons diagnosed as having Paget's disease, hyperparathyroidism, hypercalcemia of malignancy, or osteolytic bone metastases; persons diagnosed as suffering from one or more of the various forms of osteoporosis; persons belonging to a population group known to have a significantly higher than average chance of developing osteoporosis, e.g., postmenopausal women, men over age 65, and persons being treated with drugs known to cause osteopetrosis as a side effect; persons diagnosed as suffering from myositis ossificans progressiva or calcinosis universalis; and persons afflicted with arthritis, osteoarthritis, neuritis, bursitis, tendonitis and other inflammatory conditions which predispose involved tissue to deposition of calcium phosphate.

The phrase "safe and effective amount", as used herein, means an amount of a compound or composition high enough to significantly positively modify the condition to be treated, but low enough to avoid serious side effects (at a reasonable benefit/risk ratio), within the scope of sound medical judgment. The safe and effective amount of phosphonosulfonate compounds of the present invention will vary with the particular condition being treated, the age and physical condition of the patient being treated, the severity of the condition, the duration of the treatment, the nature of concurrent therapy, the specific diphosphonate employed, the particular pharmaceutically-acceptable carrier utilized, and like factors within the knowledge and expertise of the attending physician. However, single dosages can range from 0.01 mg P to 3500 mg P, or from 0.0002 to 70 mg P/kg of body weight (based on a body weight of 50 kg). Preferred single dosages are from 1 mg P to 600 mg P, or from 0.02 to 12 mg P/kg of body weight (based on a body weight of 50 kg). Up to four single dosages per day may be administered. Daily dosages greater than 500 mg P/kg are not required to produce the desired effect and may produce undesirable side effects. The higher dosages within this range are, of course, required in the case of oral administration because of limited absorption.

### Example 1

### Synthesis of 2-(1-Imidazolyl)-1-phosphonoethanesulfonic Acid

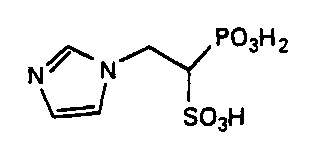

A mixture of 68 g (.01 mole) of imidazole and 2.30 g (.01 mole) of methyl 1-dimethoxyphosphinylethenesulfonate (U.S. Patent No. 5,011,938 issued to Barnett et al. on April 30, 1991) in 20 ml of chloroform is stirred at 20-50° for one day. The reaction is cooled to room temperature, and 10.7 g (.07 mole) of bromotrimethylsilane is added. The mixture is stirred at 20-30° for 2-3 days, and to it is then added 20 ml water. The mixture is stirred for about 30 minutes, and the layers are separated. The water layer is extracted several times with CHCl₃ (extracts are discarded) and is evaporated to dryness under vacuum. The residue is triturated with acetone to give a solid, which is collected by filtration and recrystallized from water/acetone to afford 2-(1-imidazolyl)-1-phosphonoethanesulfonic acid.

### Example 2

### Synthesis of 1-Phosphono-2-(3-pyridylamino)ethanesulfonic Acid

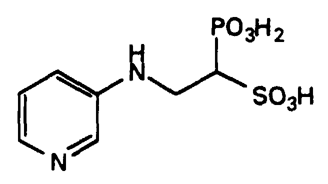

This compound is synthesized in the manner of Example 1, starting with 3-aminopyridine.

### Example 3

### Synthesis of 1-Phosphono-2-(3-piperidinylamino)ethanesulfonic Acid

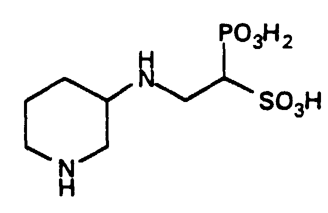

A mixture of 1 g of 1-phosphono-2-(3-pyridinylamino)ethanesulfonic acid and .5 g of palladium on charcoal catalyst in 50 ml of distilled water is hydrogenated on a Parr apparatus at 40 PSI for about 2 days. The catalyst is removed by filtration, and the filtrate is concentrated to a few mls. Ethanol is added slowly to precipitate a solid, which is recrystallized from water/ethanol to afford 1-phosphono-2-(3-piperidinylamino)ethanesulfonic acid.

### Example 4

### Synthesis of 1-Phosphono-2-(2-pyridinylamino)ethanesulfonic Acid

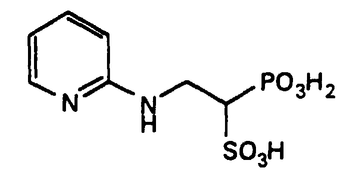

This compound is synthesized in the manner of Example 1, starting with 2-aminopyridine.

### Example 5

### Synthesis of 1-Phosphono-2-((2-piperidinylidene)amino)ethanesulfonic Acid

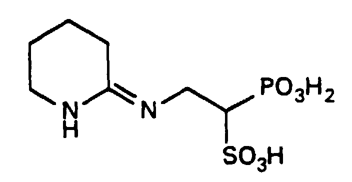

This compound is synthesized by the procedure of Example 3, starting with 1-phosphono-2-(2-pyridinylamino)ethanesulfonic acid.

### Example 6

### Synthesis of 1-Phosphono-2-(2-pyrimidinylamino)ethanesulfonic Acid

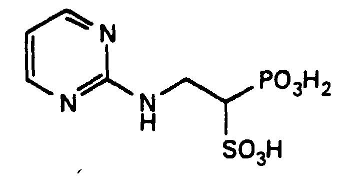

This compound is synthesized in the manner of Example 1, starting with 2-aminopyrimidine.

### Example 7

Synthesis of 1-Phosphono-2((1,3-diazacyclohexane-2-ylidene)amino)ethane-sulfonic Acid

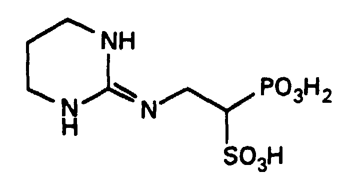

This compound is synthesized by the procedure of Example 3, starting with 1-phosphono-2-(2-pyrimidinylamino)ethanesulfonic acid.

### Example 8

### Synthesis of 1-Phosphono-2-(N-methyl(2-pyridinyl)amino)ethanesulfonic Acid

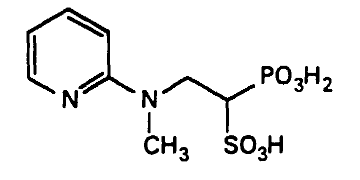

This compound is synthesized in the manner of Example 1, starting with 2-methylaminopyridine.

### Example 9

### Synthesis of 2-[(N-methyl)(3,4,5,6-tetrahydropyridin-2-yl)amino]-1-phosphonoethanesulfonic Acid

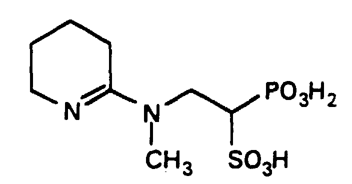

This compound is synthesized by the procedure of Example 3, starting with 2-[N-methyl(2-pyridinyl)amino]-1-phosphonoethanesulfonic acid.

### Example 10

### Synthesis of 1-Phosphono-2-(1-pyrrolidinyl)ethanesulfonic Acid

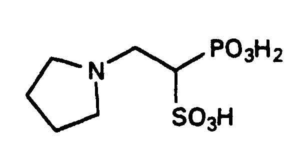

This compound is synthesized by the method of Example 1, starting with pyrrolidine.

### Example 11

### Synthesis of 2-(Hexahydro-1H-azepin-1-yl)-1-(phosphono)ethanesulfonic Acid

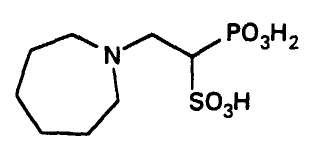

This compound is synthesized by the method of Example 1, starting with hexamethyleneimine.

### Example 12

### Synthesis of 2-(Cyclohexylamino)-1-phosphonoethanesulfonic Acid

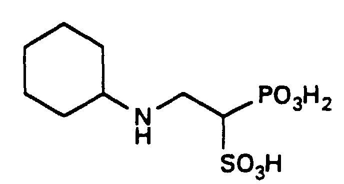

This compound is synthesized by the method of Example 1, starting with cyclohexylamine.

### Example 13

### Synthesis of 2-[2-(Dimethylamino)ethylamino]-1-phosphonoethanesulfonic Acid

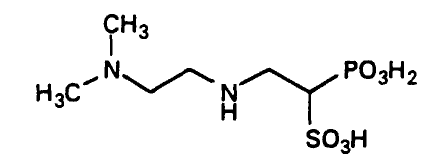

This compound is synthesized by the method of Example 1, starting with N,N-dimethylethylenediamine.

### Example 14

### Synthesis of 2-[2-(Dimethylamino)ethylthio]-1-phosphonoethanesulfonic Acid

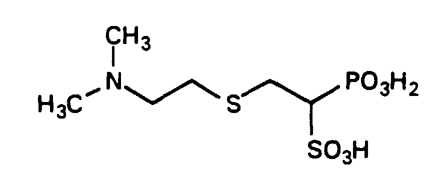

This compound is synthesized by the method of Example 1, starting with 2-dimethylaminoethanethiol.

### Example 15

### Synthesis of 3-Amino-1-phosphonopropanesulfonic Acid

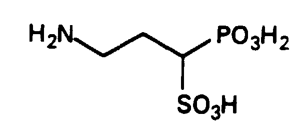

I. Synthesis of Methyl 2-Cyano-1-(dimethoxyphosphinyl)ethanesulfonate
   To a solution of 2.3 g (.01 mole) of methyl 1-dimethoxyphosphinylethylenesulfonate in a mixture of 20 ml ethanol and 10 ml water is added .60 g (.01 mole) of acetic acid followed by 1.3 g (.02 mole) of potassium cyanide. The mixture is stirred at room temperature for 1-5 hours, 20 ml more water is added, and the mixture is concentrated under vacuum on a rotary evaporator to get rid of the ethanol. The reaction is extracted several times with chloroform. The combined extracts are dried (MgSO₄) and the solvent is removed. The resulting oily residue is purified by flash chromatography on silica gel.
II. Synthesis of 2-Cyano-1-phosphonoethanesulfonate
   A solution of 2.57 g (.01 mole) of methyl 2-cyano-1-(dimethoxyphosphinyl)ethanesulfonate in 30 ml chloroform is treated with 10.7 g (.07 mole) of bromotrimethylsilane. The solution is stirred at room temperature for 2-3 days. Water (20 ml) is added, and the mixture is vigorously stirred for 30 minutes. The solvents are removed under vacuum on a rotary evaporator, and the residue is triturated with acetone to afford 2-cyano-1-phosphonoethanesulfonate.
III. Synthesis of 3-Amino-1-phosphonopropanesulfonic Acid
   The hydrogenation of 2-cyano-1-phosphonoethanesulfonate is carried out using the hydrogenation technique of Freifelder (J. Am. Chem. Soc., 82. 2386 (1960)). The cyano compound (2.15 g; .01 mole) is placed in 20 ml of 10% methanolic ammonia.. Rhodium on alumina (5%) catalyst (.5 g) is added, and the mixture is hydrogenated at 40 PSI on a Parr apparatus for several hours (until uptake of hydrogen is complete). The catalyst is filtered off, and the filtrate is evaporated dry. The product is purified by dissolving the residue in water, adding ethanol to give a precipitate, and collecting the solids by filtration. Further purification is accomplished by again recrystallizing from water/ethanol.

### Example 16

### Synthesis of 1-Phosphono-2-(3-pyridyl)ethanesulfonic Acid

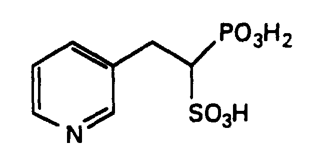

I. Synthesis of 1-Diethoxyphosphinyl-2-(3-pyridyl)ethanesulfonic Acid Lithium Salt
   A suspension of 2.38 g (.01 mole) of diethoxyphosphinylmethanesulfonate lithium salt (Carretero, et al.; Tetrahedron, 43, 5125 (1987)) in 50 ml of anhydrous tetrahydrofuran is stirred in a -40° bath under a dry nitrogen atmosphere. To this is added n-butyl lithium (4.4 ml of 2.5 M solution in hexanes; .011 mole) via syringe over 5 minutes. The reaction mixture is allowed to warm to -15°, and is stirred at this temperature for about one hour. It is then cooled to -78°, and to it is added dropwise over about 5 minutes a solution of 1.27 g (.01 mole) of 3-(chloromethyl)pyridine in 3 ml of anhydrous THF. (3-(chloromethyl)pyridine is prepared by dissolving a few grams of 3-picolylchloride hydrochloride in a minimum quantity of water and carefully adding excess solid K₂CO₃ until foaming stops and the water has been absorbed by the K₂CO₃ leaving the 3-picolylchloride floating on the surface as an oil. This is extracted with a few ml of methylene chloride. The solution is dried with MgSO₄ and is evaporated to dryness under vacuum to give 3-(chloromethyl)pyridine. This must be prepared fresh for each use, and heat should be avoided to minimize decomposition. (3-(chloromethyl)pyridine is a severe irritant, and care must be taken in handling it.) The reaction mixture is stirred for 1 hour at -78°, and is then allowed to warm to room temperature over several hours as the bath warms up. It is stirred at ambient temperature for several hours, and is then quenched by addition of 1.2 g (.02 mole) of acetic acid. The reaction mixture is evaporated to dryness on a rotary evaporator, and the resulting residue is purified by flash chromatography on silica gel using chloroform/methanol as eluant to give 1-diethoxyphosphinyl-2-(3-pyridyl)-ethanesulfonic acid lithium salt.
II. Synthesis of 1-Phosphono-2-(3-pyridyl)ethanesulfonic Acid
   The above diester is hydrolyzed by dissolving it (2 g) in 20 ml of 6 N HCl and heating this solution at reflux for 12-24 hours. It is then evaporated to dryness under vacuum. The residue is dissolved in water and is treated with cation exchange resin in H⁺ form to remove Li salts. The solution is taken to dryness on a rotary evaporator, and the residue is triturated with acetone. The resulting solid is filtered off and recrystallized from water/ethanol to yield 1-phosphono-2(3-pyridyl)ethanesulfonic acid.

### Example 17

### Synthesis of 1-Methyl-3-(2-phosphono-2-sulfonoethyl)pyridinium Iodide

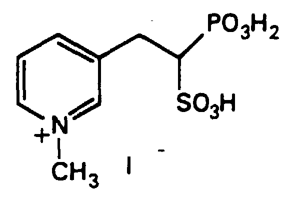

A solution of 2.67g (.01 mole) of 1-phosphono-2-(3-pyridinyl)ethanesulfonic acid (prepared as described in Example 16) in 20 ml water and 30 ml ethanol is adjusted to pH 7.0 by addition of 1N aqueous NaOH. To this is added 7.1 g (.05 mole) of methyl iodide, and the reaction is stirred at 30-50° for one day. The reaction is evaporated to dryness under reduced pressure. The resulting residue is dissolved in distilled water, and is treated with cation exchange resin in H⁺ form. The resin is filtered off and the aqueous solution is evaporated to dryness under vacuum. The residue is triturated with acetone to give a solid which is collected by filtration. This is purified by recrystallization from water/acetone to give N-methyl-3-(2-phosphono-2-sulfonoethyl)pyridinium iodide.

### Example 18

### Synthesis of N-Ethyl-3-(2-phosphono-2-sulfonoethyl)pyridinium Iodide

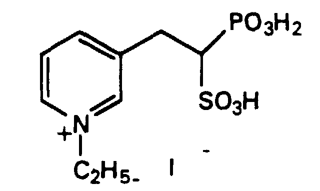

A solution of 2.67 g (.01 mole) of 1-phosphono-2-(3-pyridyl)ethanesulfonic acid (prepared as described in Example 16) in 20 ml water and 40 ml ethanol is adjusted to pH 7.0 by addition of 1N aqueous NaOH. To this is added 6.24 g (.04 mole) of ethyl iodide, and the reaction is stirred at 30-50° for one day. The reaction is evaporated to dryness under reduced pressure. The resulting residue is dissolved in distilled water, and is treated with cation exchange resin in H⁺ form. The resin is filtered off, the aqueous solution is concentrated to a few ml, and acetone is added dropwise to precipitate the product. This is purified by recrystallization from water/acetone to give N-ethyl-3-(2-phosphono-2-sulfonoethyl)pyridinium iodide.

### Example 19

### Synthesis of N-(2-(Acetylthio)ethyl)-3-(2-phosphono-2-sulfonoethyl)pyridinium Bromide

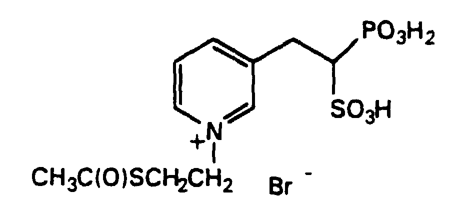

A solution of 2.67 g (.01 mole) of 1-phosphono-2-(3-pyridyl)ethanesulfonic acid (prepared as described in Example 16) in 20 ml water and 40 ml ethanol is adjusted to pH 7.0 by addition of 1N aqueous NaOH. To this is added 9.16 g (.05 mole) of S-acetyl-2-bromoethanethiol, and the reaction is heated at 40-80° for several hours. The reaction is evaporated to dryness under reduced pressure. The resulting residue is triturated with acetone several times (acetone extracts are discarded). The remaining solid is dissolved in distilled water, and is treated with cation exchange resin in H⁺ form. The resin is filtered off, the aqueous solution is concentrated to a few ml, and acetone is added dropwise to precipitate the product. This is purified by recrystallization from water/acetone to give N-(2-(acetylthio)ethyl)-3-(2-phosphono-2-sulfonoethyl)pyridinium bromide.

### Example 20

### Synthesis of 3-(2-Phosphono-2-sulfonoethyl)-N-(2-thioethyl)pyridinium Chloride

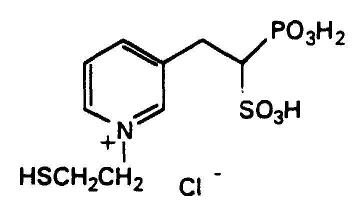

A solution of 1 g of N-(2-(acetylthio)ethyl)-3-(2-phosphono-2-sulfonoethyl)pyridinium bromide in 50 ml of water is treated with anion exchange resin in chloride form. The solution is concentrated to 20 ml, and 20 ml of 12 N HCl is added. The solution is heated at reflux under a nitrogen atmosphere for 12 hours, and is then evaporated dry. The residue is dissolved in 50 ml of fresh 6 N HCl and is again evaporated to dryness. It is then taken up in a few ml of water and is reprecipitated with ethanol to yield 3-(2-phosphono-2-sulfonoethyl)-N-(2-thioethyl)pyridinium chloride. All of these operations are carried out under N₂ atmosphere using deoxygenated solvents to minimize disulfide formation.

### Example 21

### Synthesis of 1-Phosphono-2-(2-pyridyl)ethanesulfonic Acid

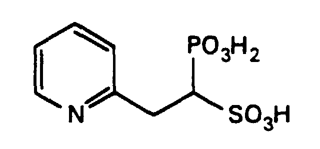

This compound is synthesized according to the method in Example 16, starting with 2-picolyl chloride hydrochloride.

### Example 22

### Synthesis of N-Methyl-2-(2-phosphono-2-sulfonoethyl)pyridinium Iodide

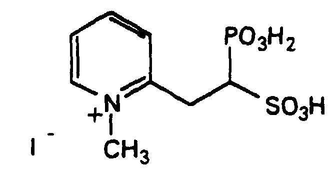

This compound is synthesized by the method of Example 17, starting with 1-phosphono-2-(2-pyridyl)ethanesulfonic acid.

### Example 23

### Synthesis of N-Ethyl-2-(2-phosphono-2-sulfonoethyl)pyridinium Iodide

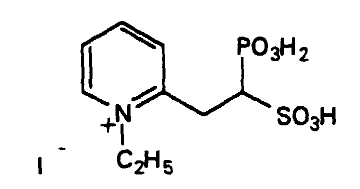

This compound is synthesized by the method of Example 18, starting with 1-phosphono-2-(2-pyridyl)ethanesuifonic acid.

### Example 24

### Synthesis of N-(2-(Acetylthio)ethyl)-2-(2-phosphono-2-sulfonoethyl)pyridinium Bromide

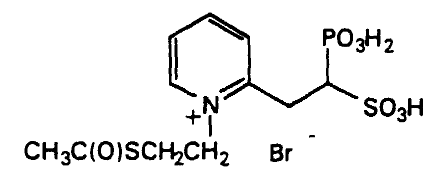

This compound is synthesized by the method of Example 19 starting with 1-phosphono-2-(2-pyridyl)ethanesulfonic acid.

### Example 25

### Synthesis of 2-(2-Phosphono-2-sulfonoethyl)-N-(2-thioethyl)pyridinium Chloride

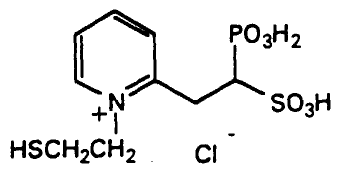

This compound is synthesized by the method of Example 20 starting with N-(2-(acetylthio)ethyl)-2-(2-phosphono-2-sulfonoethyl)pyridinium bromide.

### Example 26

### Synthesis of 2-(1-Methyl-2-piperidinyl)-1-phosphonoethanesulfonic Acid

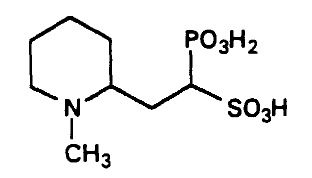

A mixture of 1 g of N-methyl-2-(2-phosphono-2-sulfonoethyl)pyridinium iodide and .5 g of palladium on charcoal catalyst in 50 ml of distilled water is hydrogenated on a Parr apparatus at 40 PSI for about 2 days. The catalyst is removed by filtration, and the filtrate is concentrated to a few mls. Ethanol is added slowly to precipitate a solid, which is recrystallized from water/ethanol to afford 2-(1-methyl-2-piperidinyl)-1-phosphonoethanesulfonic acid.

### Example 27

### Synthesis of N,N-Dimethyl-2-(2-phosphono-2-sulfonoethyl)piperidinium Iodide

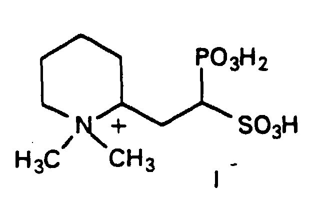

This is prepared by the method used in Example 17, starting with 2-(1-methyl-2-piperidinyl)-1-phosphonoethanesulfonic acid.

### Example 28

### Synthesis of N-Methyl-N-ethyl-2-(2-phosphono-2-sulfonoethyl)piperidinium Iodide

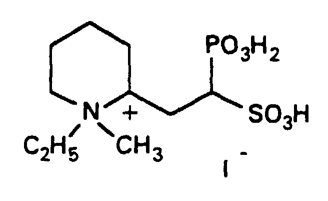

This is prepared by the method used in Example 18, starting with 2-(1-methyl-2-piperidinyl)-1-phosphonoethanesulfonic acid.

### Example 29

### Synthesis of N-(2-(Acetylthio)ethyl)-N-methyl-2(2-phosphono-2-sulfonoethyl)-piperidinium Bromide

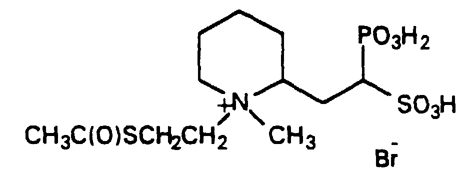

This is prepared by the method used in Example 19, starting with 2-(1-methyl-2-piperidinyl)-1-phosphonoethanesulfonic acid.

### Example 30

### Synthesis of N-Methyl-N-(2-thioethyl)-2-(2-phosphono-2-sulfonoethyl)piperidinium Chloride

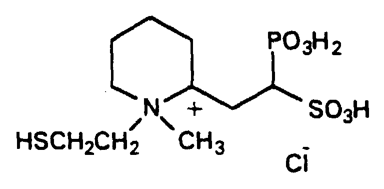

This is prepared by the method used in Example 20, starting with N-(2-(acetylthio)ethyl)-N-methyl-2-(2-phosphono-2-sulfonoethyl)piperidinium bromide.

### Example 31

### Synthesis of 2-(2-Piperidinyl)-1-phosphonoethanesulfonic Acid

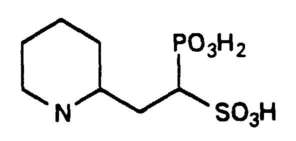

A mixture of 1 g of 1-phosphono-2-(2-pyridinyl)ethanesulfonic acid and .5 g of palladium on charcoal catalyst in 50 ml of distilled water is hydrogenated on a Parr apparatus at 40 PSI for about 2 days. The catalyst is removed by filtration, and the filtrate is concentrated to a few mls. Ethanol is added slowly to precipitate a solid, which is recrystallized from water/ethanol to afford 1-phosphono-2-(2-piperidinyl)ethanesulfonic acid.

### Example 32

### Synthesis of 2-(3-Piperidinyl)-1-phosphonoethanesulfonic Acid

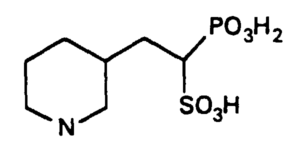

This compound is prepared by the method of Example 31 starting with 1-phosphono-2-(3-pyridinyl)ethanesulfonic acid .

### Example 33

### Synthesis of 2-(1-Methyl-3-piperidinyl)-1-phosphonoethanesulfonic Acid

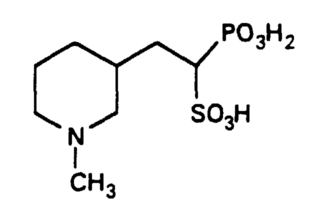

This compound is prepared by the method of Example 26, starting with 1-methyl-3-(2-phosphono-2-sulfonoethyl)pyridinium iodide.

### Example 34

### Synthesis of N,N-Dimethyl-3-(2-phosphono-2-sulfonoethyl)piperidinium Iodide

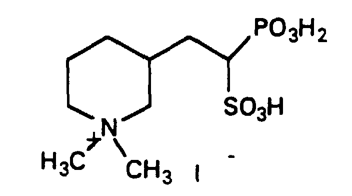

This is prepared by the method used in Example 17, starting with 2-(1-methyl-3-piperidinyl)-1-phosphonoethanesulfonic acid.

### Example 35

### Synthesis of N-(2-(Acetylthio)ethyl)-N-methyl-3-(2-phosphono-2-sulfonoethyl)-piperidinium Bromide

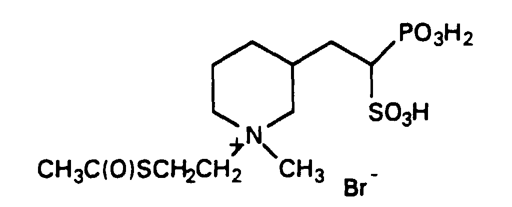

This is prepared by the method used in Example 19, starting with 2-(1-methyl-3-piperidinyl)--phosphonoethanesulfonic acid.

### Example 36

### Synthesis of N-Methyl-N-(2-thioethyl)-3-(2-phosphono-2-sulfonoethyl)piperidinium Chloride

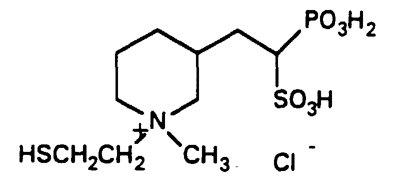

This is prepared by the method used in Example 20, starting with N-(2-(acetylthio)ethyl)-N-methyl-3-(2-phosphono-2-sulfonoethyl)piperidinium bromide.

### Example 37

### Synthesis of Phosphono(2-pyridinylthio)methanesulfonic acid

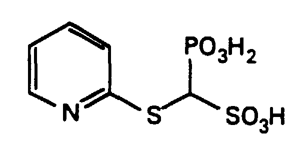

A suspension of 2.38 g (.01 mole) of diethoxyphosphinylmethanesulfonate lithium salt (Carretero, et al.; Tetrahedron, 43, 5125 (1987)) in 50 ml of anhydrous tetrahydrofuran is stirred in a -40° bath under a dry nitrogen atmosphere. To this is added n-butyl lithium (4.4 ml of 2.5 M solution in hexanes; .011 mole) via syringe over 5 minutes. The reaction mixture is allowed to warm to -15°, and is stirred at this temperature for about one hour. To it is then added rapidly a solution of 2.2 g (.01 mole) of 2,2'-dipyridyl disulfide in 30 ml of anhydrous THF. The reaction mixture is stirred for 1 hour at -15°, and is then allowed to warm to room temperature over several hours as the bath warms up. It is stirred at ambient temperature for several hours, and is then quenched by addition of 50 ml of water. The reaction mixture is concentrated under vacuum on a rotary evaporator to get rid of the THF, and the aqueous layer is extracted with ether to remove some impurities. The water layer is concentrated to 10 ml on a rotary evaporator, and 10 ml of 12 N HCl is added. The solution is heated at reflux for 12-24 hours, and is evaporated to dryness under vacuum. The residue is dissolved in water and is treated with cation exchange resin in H⁺ form to remove Li salts. The solution is taken to dryness on a rotary evaporator, and the residue is triturated with acetone. The resulting solid is filtered off and recrystallized from water/ethanol to yield phosphono(2-pyridinylthio)methanesulfonic acid.

### Example 38

### Synthesis of 4-Amino-1-phosphonobutanesulfonic Acid

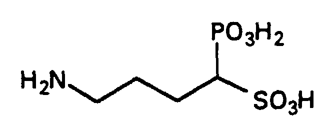

I. Synthesis of 3-Cyano-1-diethoxyphosphinylpropanesulfonic Acid Lithium Salt
   A suspension of 2.38 g (.01 mole) of diethoxyphosphinylmethanesulfonate lithium salt (Carretero, et al.; Tetrahedron, 43, 5125 (1987)) in 50 ml of anhydrous tetrahydrofuran is stirred in a -40° bath under a dry nitrogen atmosphere. To this is added n-butyl lithium (4.4 ml of 2.5 M solution in hexanes; .011 mole) via syringe over 5 minutes. The reaction mixture is allowed to warm to -15°, and is stirred at this temperature for about one hour. To it is then added rapidly a solution of .53 g (.01 mole) of acrylonitrile in 3 ml of anhydrous THF. The reaction mixture is stirred for 1 hour at -15°, and is then allowed to warm to room temperature over several hours as the bath warms up. It is stirred at ambient temperature for several hours, and then is quenched by addition of 50 ml of water containing .6 g (.01 mole) of acetic acid. The reaction mixture is concentrated under vacuum on a rotary evaporator to get rid of the THF, and the aqueous layer is extracted with ether to remove some impurities. The water layer is evaporated to dryness under vacuum, and the resulting residue is purified by flash chromatography on silica gel using chloroform/methanol as eluant, to afford 3-cyano-1-diethoxyphosphinylpropanesulfonic acid lithium salt.
II. Synthesis of 4-Amino-1-phosphonobutanesulfonic Acid
   The hydrogenation of 3-cyano-1-diethoxyphosphinylpropanesulfonic acid lithium salt is carried out using the hydrogenation technique of Freifelder (J. Am. Chem. Soc., 82, 2386 (1960)). The cyano compound (2.62 g; .01 mole) is placed in 20 ml of 10% methanolic ammonia.. Rhodium on alumina (5%) catalyst (.5 g) is added, and the mixture is hydrogenated at 40 PSI on a Parr apparatus for several hours (until uptake of hydrogen is complete). The catalyst is filtered off, and the filtrate is evaporated dry. The product is hydrolyzed by dissolving the residue in 25 ml of 6 N HCl and refluxing the solution for 12-24 hours. It is then evaporated to dryness under vacuum on a rotary evaporator. Distilled water (25 ml) is added, and the solution is again evaporated dry. The resulting residue is dissolved in a few ml H₂O, and ethanol is added to precipitate a solid. This is recrystallized from water/ethanol to yield 4-amino-1-phosphonobutanesulfonic acid.

### Example 39

### Synthesis of 1-Hydroxy-1-phosphono-2-(3-pyridinyl)ethanesulfonic Acid

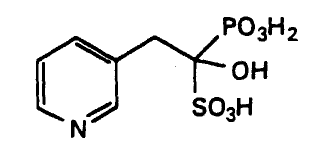

I. Synthesis of 1-Diethoxyphosphinyl-1-hydroxy-2-(3-pyridinyl)ethanesulfonic Acid Lithium Salt
   A suspension of 3.29 g (.01 mole) of 1-diethoxyphosphinyl-2-(3-pyridinyl)ethanesulfonic acid lithium salt (from part I of Example 16) in 50 ml of anhydrous tetrahydrofuran is stirred in a -40° bath under a dry nitrogen atmosphere. To this is added n-butyl lithium (4.4 ml of 2.5 M solution in hexanes; .011 mole) via syringe over 5 minutes. The reaction mixture is allowed to warm to -15°, and is stirred at this temperature for about one hour. It is then cooled to -78°, and to it is added dropwise a solution of 3.44 g (.015 mole) of camphorylsulfonyloxaziridine (J. Am. Chem. Soc., 112, 6679(1990)) in 50 ml of anhydrous THF. The reaction mixture is stirred for a few minutes at -78°, and then is placed in an ice/water bath. It is stirred at 0° for 2-10 minutes, and then is again cooled to -78°. The reaction mixture is quenched by addition of 1.2 g (.02 mole) of acetic acid and the solvents are removed under vacuum on a rotary evaporator. The residue was purified by chromatography on silica gel using chloroform/methanol as eluant, to yield 1-diethoxyphosphinyl-1-hydroxy-2-(3-pyridinyl)ethanesulfonic acid lithium salt.
II. Synthesis of 1-Hydroxy-1-phosphono-2-(3-pyridinyl)ethanesulfonic Acid
   Hydrolysis of the above diester is effected by dissolving it (1 g) in 15 ml of 6 N HCl and heating the solution at reflux for 12-24 hours. The solution is then evaporated to dryness. The residue is redissolved in water and treated with cation exchange resin in H⁺ form. The solution is again taken to dryness and is triturated with acetone to give a solid. This is recrystallized from water/ethanol to yield 1-hydroxy-1-phosphono-2-(3-pyridinyl)ethanesulfonic acid.

### Example 40

### Synthesis of 1-Methyl-3-(2-hydroxy-2-phosphono-2-sulfonoethyl)pyridinium Iodide

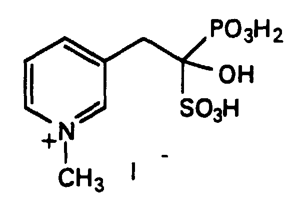

This compound is synthesized by the method of Example 17, starting with 1-hydroxy-1-phosphono-2-(3-pyridinyl)ethanesulfonic acid.

### Example 41

### Synthesis of N-Ethyl-3-(2-hydroxy-2-phosphono-2-sulfonoethyl)pyridinium Iodide

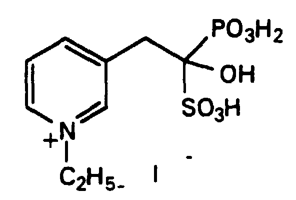

This compound is synthesized by the method of Example 18, starting with 1-hydroxy-1-phosphono-2-(3-pyridinyl)ethanesulfonic acid.

### Example 42

### Synthesis of N-(2-(Acetylthio)ethyl)-3-(2-hydroxy-2-phosphono-2-sulfonoethyl)pyridinium Bromide

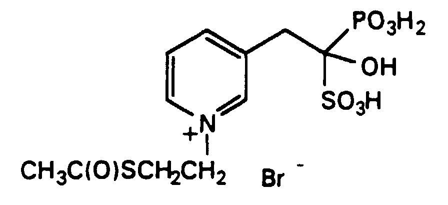

This compound is synthesized by the method of Example 19, starting with 1-hydroxy-1-phosphono-2-(3-pyridinyl)ethanesulfonic acid.

### Example 43

### Synthesis of 3-(2-Hydroxy-2-phosphono-2-sulfonoethyl)-N-(2-thioethyl)-pyridinium Chloride

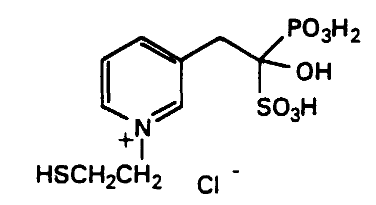

This compound is synthesized by the method of Example 20, starting with N-(2-(acetylthio)ethyl)-3-(2-hydroxy-2-phosphono-2-sulfonoethyl)pyridinium bromide.

### Example 44

### Synthesis of 1-Hydroxy-1-phosphono-2-(2-pyridyl)ethanesulfonic Acid

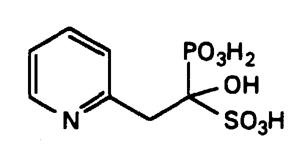

This compound is synthesized according to the method in Example 39, starting with 1-diethoxyphosphinyl-2-(2-pyridinyl)ethanesulfonic acid lithium salt.

### Example 45

### Synthesis of N-Methyl-2-(2-hydroxy-2-phosphono-2-sulfonoethyl)pyridinium Iodide

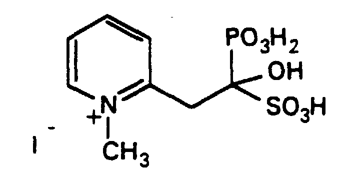

This compound is synthesized by the method of Example 17, starting with 1-hydroxy-1-phosphono-2-(2-pyridinyl)ethanesulfonic acid.

### Example 46

### Synthesis of N-Ethyl-2-(2-hydroxy-2-phosphono-2-sulfonoethyl)pyridinium Iodide

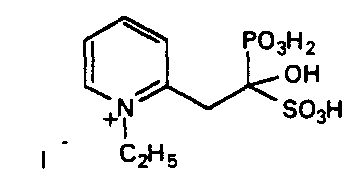

This compound is synthesized by the method of Example 18, starting with 1-hydroxy-1-phosphono-2-(2-pyridinyl)ethanesulfonic acid.

### Example 47

### Synthesis of N-(2-(Acetylthio)ethyl)-2-(2-hydroxy-2-phosphono-2-sulfonoethyl)pyridinium Bromide

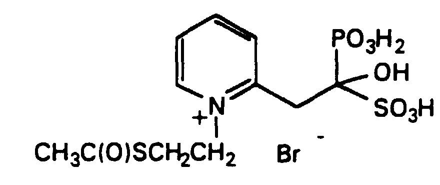

This compound is synthesized by the method of Example 19 starting with 1-hydroxy-1-phosphono-2-(2-pyridinyl)ethanesulfonic acid.

### Example 48

### Synthesis of 2-(2-Hydroxy-2-phosphono-2-sulfonoethyl)-N-(2-thioethyl)-pyridinium Chloride

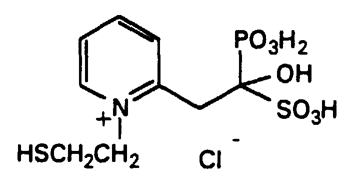

This compound is synthesized by the method of Example 20 starting with N-(2-(acetylthio)ethyl)-2-(2-hydroxy-2-phosphono-2-sulfonoethyl)pyridinium bromide.

### Example 49

### Synthesis of 1-Hydroxy-2-(1-methyl-2-piperidinyl)-1-phosphonoethanesulfonic Acid

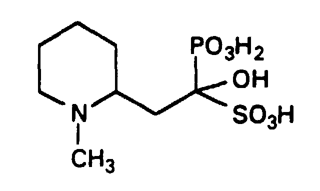

A mixture of 1 g of N-methyl-2-(2-hydroxy-2-phosphono-2-sulfonoethyl)pyridinium iodide and .5 g of palladium on charcoal catalyst in 50 ml of distilled water is hydrogenated on a Parr apparatus at 40 PSI for about 2 days. The catalyst is removed by filtration, and the filtrate is concentrated to a few mls. Ethanol is added slowly to precipitate a solid, which is recrystallized from water/ethanol to afford 1-hydroxy-2-(1-methyl-2-piperidinyl)-1-phosphonoethanesulfonic acid.

### Example 50

### Synthesis of N,N-Dimethyl-2-(2-hydroxy-2-phosphono-2-sulfonoethyl)piperidinium Iodide

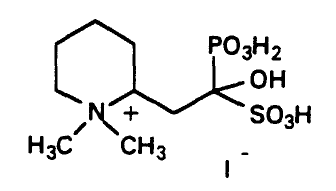

This is prepared by the method used in Example 17, starting with 1-hydroxy-2-(1-methyl-2-piperidinyl)-1-phosphonoethanesulfonic acid.

### Example 51

### Synthesis of N-Methyl-N-ethyl-2-(2-hydroxy-2-phosphono-2-sulfonoethyl)-piperidinium Iodide

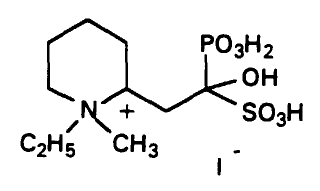

This is prepared by the method used in Example 18, starting with 1-hydroxy-2-(1-methyl-2-piperidinyl)-1-phosphonoethanesulfonic acid.

### Example 52

### Synthesis of N-(2-(Acetylthio)ethyl)-N-methyl-2-(2-hydroxy-2-phosphono-2-sulfonoethyl)piperidinium Bromide

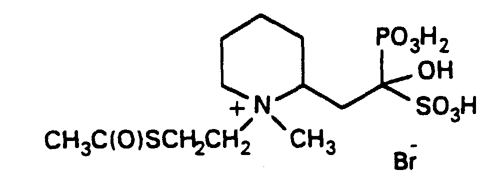

This is prepared by the method used in Example 19, starting with 1-hydroxy-2-(1-methyl-2-piperidinyl)-1-phosphonoethanesulfonic acid.

### Example 53

### Synthesis of N-Methyl-N-(2-thioethyl)-2-(2-hydroxy-2-phosphono-2-sulfonoethyl)piperidinium Chloride

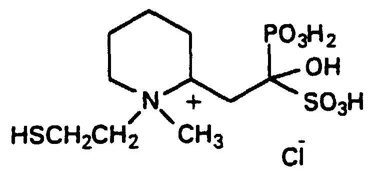

This is prepared by the method used in Example 20, starting with N-(2-(acetylthio)ethyl)-N-methyl-2-(2-hydroxy-2-phosphono-2-sulfonoethyl)piperidinium bromide.

### Example 54

### Synthesis of 1-Hydroxy-2-(2-Piperidinyl)-1-phosphonoethanesulfonic Acid

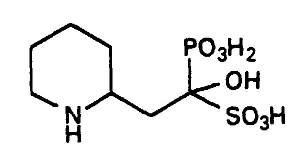

A mixture of 1 g of 1-hydroxy-1-phosphono-2-(2-pyridinyl)-ethanesulfonic acid and .5 g of palladium on charcoal catalyst in 50 ml of distilled water is hydrogenated on a Parr apparatus at 40 PSI for about 2 days. The catalyst is removed by filtration, and the filtrate is concentrated to a few mls. Ethanol is added slowly to precipitate a solid, which is recrystallized from water/ethanol to afford 1-hydroxy-1-phosphono-2-(3-piperidinyl)ethanesulfonic add.

### Example 55

### Synthesis of 1-Hydroxy-2-(3-piperidinyl)-1-phosphonoethanesulfonic Acid

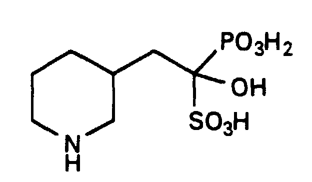

This compound is prepared by the method of Example 54 starting with 1-hydroxy-1-phosphono-2-(3-pyridinyl)ethanesulfonic acid .

### Example 56

### Synthesis of 1-Hydroxy-2-(1-methyl-3-piperidinyl)-1-phosphonoethanesulfonic Acid

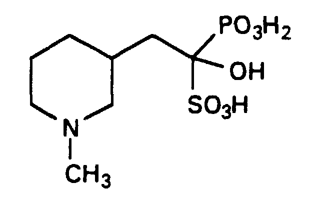

This compound is prepared by the method of Example 54, starting with 1-methyl-3-(2-hydroxy-2-phosphono-2-sulfonoethyl)pyridinium iodide.

### Example 57

### Synthesis of N,N-Dimethyl-3-(2-hydroxy-2-phosphono-2-sulfonoethyl)piperidinium Iodide

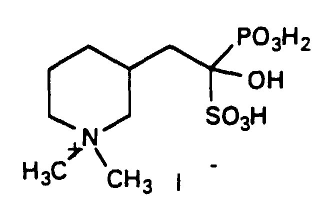

This is prepared by the method used in Example 17, starting with 1-hydroxy-2-(1-methyl-3-piperidinyl)-1-phosphonoethanesulfonic acid.

### Example 58

### Synthesis of N-(2-(Acetylthio)ethyl)-N-methyl-3-(2-hydroxy-2-phosphono-2-sulfonoethyl)piperidinium Bromide

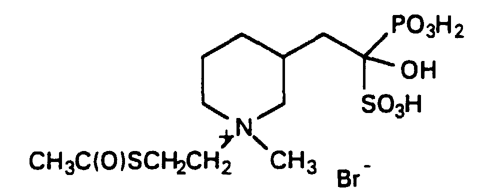

This is prepared by the method used in Example 19, starting with 1-hydroxy-2-(1-methyl-3-piperidinyl)-1-phosphonoethanesulfonic acid.

### Example 59

### Synthesis of N-Methyl-N-(2-thioethyl)-3-(2-hydroxy-2-phosphono-2-sulfonoethyl)piperidinium Chloride

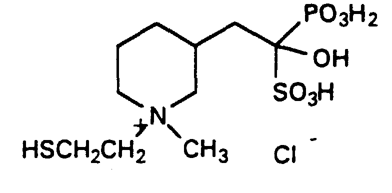

This is prepared by the method used in Example 20, staring with N-(2-(acetylthio)ethyl)-N-methyl-3-(2-hydroxy-2-phosphono-2-sulfonoethyl)piperidinium bromide.

### Example 60

### Synthesis of Dihydro-6-phosphono-1-pyrindine-6-sulfonic Acid

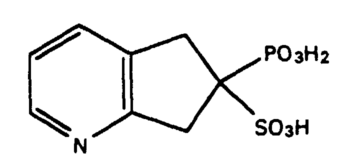

To 70 ml of anhydrous dimethylsulfoxide (DMSO), stirred under nitrogen atmosphere in an ice bath, is added 1.6 g of 60% NaH in mineral oil (0.04 mole). When this is dissolved, there is added dropwise to the solution (still stirred at 0°) a solution of diethoxyphosphinyl-methanesulfonic acid lithium salt (4.76 g, 0.02 mole) in 30 ml of DMSO. The reaction mixture is stirred at room temperature for one hour. To it is then added dropwise a solution of 3.48 g (0.02 mole) of 2,3-bis(chloromethyl)pyridine (see K. Tsuda, et al., Chem. Pharm. Bull., 1, 142 (1953)) in 15 ml of DMSO. The mixture is stirred at room at 80° for 1-3 hours. The DMSO is removed under vacuum, and the residue is purified by flash chromatography on silica gel using 1-15% methanol in methylene chloride gradient as eluant.
The above ester is hydrolyzed by refluxing it in 6 N HCl for 20 hours. Upon concentration of the reaction solution and cooling it in ice a precipitate forms. This is recrystallized from water to afford dihydro-1-pyrindine-6-phosphono-6-sulfonic acid.

### Example 61

### Synthesis of Octahydro-6-phosphono-1-pyrindine-6-carboxylic Acid

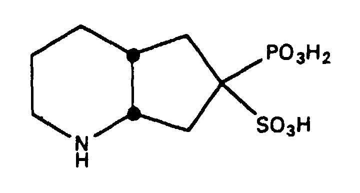

1.0 g of dihydro-6-phosphono-1-pyrindine-6-carboxylic acid hydrochloride (from example 60) in 50 ml of H₂O with .5 g of PtO₂ is hydrogenated on a Parr apparatus at 40 PSI for 3 days. The catalyst is filtered off, and the filtrate is taken to dryness under vacuum. The resulting solid is taken up in the minimum amount of water, and precipitated by slow addition of ethanol to give octahydro-6-phosphono-1-pyrindine-6-sulfonic acid.

### Example 62

Capsules are prepared having the following composition:

| Active Ingredient | Mg Per Capsule |
|---|---|
| 1-hydroxy-1-phosphono-2-(3-pyridinyl)ethanesulfonic Acid | 350.0 |

| Excipients | |
|---|---|
| Lactose | 90.0 |
| Microcrystalline Cellulose | 60.0 |
| Magnesium Stearate | 1.0 |

The capsules having the above composition are prepared using conventional methods as described below:

The active ingredient is mixed with the microcrystalline cellulose in a turn shell blender for approximately ten (10) minutes.

The resulting mixture is passed through a hammer mill with an 80 mesh screen.

The mixture is put back into the twin shell blender along with the lactose and is then mixed for approximately fifteen (15) minutes.

The magnesium stearate is next added and blended for an additional five (5) minutes. The resulting blend is then compressed on a piston-activated capsule filler.

Any of the compounds prepared according to Examples 1 to 61 may be substituted for the active ingredient in the capsule prepared hereinabove.

### Example 63

Tablets are prepared having the following composition:

| Active Ingredient | Mg Per Tablet |
|---|---|
| 3-(2-Hydroxy-2-phosphono-2-sulfonoethyl)-N-(2-thioethyl)pyridinium chloride | 700.00 |

| Excipients | |
|---|---|
| Lactose (spray-dried) | 200.0 |
| Starch (1500) | 100.0 |
| Magnesium Stearate | 25.0 |

Tablets are prepared having the above composition using conventional methods as described below:

The active ingredient is ground in a ball mill for approximately thirty (30) minutes. The milled active ingredient is then blended in a twinblade mixer with the spray-dried lactose for approximately twenty (20) minutes.

The starch is added to the mixture and is then mixed for an additional fifteen (15) minutes. The blend is compressed into tablets on a standard tablet press.

Any of the compounds prepared according to Examples 1 to 61 may be substituted for the active ingredient in the tablet prepared hereinabove.

### Example 64

Injectable solutions are prepared by conventional methods using 100 ml of physiological saline solution and 70 mg P of 3-(2-hydroxy-2-phosphono-2-sulfonoethyl)-N-(2-thioethyl)pyridinium chloride, adjusted to pH = 7.4.

One injection, one time daily for 4 days, results in appreciable alleviation of hypercalcemia of malignancy in patients weighing approximately 70 kilograms.

Any of the compounds prepared according to Examples 1 to 61 may be substituted for the active ingredient in the injection prepared hereinabove.

### Example 65

A Caucasian male, weighing approximately 92 kilograms, seventy-two years of age, suffering from moderate to severe pain, and occasional swelling, of the right knee. After approximately one year of steadily increasing discomfort, he visits a physician who renders a clinical diagnosis of osteoarthritis of the right knee, which is subsequently verified by X-ray diagnosis.

After a period of ameliorative therapy of various NSAIDs, including aspirin, naprosen, and ketoprofen, his symptoms continue to worsen and his condition appears to degenerate. He returns to his physician who then prescribes the tablets prepared as described in Example 63 twice daily two hours before or after meals for a period of three months. His clinical symptoms of pain and swelling, particularly with extended walking, improves significantly after his 3 months of therapy. At the conclusion of three months at a dosage of 2 tablets per day, the therapy is continued at one-half the dosage originally prescribed (i.e. 1 tablets per day) indefinitely.

### Example 66

A black female, weighing approximately 65 kilograms, fifty-five years of age, presents with swelling and deformation of the finger joints of both hands, with partial loss of strength and/or dexterity of her fingers and hands. Upon visual and X-ray examination and various appropriate clinical tests approved by the American Rheumatological Association (ARA) she is diagnosed with rheumatoid arthritis.

After an unsuccessful analgesic and anti-inflammatory therapy, her physician prescribes the tablets prepared in Example 63, two times daily two hours before or after meals for a period of four months. After a month of therapy, her symptoms of knuckle swelling noticeably improves and her range of finger motion increases significantly; she continues therapy for the remainder of the four months, after which her physician continues the prescribed dose for an additional two months.

### Example 67

A female of Hispanic origin, twelve years of age, weighing approximately 37 kilograms, presents to the physician with idiopathic juvenile rheumatoid arthritis. Her symptoms include marked inflammation of multiple joints, complicated by heat and tenderness and indicating rapid and pathological degeneration of joint function.

Her physician refers her to a rheumatologist who immediately prescribes aggressive therapy by IV administration of the solution prepared as described in Example 64 over a period of three days, at the rate of 1 injection per day, administered over two hours. At the conclusion of the IV regimen, the physician prescribes the tablets prepared as described in Example 63, for a period of two months, during which she exhibits marked improvement with increased mobility and decreased pain. For the succeeding two months, the physician reduces her dose to 3/4 of the original oral dose by prescribing 3 tablets over a period of two days, i.e. one 2-tablet day alternating with one 1-tablet day. At the conclusion of this regimen the dosage is again reduced to 1/4 of the original dose by giving her the tablets prepared as described in Example 63, 1 tablet every day for an additional four months.

### Example 68

A 60-year-old Caucasian female weighing 62 kg, experiences severe back pain. Her physician, with the aid of a radiologist diagnoses her as having a crush fracture of the L1 vertebrae presumably due to osteoporotic bone loss. The patient is prescribed a three month, once-daily dosage regimen of a 700 mg tablet prepared according to the procedure described in Example 62. The 700 mg tablet is taken either two hours before or two hours after any given meal. After three months, the dosage is reduced to a 350 mg capsule, prepared as described in Example 63, taken every other day for a period of three months. Her physician then puts her on a maintenance dosing regimen wherein she takes a 100 mg capsule every day for six months. After six months on the maintenance dosing regimen the patient is not experiencing any further back pain. Follow-up x-rays reveal no additional fractures.

### Example 69

A 75-year-old Oriental female weighing 53 kg suffers a fractured hip after a fall. She is hospitalized and diagnosed as having osteoporosis. A treatment regimen of calcitonin injections is prescribed. The calcitonin injections are painful to the patient and she is unable to comply with the calcitonin regimen. Her physician then switches her therapy to an oral phosphonate regimen. She is administered a 700 mg tablet prepared according to the procedure described in Example 63, twice daily for one month. At the end of this one month of therapy, she is given a 700 mg tablet, once daily for two months. At the end of this two month period, she is given a 100 mg capsule, prepared according to the procedure described in Example 12, daily for three months. A follow-up visit to her physician reveals no apparent decrease in mineral density of the forearm as determined by photonabsorptimetry.

### Example 70

A 85-year-old Native American male weighing 65 kg presents to his physician with severe back pain. X-rays reveal multiple minor vertebral body collapse resulting from significant bone loss due to osteoporosis. The patient is prescribed a two month regimen of a 700 mg tablet and a 350 mg capsule to be taken on the same day, eight hours apart, prepared according to the procedures described in Examples 63 and 62, respectively. After two months on this regimen, his dosage is reduced to 350 mg tablet once a day for two months. X-rays are taken and an additional- crush fracture is noted. He is then put on a maintenance regimen of a 100 mg capsule, prepared according to the procedure described in Example 62, once a day for six months. At the end of this six months, no significant apparent decrease in bone density is observed.

## Claims

1. Phosphonosulfonates, and the pharmaceutically-acceptable salts and esters thereof, according to formula (I): 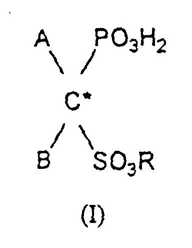 wherein
(A)
(1) A is selected from the group consisting of hydrogen; halogen; SR¹; R²SR¹ ; amino; hydroxy; and substituted or unsubstituted C₁-C₈ alkyl;
(2) B is
(a) -NH₂;
(b) a saturated or unsaturated C₁-C₁₅ alkyl chain substituted with one or more substituents selected from the group consisting of -R³N(R⁴)₂; -R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
(c) a substituted or unsubstituted, saturated or unsaturated heteroalkyl chain having from 2 to 15 chain atoms, where one or more of said chain atoms is nitrogen;
(d) a saturated or unsaturated heteroalkyl chain having from 2 to 15 chain atoms, where one or more of said chain atoms is selected from S and O; and where said heteroalkyl chain is substituted with one or more substituents selected from the group consisting of -R³N(R⁴)2; -R³[-N(R⁵)₃]⁺; -R³N(R⁴)C(O)R⁴; -R³N(R⁴)C(S)R⁴; -R³N(R⁴)C(N)R⁴; and -R³C(O)N(R⁴)₂; or
(e) R⁶-L- where
(i) L is selected from the group consisting of nil; S; O; a substituted or unsubstituted, saturated or unsaturated C₁-C₁₅ alkylene chain; and a substituted or unsubstituted, saturated or unsaturated heteroalkylene chain having from 2 to 15 chain atoms, where one or more of said chain atoms is N, S, or O; and
(ii) R⁶ is selected from the group consisting of saturated monocyclic or polycyclic carbocyclic rings; unsaturated monocyclic or polycyclic carbocyclic rings; saturated monocyclic or polycyclic heterocyclic rings; and unsaturated monocyclic or polycyclic heterocyclic rings; wherein R⁶ may be substituted with one or more substituents independently selected from the group consisting of -R³SR¹; substituted or unsubstituted C₁-C₈ alkyl; -R³OR⁴; -R³CO₂R⁴; -R³O₂CR⁴; -R³N(R⁴)₂; R³[-N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; arylalkyl; nitro; substituted or unsubstituted aryl; and hydroxy; and
(3)
(a) R¹ is independently selected from the group consisting of hydrogen; -C(O)R⁷; -C(S)R⁷; -C(O)N(R⁷)₂; -C(O)OR⁷; -C(S)N(R⁷)₂; and -C(S)OR⁷; where R⁷ is hydrogen or substituted or unsubstituted C₁-C₈ alkyl;
(b) R² is substituted or unsubstituted C₁-C₈ alkylene
(c) R³ is selected from the group consisting of nil and substituted or unsubstituted C₁-C₈ alkylene ;
(d) R⁴ is independently selected from the group consisting of hydrogen; substituted or unsubstituted C₁-C₈ alkyl; and -R²SR¹; and
(e) R⁵ is independently selected from the group consisting of substituted or unsubstituted C₁-C₁₅ alkyl; substituted or unsubstituted phenyl; benzyl; and -R²SR¹;
or
(B) A and B are covalently linked together with C* to form a monocyclic or bicyclic ring having the following structure: 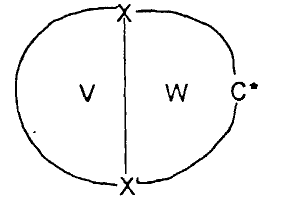 where
(1) W is a substituted or unsubstituted, saturated or unsaturated carbocyclic ring comprising C*, X, and X', said carbocyclic ring having a total of from 3 to 6 ring carbon atoms; or a substituted or unsubstituted, saturated or unsaturated heterocyclic ring comprising C*, X, and X', said heterocyclic ring having a total of from 4 to 6 ring atoms, where one or more of said ring atoms is N, O, or S;
(2) V is nil; a substituted or unsubstituted, saturated or unsaturated carbocyclic ring comprising X and X', said carbocyclic ring having a total of from 3 to 8 ring carbon atoms; or a substituted or unsubstituted, saturated or unsaturated heterocyclic ring comprising X and X', said heterocyclic ring having a total of from 3 to 8 ring atoms, where one or more of said ring atoms is N, O, or S; and
(3) X and X' are independently N or C;
except that if neither V nor W is a nitrogen containing heterocycle, then at least one of V or W is substituted with one or more substituents selected from the group consisting of -R³N(R⁴)₂; R³[-N(R⁵)₃]⁺; -R³N(R⁴)C(O)R⁴; -R³N(R⁴)C(S)R⁴; -R³N(R⁴)C(N)R⁴; and -R³C(O)N(R⁴)₂;
and wherein R is selected from the group consisting of hydrogen, lower alkyl, lower acyloxyalkyl, aminocarbonyloxyalkyl, pivaloyloxymethyl, lower alkoxyacyloxyalkyl, alkoxyalkyl, choline and acylamino alkyl.

2. A phosphonosulfonate according to Claim 1, wherein A is hydroxy; and B is a substituted or unsubstituted, saturated or unsaturated heteroalkyl chain having from 2 to 15 chain atoms, where one or more of said chain atoms is nitrogen, or B is R⁶-L-, where L is a C₁-C₁₅ alkylene chain, or a nitrogen containing heteroalkylene chain having from 2 to 15 chain atoms, and wherein R⁶ is a monocyclic or polycyclic heterocycle or carbocycle.

3. A phosphonosulfonate according to Claim 2, wherein B is R⁶-L- and L is a C₁-C₁₅ alkylene chain, where said alkylene chain is substituted with one or more substituents selected from the group consisting of -R³SR¹, substituted or unsubstituted C₁-C₈ alkyl, -R³OR⁴, and -R³CO₂R⁴; or a heteroalkylene chain having from 2 to 15 chain atoms, where said heteroalkylene chain is substituted with one or more substituents selected from the group consisting of -R³SR¹, -R³N(R⁴)₂; -R³[N(R⁵)₃+], and -R³N(R⁴)C(O)R⁴.

4. A phosphonosulfonate according to Claim 3, wherein R⁶ is a substituted or unsubstituted six-membered monocyclic heterocycle selected from the group consisting of pyridine, pyrimidine, piperidine, pyridinium, pyrimidinium, and piperidinium; a substituted or unsubsituted five-membered monocyclic heterocycle selected from the group consisting of imidazol, pyrrole, pyrrolidine, imidazolium, pyrrolium and pyrrolidinium; a substituted or unsubstituted monocyclic carbocycle selected from the group consisting of cycloheptyl or cyclohexyl; a substituted or unsubstituted polycyclic heterocycle having a six-membered ring fused to a five-membered ring, said polycyclic heterocycle being selected from the group consisting of indol, indolium, pyrindine, imidazol-(1,2-a-)pyridine, imidazol-(1,2-a-)pyridinium, and pyrindinium; or a substituted or unsubstituted polycyclic heterocycle having a six-membered ring fused to a six-membered ring, said polycyclic heterocycle being selected from the group consisting of quinoline, isoquinoline, tetrahydroquinoline, octahydroquinoline, quinolinium, isoquinolinium, tetrahydroquinolinium, and octahydroquinolinium.

5. A phosphonosulfonate according to Claim 4, wherein R⁶ is substituted with one or more substituents selected from the group consisting of -SR¹, -R³N(R⁴)₂, -R³[N(R⁵)₃], and -R³N(R⁴).

6. A phosphonosulfonate according to Claim 2, wherein B is a substituted or unsubstituted, saturated or unsaturated heteroalkyl chain having from 2 to 15 chain atoms, where one or more of said chain atoms is nitrogen and said heteroalkyl chain is substituted with one or more substituents selected from the group consisting of -R3SR1; substituted or unsubstituted C₁-C₈ alkyl; -R³OR⁴4; -R³CO₂R⁴; -R³N(R⁴)₂; -R³[N(R⁵)₃+]; and -R³N(R⁴)C(O)R⁴.

7. A phosphonosulfonate according to Claim 1, wherein A and B, together with C*, form a bicyclic ring, when W is a five-membered carbocyclic ring; and where V is a five-membered heterocyclic ring containing at least one ring nitrogen atom or, preferably, a six-membered heterocyclic ring containing at least one ring nitrogen atom.

8. A phosphonosulfonate according to Claim 7, wherein said bicyclic ring is substituted with one or more substituents selected from the group conisting of substituted or unsubstituted C₁-C₈ alkyl, -R³OR⁴, -R³O₂CR⁴, -R³N(R⁴)C(S)R⁴, -R³N(R⁴)C(N)R⁴, -R³C(O)N(R⁴)₂, -R³SR¹, -R³CO₂R⁴, -R³N(R⁴)₂, -R³[N(R⁵)₃+], and -R³N(R⁴)C(O)R⁴.

9. Phosphonosulfonates, and pharmaceutically-acceptable salts and esters thereof, according formula (I): 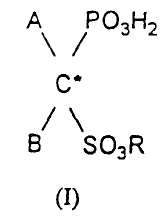 wherein
(A)
(1) A is hydroxy; and
(2) B is 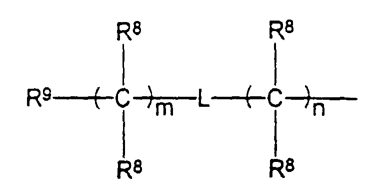 wherein
(a) m is an integer from 0 to 10; n is an integer from 0 to 10; and m+n is an integer from 0 to 10;
(b) R⁸ is independently selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; -R³[N(R⁵)₃]+ -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; nitro; hydroxy; substituted or unsubstituted saturated monocyclic or polycyclic carbocyclic rings; substituted or unsubstituted unsaturated monocyclic or polycyclic carbocyclic rings; substituted or unsubstituted saturated monocyclic or polycyclic heterocyclic rings; and substituted or unsubstituted unsaturated monocyclic or polycyclic heterocyclic rings;
(c) R¹ is independently selected from the group consisting of hydrogen; -C(O)R⁷; -C(S)R⁷; -C(O)N(R⁷)₂; -C(O)OR⁷; -C(S)N(R⁷)₂; and -C(S)OR⁷; where R⁷ is hydrogen or substituted or unsubstituted C₁-C₈ alkyl;
(d) R³ is selected from the group consisting of nil and substituted or unsubstituted C₁-C₈ alkylene;
(e) R⁴ is independently selected from the group consisting of hydrogen; substituted or unsubstituted C₁-C₈ alkyl; and -R²SR¹;
(f) R⁵ is independently selected from the group consisting of substituted or unsubstituted C₁-C₃₅ alkyl; substituted or unsubstituted phenyl; benzyl; and -R²SR¹;
(g) L is selected from the group consisting of nil; -N(R⁸)-; [-N(R⁵)₂-]+; -S-; -O-; and -D-C(=E)-S-, where D is selected from the group consisting of covalent bond, O, or S, and E is O or S; and wherein
(i) when L is -N(R⁸)-, or when L is [-N(R⁵)₂-]⁺ and m is an integer from 1 to 10, R⁹ is independently selected from the group consisting of hydrogen; substituted or unsubstituted C₁-C₁₅ alkyl; R²SR¹; and R¹⁰;
(ii) when L is [-N(R⁵)₂-]+ and m = 0, R⁹ is selected from the group consisting of substituted or unsubstituted C₁-C₁₅ alkyl; R²SR¹; and R¹⁰; or
(iii) when L is nil, -S-, -O-, or -D-C(=E)-S, R⁹ is R¹⁰;
(h) R¹⁰ is a saturated, unsaturated, or aromatic monocyclic or polycylic carbocycle or a saturated, unsaturated, or aromatic monocyclic or polycylic heterocycle and containing one or more heteroatoms; where said carbocycle or heterocycle is substituted with one or more R¹¹ substituents; and
(i) each R¹¹ is independently selected from the group consisting of -R³SR¹; substituted or unsubstituted C₁-C₈ alkyl; -R³OR⁴; -R³CO₂R⁴; -R³O₂CR⁴; -R³N(R⁴)₂; -R³[-N(R⁵)₃]+; -R³N(R⁴)C(O)R⁴; -R³N(R⁴)C(S)R⁴; -R³N(R⁴)C(N)R⁴; -R³C(O)N(R⁴)₂; halogen; -R³C(O)R⁴; hydroxy; substituted or unsubstituted arylalkyl; nitro; and unsubstituted or substituted aryl;
(j) R² is substituted or unsubstituted C₁-C₈ alkylene
or (B) A and B are covalently linked together with C* to form a bicyclic ring having the following structure: 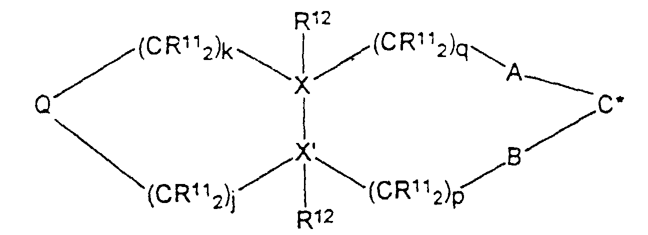 wherein
(a) A and B are independently selected from the group consisting of nil, -O-, -S-, and -NR¹⁰-;
(b) Q is selected from the group consisting of nil; -NR¹²-; and [-N(R¹³)₂-]+;
(c) X and X' are independently selected from C or N;
(d) each R¹² is independently selected from the group consisting of -R³SR¹; hydrogen; substituted or unsubstituted C₁-C₈ alkyl; -R³OR⁴; -R³CO₂R⁴; -R³O₂CR⁴; -R³N(R⁴)₂; R³[-N(R⁵)₃]+; -R³N(R⁴)C(O)R⁴; -R³C(O)N(R⁴)₂; halogen; -R³C(O)R⁴; hydroxy; substituted or unsubstituted arylalkyl; nitro; and unsubstituted or substituted aryl; and
(e) each R¹³ is selected from the group consisting of substituted or unsubitituted C₁-C₃₅ alkyl; substituted or unsuostituted phenyl; benzyl; and -R²SR¹;
(f) When Q is other than nil, k and j and k+j are integers from 0 to 5; when Q is nil, k and j and k+j are integers from 0 to 6; and
(g) p and q and p+q are independently integers from 0 to 3; except that if Q is nil, then at least one of R¹¹ or R¹² is selected from the group consisting of -R³N(R⁴)₂; R³[-N(R⁵)₃]+; -R³N(R⁴)C(O)R⁴; -R³N(R⁴)C(S)R⁴; -R³N(R⁴)C(N)R⁴; and -R³C(O)N(R⁴)₂;
and wherein R is selected from the group consisting of hydrogen, lower alkyl, lower acyloxyalkyl, aminocarbonyloxyalkyl, pivaloyloxymethyl, lower alkoxyacyloxyalkyl, alkoxyalkyl, choline and acylamino alkyl.

10. A pharmaceutical composition comprising:
(a) a safe and effective amount of a phosphonosulfonate according to Claim 1, 2, 3, 4, 5, 6, 7, 8, or 9; and
(b) pharmaceutically-acceptable carriers.

11. The use of a phosphonosulfonate, according to Claim 1, 2, 3, 4, 5, 6, 7, 8, or 9, for the manufacture of a medicament for the treatment of abnormal calcium and phosphate metabolism.

## Patentansprüche

1. Phosphonosulfonate und die pharmazeutisch annehmbaren Salze und Ester hievon gemäß der Formel (I): 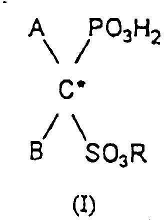 worin
(A)
(1) A von der aus Wasserstoff, Halogen, SR¹, R²SR¹, Amino, Hydroxy, und substituiertem oder unsubstituiertem C₁-C₈Alkyl bestehenden Gruppe ausgewählt ist;
(2) B für
(a) -NH₂;
(b) eine gesättigte oder ungesättigte C₁-C₁₅-Alkylkette, die mit einem oder mehreren Substituenten substituiert ist, welche von der aus -R³N(R⁴)₂, -R³[-N(R⁵)₃]+, -R³N(R⁴)C(O)R⁴, -R³N(R⁴)C(S)R⁴, -R³N(R⁴)C(N)R⁴ und -R³C(O)N(R⁴)₂ bestehenden Gruppe ausgewählt sind;
(c) eine substituierte oder unsubstituierte, gesättigte oder ungesättigte Heteroalkylkette mit 2 bis 15 Kettenatomen, worin eines oder mehrere der genannten Kettenatome Stickstoff sind;
(d) eine gesättigte oder ungesättigte Heteroalkylkette mit 2 bis 15 Kettenatomen, worin eines oder mehrere der genannten Kettenatome unter S und O ausgewählt sind; und worin die genannte Heteroalkylkette mit einem oder mehreren Substituenten substituiert ist, welche von der aus -R³N(R⁴)₂, -R³[-N(R⁵)₃]+, -R³N(R⁴)C(O)R⁴, -R³N(R⁴)C(S)R⁴, -R³N(R⁴)C(N)R⁴ und -R³C(O)N(R⁴)₂ bestehenden Gruppe ausgewählt sind; oder
(e) R⁶-L- steht, worin
(i) L von der Gruppe ausgewählt ist, welche aus S, O, einer substituierten oder unsubstituierten, gesättigten oder ungesättigten C₁-C₁₅Alkylenkette, und einer substituierten oder unsubstituierten, gesättigten oder ungesättigten Heteroalkylenkette mit 2 bis 15 Kettenatomen besteht, worin eines oder mehrere der genannten Kettenatome N, S oder O sind, oder worin L fehlt; und (ii) R⁶ von der Gruppe ausgewählt ist, welche aus gesättigten, monocyclischen oder polycyclischen, carbocyclischen Ringen; ungesättigten, monocyclischen oder polycyclischen, carbocyclischen Ringen; gesättigten, monocyclischen oder polycyclischen, heterocyclischen Ringen; und ungesättigten, monocyclischen oder polycyclischen, heterocyclischen Ringen beeteht; worin R⁶ mit einem oder mehreren Substituenten substituiert sein kann, welche unabhängig von der aus -R³SR¹, substituiertem oder unsubstituiertem C₁-C₈Alkyl, -R³OR⁴, -R³CO₂R⁴, -R³O₂CR⁴, -R³N(R⁴)₂, R³[-N(R⁵)₃]+, -R³N(R⁴)C(O)R⁴, -R³N(R⁴)C(S)R⁴, -R³N(R⁴)C(N)R⁴, -R³C(O)N(R⁴)₂, Halogen, -R³C(O)R⁴, Arylalkyl, Nitro, substituiertem oder unsubstituiertem Aryl und Hydroxy besteht; und
(3)
(a) R¹ unabhängig von der aus Wasserstoff, -C(O)R⁷, -C(S)R⁷, -C(O)N(R⁷)₂, -C(O)OR⁷, -C(S)N(R⁷)₂ und -C(S)OR⁷ bestehenden Gruppe ausgewählt ist, worin R⁷ für Wasserstoff, oder substituiertes oder unsubstituiertes C₁-C₈Alkyl steht;
(b) R² substituiertes oder unsubstituiertes C₁-C₈Alkylen ist;
(c) R³ von der aus substituiertem oder unsubstituiertem C₁-C₈Alkylen bestehenden Gruppe ausgewählt ist oder fehlt;
(d) R⁴ unabhängig von der aus Wasserstoff, substituiertem oder unsubstituiertem C₁-C₈Alkyl und -R²SR¹ bestehenden Gruppe ausgewählt ist; und
(e) R⁵ unabhängig von der aus substituiertem oder unsubstituiertem C₁-C₁₅Alkyl, substituiertem oder unsubstituiertem Phenyl: Benzyl und -R²SR¹ bestehenden Gruppe ausgewählt ist;
oder
(B) A und B mit C* kovalent verbunden sind, um einen monocyclischen oder bicyclischen Ring der folgenden Struktur auszubilden: 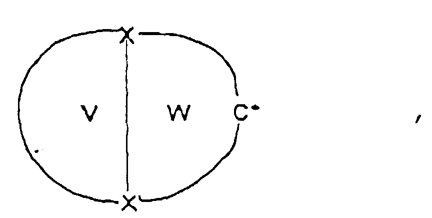 worin
(1) W ein substituierter oder unsubstituierter, gesättigter oder ungesättigter, C*, X und X' umfassender carbocyclischer Ring ist, welcher carbocyclische Ring insgesamt 3 bis 6 Ringkohlenstoffatome aufweist; oder ein substituierter oder unsubstituierter, gesättigter oder ungesättigter, C*, X und X' umfassender heterocyclischer Ring ist, welcher heterocyclische Ring insgesamt 4 bis 6 Ringatome besitzt, wobei eines oder mehrere der genannten Ringatome N, O oder S sind;
(2) V ein substituierter oder unsubstituierter, gesättigter oder ungesättigter, X und X' umfassender carbocyclischer Ring ist, welcher carbocyclische Ring insgesamt 3 bis 8 Ringkohlenstoffatome aufweist; oder ein substituierter oder unsubstituierter, gesättigter oder ungesättigter, X und X' umfassender heterocyclischer Ring ist, welcher heterocyclische Ring insgesamt 3 bis 8 Ringatome besitzt, wobei eines oder mehrere der genannten Ringatome N, O oder S sind; oder worin V fehlt;
(3) X und X' unabhängig voneinander N oder C sind;
mit der Ausnahme, daß wenn weder V noch W einen Stickstoff enthaltenden Heterocyclus darstellt, mindestens einer von V oder W mit einem oder mehreren Substituenten substituiert ist, welche von der aus -R³N(R⁴)₂, R³[-N(R⁵)₃]+, -R³N(R⁴)C(O)R⁴, -R³N(R⁴)C(S)R⁴, -R³N(R⁴)C(N)R⁴ und -R³C(O)N(R⁴)₂ bestehenden Gruppe ausgewählt sind;
und worin R von der aus Wasserstoff, Niederalkyl, Niederacyloxyalkyl, Aminocarbonyloxyalkyl, Pivaloyloxymethyl, Niederalkoxyacyloxyalkyl, Alkoxyalkyl, Cholin und Acylaminoalkyl bestehenden Gruppe ausgewählt ist.

2. Phosphonosulfonat nach Anspruch 1, worin A Hydroxy darstellt; und B eine substituierte oder unsubstituierte, gesättigte oder ungesättigte Heteroalkylkette mit 2 bis 15 Kettenatomen bedeutet, worin eines oder mehrere der genannten Kettenatome Stickstoff sind, oder B für R⁶-L- steht, worin L eine C₁-C₁₅Alkylenkette oder eine Stickstoff enthaltende Heteroalkylenkette mit 2 bis 15 Kettenatomen ist, und worin R⁶ für einen monocyclischen oder polycyclischen Heterocyclus oder Carbocyclus steht.

3. Phosphonosulfonat nach Anspruch 2, worin B R⁶-L- bedeutet und L eine C₁-C₁₅Alkylenkette, wobei die genannte Alkylenkette mit einem oder mehreren Substituenten substituiert ist, welche von der aus -R³SR¹, substituiertem oder unsubstituiertem C₁-C₈Alkyl, -R³OR⁴ und -R³CO₂R⁴ bestehenden Gruppe ausgewählt sind, oder eine Heteroalkylenkette mit 2 bis 15 Kettenatomen darstellt, wobei die genannte Heteroalkylenkette mit einem oder mehreren Substituenten substituiert ist, welche von der aus -R³SR¹, -R³N(R⁴)₂, -R³[N(R⁵)₃+] und -R³N(R⁴)C(O)R⁴ bestehenden Gruppe ausgewählt sind.

4. Phosphonosulfonat nach Anspruch 3, worin R⁶ ein substituierter oder unsubstituierter, sechsgliedriger, monocyclischer Heterocyclus, welcher von der aus Pyridin, Pyrimidin, Piperidin, Pyridinium, Pyrimidinium und Piperidinium bestehenden Gruppe ausgewählt ist; ein substituierter oder unsubstituierter, fünfgliedriger, monocyclischer Heterocyclus, welcher von der aus Imidazol, Pyrrol, Pyrrolidin, Imidazolium, Pyrrolium und Pyrrolidinium bestehenden Gruppe ausgewählt ist; ein substituierter oder unsubstituierter, monocyclischer Carbocyclus, welcher von der aus Cycloheptyl oder Cyclohexyl bestehenden Gruppe ausgewählt ist; ein substituierter oder unsubstituierter, polycyclischer Heterocyclus mit einem an einen fünfgliedrigen Ring kondensierten sechsgliedrigen Ring, welcher polycyclische Heterocyclus von der aus Indol, Indolium, Pyrindin, Imidazol-(1,2-a-)pyridin, Imidazol-(1,2-a-)pyridinium und Pyrindinium bestehenden Gruppe ausgewählt ist; oder ein substituierter oder unsubstituierter, polycyclischer Heterocyclus mit einem an einen sechsgliedrigen Ring kondensierten sechsgliedrigen Ring ist, welcher polycyclische Heterocyclus von der aus Chinolin, Isochinolin, Tetrahydrochinolin, Octahydrochinolin, Chinolinium, Isochinolinium, Tetrahydrochinolinium und Octahydrochinolinium bestehenden Gruppe ausgewählt ist.

5. Phosphonosulfonat nach Anspruch 4, worin R⁶ mit einem oder mehreren Substituenten substituiert ist, welche von der aus -SR¹, -R³N(R⁴)₂, -R³[N(R⁵)₃]+ und -R³N(R⁴) bestehenden Gruppe ausgewählt sind.

6. Phosphonosulfonat nach Anspruch 2, worin B eine substituierte oder unsubstituierte, gesättigte oder ungesättigte Heteroalkylkette mit 2 bis 15 Kettenatomen bedeutet, worin eines oder mehrere der genannten Kettenatome Stickstoff sind, und die genannte Heteroalkylkette mit einem oder mehreren substituenten substituiert ist, welche von der aus -R³SR¹, substituiertem oder unsubstituiertem C₁-C₈Alkyl, -R³OR⁴, -R³CO₂R^{4,} -R³N(R⁴)₂, -R³[N(R⁵)₃]+ und -R³N(R⁴)C(O)R⁴ bestehenden Gruppe ausgewählt sind.

7. Phosphonosulfonat nach Anspruch 1, worin A und B gemeinsam mit C* einen bicyclischen Ring ausbilden, worin W einen fünfgliedrigen carbocyclischen Ring darstellt, und worin V ein funfgliedriger heterocyclischer Ring mit mindestens einem Ringstickstoffatom oder vorzugsweise ein sechsgliedriger heterocyclischer Ring mit mindestens einem Ringstickstoffatom ist.

8. Phosphonosulfonat nach Anspruch 7, worin der genannte bicyclische Ring mit einem oder mehreren Substituenten substituiert ist, welche von der aus substituiertem oder unsubstituiertem C₁-C₈Alkyl, -R³OR⁴, -R³O₂CR⁴, -R³N(R⁴)C(S)R⁴, -R³N(R⁴)C(N)R⁴, -R³C(O)N(R⁴)₂, -R³SR¹, -R³CO₂R⁴, -R³N(R⁴)₂, -R³[N(R⁵)₃]+ und -R³N(R⁴)C(O)R⁴ bestehenden Gruppe ausgewählt sind.

9. Phosphonosulfonate und pharmazeutisch annehmbare Salze und Ester hievon nach Formel (I): 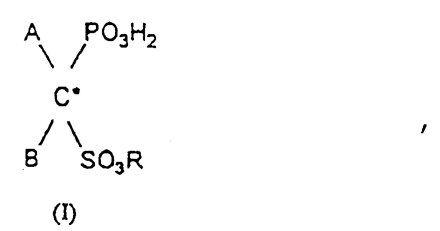 worin
(A)
(1) A Hydroxy darstellt; und
(2) B für 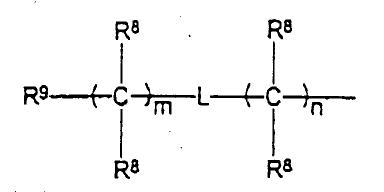 steht, worin
(a) m eine ganze Zahl von 0 bis 10 darstellt, n eine ganze Zahl von 0 bis 10 darstellt, und m+n eine ganze Zahl von 0 bis 10 ist;
(b) R⁸ unabhängig von der aus -R³SR¹, Wasserstoff, substituiertem oder unsubstituiertem C₁-C₈Alkyl, -R³OR⁴, -R³CO₂R⁴, -R³O₂CR⁴, -R³N(R⁴)₂, -R³[N(R⁵)₃]+, -R³N(R⁴)C(O)R⁴, -R³N(R⁴)C(S)R⁴, -R³N(R⁴)C(N)R⁴, -R³C(O)N(R⁴)₂, Halogen, -R³C(O)R⁴, Nitro, Hydroxy, substituierten oder unsubstituierten, gesättigten, monocyclischen oder polycyclischen, carbocyclischen Ringen; substituierten oder unsubstituierten, ungesättigten, monocyclischen oder polycyclischen, carbocyclischen Ringen; substituierten oder unsubstituierten, gesättigten, monocyclischen oder polycyclischen, heterocyclischen Ringen; und substituierten oder unsubstituierten, ungesättigten, monocyclischen oder polycyclischen, heterocyclischen Ringen bestehenden Gruppe ausgewählt ist;
(c) R¹ unabhängig von der aus Wasserstoff, -C(O)R⁷, -C(S)R⁷, -C(O)N(R⁷)₂, -C(O)OR⁷, -C(S)N(R⁷)₂ und -C(S)OR⁷ bestehenden Gruppe ausgewählt ist, worin R⁷ Wasserstoff oder substituiertes oder unsubstituiertes C₁-C₈Alkyl bedeutet;
(d) R³ von der aus substituiertem oder unsubstituiertem C₁-C₈Alkylen bestehenden Gruppe ausgewählt ist oder fehlt;
(e) R⁴ unabhängig von der aus Wasserstoff, substituiertem oder unsubstituiertem C₁-C₈Alkyl und -R²SR¹ bestehenden Gruppe ausgewählt ist;
(f) R⁵ unabhängig von der aus substituiertem oder unsubstituiertem C₁-C₃₅Alkyl, substituiertem oder unsubstituiertem Phenyl; Benzyl und -R²SR¹ bestehenden Gruppe ausgewählt ist;
(g) L von der Gruppe ausgewählt ist, welche aus -N(R⁸)-, [-N(R⁵)₂-]+, -S-, -O- und -D-C(=E)-S- besteht, worin D von der aus einer kovalenten Bindung, O oder S bestehenden Gruppe ausgewählt ist, und E für O oder S steht; oder L fehlt; und worin
(i) wenn L für -N(R⁸)- steht, oder wenn L [-N(R⁵)₂-]+ ist und m eine ganze Zahl von 1 bis 10 darstellt, R⁹ unabhängig von der aus Wasserstoff, substituiertem oder unsubstituiertem C₁-C₁₅Alkyl, R²SR¹ und R¹⁰ bestehenden Gruppe ausgewählt ist;
(ii) wenn L [-N(R⁵)₂-]+ ist und m 0 bedeutet, R⁹ von der aus substituiertem oder unsubstituiertem C₁-C₁₅Alkyl, R²SR¹ und R¹⁰ bestehenden Gruppe ausgewählt ist; oder
(iii)wenn L fehlt oder -S-, -O- oder -D-C(=E)-S bedeutet, R⁹ für R¹⁰ steht;
(h) R¹⁰ ein gesättigter, ungesättigter oder aromatischer, monocyclischer oder polycyclischer Carbocyclus, oder ein gesättigter, ungesättigter oder aromatischer, monocyclischer oder polycyclischer Heterocyclus mit einem oder mehreren Heteroatomen ist, wobei der genannte Carbocyclus oder Heterocyclus mit einem oder mehreren R¹¹-Substituenten substituiert ist; und
(i) jeder R¹¹-Substituent unabhängig von der aus -R³SR¹, substituiertem oder unsubstituiertem C₁-C₈Alkyl, -R³OR⁴, -R³CO₂R⁴, -R³O₂CR⁴, -R³N(R⁴)₂, -R³[-N(R⁵)₃]+, -R³N(R⁴)C(O)R⁴, -R³N(R⁴)C(S)R⁴, -R³N(R⁴)C(N)R⁴, -R³C(O)N(R⁴)₂, Halogen, -R³C(O)R⁴, Hydroxy, substituiertem oder unsubstituiertem Arylalkyl, Nitro und unsubstituiertem oder substituiertem Aryl bestehenden Gruppe ausgewählt ist;
(j) R² substituiertes oder unsubstituiertes C₁-C₈Alkylen darstellt;
oder
(B) A und B gemeinsam mit C* kovalent verbunden sind, um einen bicyclischen Ring der folgenden Struktur: 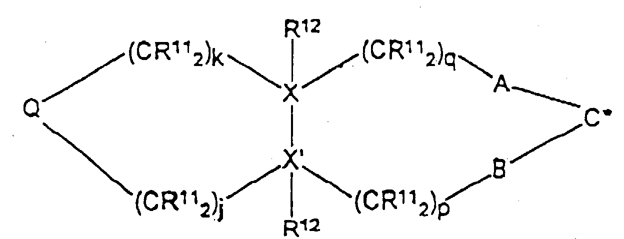 auszubilden, worin
(a) A und B unabhängig von der aus -O-, -S- und -NR¹⁰- bestehenden Gruppe ausgewählt sind oder fehlen;
(b) Q von der aus -NR¹²⁻ und [-N(R¹³)₂-]+ bestehenden Gruppe ausgewählt ist oder fehlt;
(c) X und X' unabhängig unter C oder N ausgewählt sind;
(d) jeder Rest R¹² unabhängig von der aus -R³SR¹, Wasserstoff, substituiertem oder unsubstituiertem C₁-C₈-Alkyl, -R³OR⁴, -R³CO₂R⁴, -R³O₂CR⁴, -R³N(R⁴)₂, -R³[-N(R⁵)₃]+, -R³N(R⁴)C(O)R⁴, -R³C(O)N(R⁴)₂, Halogen, -R³C(O)R⁴, Hydroxy, substituiertem oder unsubstituiertem Arylalkyl, Nitro und unsubstituiertem oder substituiertem Aryl bestehenden Gruppe ausgewählt ist; und
(e) jeder Rest R¹³ von der aus substituiertem oder unsubstituiertem C₁-C₃₅Alkyl, substituiertem oder unsubstituiertem Phenyl; Benzyl und -R²SR¹ bestehenden Gruppe ausgewählt ist,
(f) wenn Q vorhanden ist, k und j und k+j ganze Zahlen von 0 bis 5 darstellen; wenn Q fehlt, k und j und k+j ganze Zahlen von 0 bis 6 sind; und
(g) p und q und p+q unabhängig ganze Zahlen von 0 bis 3 darstellen,
mit der Ausnahme, daß wenn Q fehlt, mindestens einer der Reste R¹¹ oder R¹² von der aus -R³N(R⁴)₂; -R³[-N(R⁵)₃]+, -R³N(R⁴)C(O)R⁴, -R³N(R⁴)C(S)R⁴, -R³N(R⁴)C(N)R⁴ und -R³C(O)N(R⁴)₂ bestehenden Gruppe ausgewählt ist;
und worin R von der aus Wasserstoff, Niederalkyl, Niederacyloxyalkyl, Aminocarbonyloxyalkyl, Pivaloyloxymethyl, Niederalkoxyacyloxyalkyl, Alkoxyalkyl, Cholin und Acylaminoalkyl bestehenden Gruppe ausgewählt ist.

10. Pharmazeutische Zusammensetzung, umfassend:
(a) eine sichere und wirksame Menge von einem Phosphonosulfonat nach Anspruch 1, 2, 3, 4, 5, 6, 7, 8 oder 9 und
(b) pharmazeutisch annehmbare Träger.

11. Verwendung von einem Phosphonosulfonat nach Anspruch 1,2, 3, 4, 5, 6, 7, 8 oder 9 zur Herstellung eines Arzneimittels zur Behandlung von abnormem Calcium- und Phosphatstoffwechsel.

## Revendications

1. Phosphonosulfonates, et leurs sels et esters pharmaceutiquement acceptables, de formule (I): 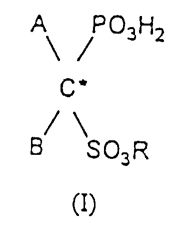 dans laquelle
(A)
(1) A est choisi dans le groupe constitué par un atome d'hydrogène; un atome d'halogène; SR¹ ; R²SR¹; et les groupes amino; hydroxy et alkyle en C₁-C₈ substitués ou non substitués;
(2) B est
(a) -NH₂;
(b) une chaîne alkyle en C₁-C₁₅, saturée ou insaturée, substituée par un ou plusieurs substituants choisis dans le groupe constitué par -R³N(R⁴)₂; -R³[N(R⁵)₃]⁺; -R³N(R⁴)C(O)R⁴; -R³N(R⁴)C(S)R⁴; -R³N(R⁴)C(N)R⁴; et -R³C(O)N(R⁴)₂;
(c) une chaîne hétéroalkyle saturée ou insaturée, substituée ou non substituée, comportant de 2 à 15 atomes dans la chaîne, dans laquelle un ou plusieurs desdits atomes de la chaîne sont des atomes d'azote;
(d) une chaîne hétéroalkyle saturée ou insaturée comportant de 2 à 15 atomes dans la chaîne, dans laquelle un ou plusieurs desdits atomes de la chaîne sont choisis parmi S et O; et dans laquelle ladite chaîne hétéroalkyle est substituée par un ou plusieurs substituants choisis dans le groupe constitué par -R³N(R⁴)₂; -R³[N(R⁵)₃]⁺; -R³N(R⁴)C(O)R⁴; -R³N(R⁴)C(S)R⁴; -R³N(R⁴)C(N)R⁴; et -R³C(O)N(R⁴)₂; ou
(e) R⁶-L-, où
(i) L est choisi dans le groupe constitué par rien; S; O; une chaîne alkylène en C₁-C₁₅, saturée ou insaturée, substituée ou non substituée; et une chaîne hétéroalkylène saturée ou insaturée, substituée ou non substituée, comportant de 2 à 15 atomes dans la chaîne, dans laquelle un ou plusieurs desdits atomes de la chaîne sont N, S ou O; et
(ii) R⁶ est choisi dans le groupe constitué par les noyaux carbocycliques monocycliques ou polycycliques saturés; les noyaux carbocycliques monocycliques ou polycycliques insaturés; les noyaux hétérocycliques monocycliques ou polycycliques saturés; et les noyaux hétérocycliques monocycliques ou polycycliques insaturés; où R⁶ peut être substitué par un ou plusieurs substituants choisis indépendamment dans le groupe constitué par -R³SR¹; les groupes alkyle en C₁-C₈ substitués ou non substitués; -R³OR⁴; -R³CO₂R⁴; -R³O₂CR⁴; -R³N(R⁴)₂; -R³[N(R⁵)₃]⁺; -R³N(R⁴)C(O)R⁴; -R³N(R⁴)C(S)R⁴; -R³N(R⁴)C(N)R⁴; -R³C(O)N(R⁴)₂; les atomes d'halogène; -R³C(O)R⁴; les groupes arylalkyle; nitro; aryle substitué ou non substitué; et hydroxy; et
(3)
(a) R¹ est choisi indépendamment dans le groupe constitué par un atome d'hydrogène; -C(O)R⁷; -C(S)R⁷; -C(O)N(R⁷)₂; -C(O)OR⁷; -C(S)N(R⁷)₂; et -C(S)OR⁷; où R⁷ est un atome d'hydrogène ou un groupe alkyle en C₁-C₈ substitué ou non substitué;
(b) R² est un groupe alkylène en C₁-C₈ substitué ou non substitué;
(c) R³ est choisi dans le groupe constitué par rien et un groupe alkylène en C₁-C₈ substitué ou non substitué;
(d) R⁴ est choisi indépendamment dans le groupe constitué par un atome d'hydrogène; un groupe alkyle en C₁-C₈ substitué ou non substitué; et -R²SR¹; et
(e) R⁵ est choisi indépendamment dans le groupe constitué par un groupe alkyle en C₁-C₁₅ substitué ou non substitué; phényle substitué ou non substitué; benzyle; et -R²SR¹;
ou
(B) A et B sont liés par covalence avec C* pour former un noyau monocyclique ou bicyclique de structure suivante: 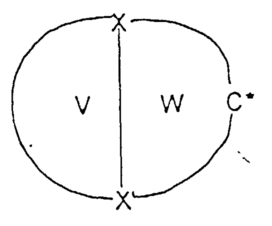 dans laquelle
(1) W est un noyau carbocyclique saturé ou insaturé, substitué ou non substitué, comprenant C*, X et X', ledit noyau carbocyclique comportant un total de 3 à 6 atomes de carbone dans le noyau; ou un noyau hétérocyclique saturé ou insaturé, substitué ou non substitué, comprenant C*, X et X', ledit noyau hétérocyclique comportant un total de 4 à 6 atomes dans le noyau, où un ou plusieurs desdits atomes du noyau sont N, O ou S;
(2) V est rien; un noyau carbocyclique saturé ou insaturé, substitué ou non substitué, comprenant X et X', ledit noyau carbocyclique comportant un total de 3 à 8 atomes de carbone dans le noyau; ou un noyau hétérocyclique saturé ou insaturé, substitué ou non substitué, comprenant X et X', ledit noyau hétérocyclique comportant un total de 3 à 8 atomes dans le noyau, où un ou plusieurs desdits atomes du noyau sont N, O ou S; et
(3) X et X' sont indépendamment N ou C;
sauf que, si ni V ni W n'est un hétérocycle azoté, l'un au moins des noyaux V et W est substitué par un ou plusieurs substituants choisis dans le groupe constitué par -R³N(R⁴)₂; -R³[N(R⁵)₃]⁺; -R³N(R⁴)C(O)R⁴; -R³N(R⁴)C(S)R⁴; -R³N(R⁴)C(N)R⁴; et -R³C(O)N(R⁴)₂;
et dans laquelle R est choisi dans le groupe constitué par un atome d'hydrogène, un groupe alkyle inférieur, acyloxyalkyle inférieur, aminocarbonyloxyalkyle, pivaloyloxyméthyle, alcoxyacyloxyalkyle inférieur, alcoxyalkyle, choline et acylaminoalkyle.

2. Phosphonosulfonate selon la revendication 1, dans lequel A est un groupe hydroxy; et B est une chaîne hétéroalkyle saturée ou insaturée, substituée ou non substituée, comportant de 2 à 15 atomes dans la chaîne, dans laquelle un ou plusieurs desdits atomes de la chaîne sont des atomes d'azote, ou B est R⁶-L-, où L est une chaîne alkylène en C₁-C₁₅, ou une chaîne hétéroalkylène azotée comportant de 2 à 15 atomes dans la chaîne, et où R⁶ est un hétérocycle ou un carbocycle, monocyclique ou polycyclique.

3. Phosphonosulfonate selon la revendication 2, dans lequel B est R⁶-L- et L est une chaîne alkylène en C₁-C₁₅, où ladite chaîne alkylène est substituée par un ou plusieurs substituants choisis dans le groupe constitué par -R³SR¹, les groupes alkyle en C₁-C₈ substitués ou non substitués, -R³OR⁴ et -R³CO₂R⁴; ou une chaîne hétéroalkylène comportant de 2 à 15 atomes dans la chaîne, où ladite chaîne hétéroalkylène est substituée par un ou plusieurs substituants choisis dans le groupe constitué par -R³SR¹, -R³N(R⁴)₂, -R³[N(R⁵)₃]⁺ et -R³N(R⁴)C(O)R⁴.

4. Phosphonosulfonate selon la revendication 3, dans lequel R⁶ est un hétérocycle monocyclique à six chaînons, substitué ou non substitué, choisi dans le groupe constitué par la pyridine, la pyrimidine, la pipéridine, le pyridinium, le pyrimidinium et le pipéridinium; un hétérocycle monocyclique à cinq chaînons, substitué ou non substitué, choisi dans le groupe constitué par l'imidazole, le pyrrole, la pyrrolidine, l'imidazolium, le pyrrolium et le pyrrolidinium; un carbocycle monocyclique substitué ou non substitué choisi dans le groupe constitué par le cycloheptyle ou le cyclohexyle; un hétérocycle polycyclique substitué ou non substitué possédant un noyau à six chaînons condensé à un noyau à cinq chaînons, ledit hétérocycle polycyclique étant choisi dans le groupe constitué par l'indole, l'indolium, la pyrindine, l'imidazole(1,2-a)pyridine, l'imidazole(1,2-a)pyridinium et le pyrindinium; ou un hétérocycle polycyclique substitué ou non substitué possédant un noyau à six chaînons condensé avec un noyau à six chaînons, ledit hétérocycle polycyclique étant choisi dans le groupe constitué par la quinoléine, l'isoquinoléine, la tétrahydroquinoléine, l'octahydroquinoléine, le quinolinium, l'isoquinolinium, le tétrahydroquinolinium et l'octahydroquinolinium.

5. Phosphonosulfonate selon la revendication 4, dans lequel R⁶ est substitué par un ou plusieurs substituants choisis dans le groupe constitué par -SR¹, -R³N(R⁴)₂, -R³[N(R⁵)₃]⁺ et -R³N(R⁴).

6. Phosphonosulfonate selon la revendication 2, dans lequel B est une chaîne hétéroalkyle saturée ou insaturée, substituée ou non substituée, comportant de 2 à 15 atomes dans la chaîne, dans laquelle un ou plusieurs desdits atomes de la chaîne sont des atomes d'azote et ladite chaîne hétéroalkyle est substituée par un ou plusieurs substituants choisis dans le groupe constitué par -R³SR¹; les groupes alkyle en C₁-C₈ substitués ou non substitués; -R³OR⁴; -R³CO₂R⁴; -R³N(R⁴)₂; -R³[N(R⁵)₃]⁺ et -R³N(R⁴)C(O)R⁴.

7. Phosphonosulfonate selon la revendication 1, dans lequel A et B, avec C*, forment un noyau bicyclique, dans lequel W est un noyau carbocyclique à cinq chaînons; et dans lequel V est un noyau hétérocyclique à cinq chaînons contenant au moins un atome d'azote dans le noyau ou, de préférence, un noyau hétérocyclique à six chaînons contenant au moins un atome d'azote dans le noyau.

8. Phosphonosulfonate selon la revendication 7, dans lequel ledit noyau bicyclique est substitué par un ou plusieurs substituants choisis dans le groupe constitué par les groupes alkyle en C₁-C₈ substitués ou non substitués; -R³OR⁴; -R³O₂CR⁴; -R³N(R⁴)C(S)R⁴; -R³N(R⁴)C(N)R⁴; -R³C(O)N(R⁴)₂; -R³SR¹; -R³CO₂R⁴; -R³N(R⁴)₂; -R³[N(R⁵)₃]⁺; et -R³N(R⁴)C(O)R⁴.

9. Phosphonosulfonates, et leurs sels et esters pharmaceutiquement acceptables, de formule (I): 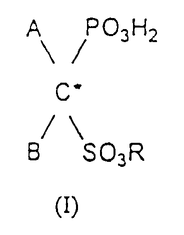 dans laquelle
(A)
(1) A est un groupe hydroxy; et
(2) B est 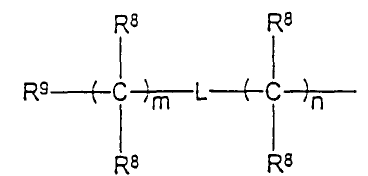 où
(a) m est un nombre entier de 0 à 10; n est un nombre entier de 0 à 10; et m+n est un nombre entier de 0 à 10;
(b) R⁸ est choisi indépendamment dans le groupe constitué par -R³SR¹; l'atome d'hydrogène ; les groupes alkyle en C₁-C₈ substitués ou non substitués; -R³OR⁴; -R³CO₂R⁴; -R³O₂CR⁴; -R³N(R⁴)₂; -R³[N(R⁵)₃]⁺; -R³N(R⁴)C(O)R⁴; -R³N(R⁴)C(S)R⁴; -R³N(R⁴)C(N)R⁴; -R³C(O)N(R⁴)₂; un atome d'halogène; -R³C(O)R⁴; les groupes nitro; hydroxy; les noyaux carbocycliques monocycliques ou polycycliques saturés substitués ou non substitués; les noyaux carbocycliques monocycliques ou polycycliques insaturés substitués ou non substitués; les noyaux hétérocycliques monocycliques ou polycycliques saturés substitués ou non substitués; et les noyaux hétérocycliques monocycliques ou polycycliques insaturés substitués ou non substitués;
(c) R¹ est choisi indépendamment dans le groupe constitué par un atome d'hydrogène; -C(O)R⁷; -C(S)R⁷; -C(O)N(R⁷)₂; -C(O)OR⁷; -C(S)N(R⁷)₂; et -C(S)OR⁷; où R⁷ est un atome d'hydrogène ou un groupe alkyle en C₁-C₈ substitué ou non substitué;
(d) R³ est choisi dans le groupe constitué par rien et un groupe alkylène en C₁-C₈ substitué ou non substitué;
(e) R⁴ est choisi indépendamment dans le groupe constitué par un atome d'hydrogène; un groupe alkyle en C₁-C₈ substitué ou non substitué; et -R²SR¹;
(f) R⁵ est choisi indépendamment dans le groupe constitué par un groupe alkyle en C₁-C₃₅ substitué ou non substitué; phényle substitué ou non substitué; benzyle; et -R²SR¹;
(g) L est choisi dans le groupe constitué par rien; -N(R⁸)-; -[N(R⁵)₂]⁺ -; -S-; -O-; et -D-C(=E)-S-; où D est choisi dans le groupe constitué par une liaison covalente, O ou S, et E est O ou S; et dans lequel
(i) lorsque L est -N(R⁸)-, ou lorsque L est-[N(R⁵)₂]⁺- et que m est un nombre entier de 1 à 10, R⁹ est choisi indépendamment dans le groupe constitué par un atome d'hydrogène; un groupe alkyle en C₁-C₁₅ substitué ou non substitué; R²SR¹; et R¹⁰;
(ii) lorsque L est -[N(R⁵)₂]⁺- et que m = 0, R⁹ est choisi dans le groupe constitué par un groupe alkyle en C₁-C₁₅ substitué ou non substitué; R²SR¹; et R¹⁰; ou
(iii) lorsque L est rien, -S-, -O- ou -D-C(=E)-S, R⁹ est R¹⁰;
(h) R¹⁰ est un carbocycle monocyclique ou polycyclique, saturé, insaturé ou aromatique, ou un hétérocycle monocyclique ou polycyclique, saturé, insaturé ou aromatique, et contenant un ou plusieurs hétéroatomes; dans lequel ledit carbocycle ou hétérocycle est substitué par un ou plusieurs substituants R¹¹; et
(i) chaque R¹¹ est choisi indépendamment dans le groupe constitué par -R³SR¹; un groupe alkyle en C₁-C₈ substitué ou non substitué; -R³OR⁴; -R³CO₂R⁴; -R³O₂CR⁴; -R³N(R⁴)₂; -R³[N(R⁵)₃]⁺; -R³N(R⁴)C(O)R⁴; -R³N(R⁴)C(S)R⁴; -R³N(R⁴)C(N)R⁴; -R³C(O)N(R⁴)₂; un atome d'halogène; -R³C(O)R⁴; un groupe hydroxy; arylalkyle substitué ou non substitué; nitro; et aryle substitué ou non substitué;
(j) R² est un groupe alkylène en C₁-C₈ substitué ou non substitué;
ou (B) A et B sont liés par covalence avec C* pour former un noyau bicyclique de structure suivante 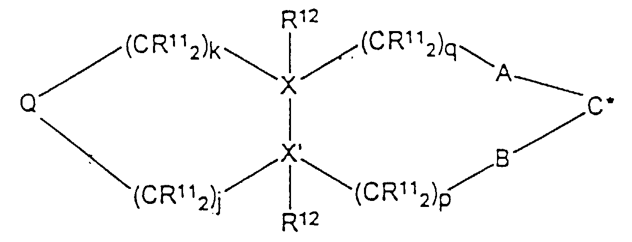
dans laquelle
(a) A et B sont choisis indépendamment dans le groupe constitué par rien, -O-, -S- et -NR¹⁰-;
(b) Q est choisi dans le groupe constitué par rien; -NR¹²-; et [-N(R¹³)₂-]⁺;
(c) X et X' sont choisis indépendamment parmi C et N;
(d) chaque R¹² est choisi indépendamment dans le groupe constitué par -R³SR¹; un atome d'hydrogène ; un groupe alkyle en C₁-C₈ substitué ou non substitué; -R³OR⁴; -R³CO₂R⁴; -R³O₂CR⁴; -R³N(R⁴)₂; -R³[N(R⁵)₃]⁺; -R³N(R⁴)C(O)R⁴; -R³C(O)N(R⁴)₂; un atome d'halogène; -R³C(O)R⁴; un groupe hydroxy; arylalkyle substitué ou non substitué; nitro; et aryle substitué ou non substitué; et
(e) chaque R¹³ est choisi dans le groupe constitué par un groupe alkyle en C₁-C₃₅ substitué ou non substitué; phényle substitué ou non substitué; benzyle; et -R²SR¹;
(f) lorsque Q est autre que rien, k et j et k+j sont des nombres entiers de 0 à 5; lorsque Q est rien, k et j et k+j sont des nombres entiers de 0 à 6; et
(g) p et q et p+q sont indépendamment des nombres entiers de 0 à 3 ; sauf que, si Q est rien, au moins un des radicaux R¹¹ et R¹² est choisi dans le groupe constitué par -R³N(R⁴)₂; -R³[N(R⁵)₃]⁺; -R³N(R⁴)C(O)R⁴; -R³N(R⁴)C(S)R⁴; -R³N(R⁴)C(N)R⁴; et -R³C(O)N(R⁴)₂;
et dans laquelle R est choisi dans le groupe constitué par un atome d'hydrogène, un groupe alkyle inférieur, acyloxyalkyle inférieur, aminocarbonyloxyalkyle, pivaloyloxyméthyle, alcoxyacyloxyalkyle inférieur, alcoxyalkyle, choline et acylaminoalkyle.

10. Composition pharmaceutique, comprenant:
(a) une quantité sans danger et efficace d'un phosphonosulfonate selon la revendication 1, 2, 3, 4, 5, 6, 7, 8 ou 9; et
(b) des véhicules pharmaceutiquement acceptables.

11. Utilisation d'un phosphonosulfonate, selon la revendication 1, 2, 3, 4, 5, 6, 7, 8 ou 9, pour la fabrication d'un médicament pour le traitement du métabolisme anormal du calcium et du phosphate.
